# EUROPEAN PATENT APPLICATION

(11) **EP 3 858 991 A1**
(43) Date of publication of application: **04.08.2021**
(21) Application number: 20196219.8
(22) Date of filing: 25.09.2015
(51) Int. Cl.: C12N 15/09, C07K 16/28, C12N 5/10, A61P 35/00, C12N 5/0783, A61K 35/17, C07K 14/725, C07K 14/705

(54) **GLYPICAN-3 SPECIFIC CHIMERIC ANTIGEN RECEPTORS FOR ADOPTIVE IMMUNOTHERAPY**

(30) Priority: 26.09.2014 US 201462055979 P
(62) Divisional of application: 15845117.9
(71) Applicant: Baylor College of Medicine, Houston, TX 77030 (US)
(72) Inventor: HECZEY, Andras, Houston, TX 77025 (US); GOTTSCHALK, Stephen, M. G., Houston, TX 77030 (US); METELITSA, Leonid, S., Sugar Land, TX 77479 (US); DOTTI, Gianpietro, Houston, TX Texas (US); LI, Wenpeng, Houston, TX 77030-3411 (US)
(74) Representative: Grünecker Patent- und Rechtsanwälte PartG mbB

(57) **Abstract**

Embodiments of the disclosure include methods and compositions related to immunotherapy that targets glypican-3. In particular embodiments, immune cells engineered to comprise a chimeric antigen receptor that targets glypican-3 are contemplated, and uses thereof. In particular embodiments, medical conditions that are associated with glypican-3 expression or an overexpression of glypican-3 are treated with GPC3 CARs.

SEQUENCE LISTING

<110> Heczey, Andras Attila
      Gottschalk, Stephen M.G.
      Metelitsa, Leonid S.
      Dotti, Gianpietro

<120> GLYPICAN-3 CHIMERIC ANTIGEN RECEPTORS

<130> N410522EP-A

<140> TBC
<141> TBC

<150> EP 15845117.9
<151> 2015-09-25

<160> 33

<170> PatentIn version 3.5

<210> 1
<211> 261
<212> PRT
<213> Homo sapiens

<400> 1

Met Asp Trp Ile Trp Arg Ile Leu Phe Leu Val Gly Ala Ala Thr Gly
1               5                   10                  15

Ala His Ser Gln Val Gln Leu Gln Gln Ser Gly Ala Glu Leu Val Arg
            20                  25                  30

Pro Gly Ala Ser Val Lys Leu Ser Cys Lys Ala Ser Gly Tyr Thr Phe
        35                  40                  45

Thr Asp Tyr Glu Met His Trp Val Lys Gln Thr Pro Val His Gly Leu
    50                  55                  60

Lys Trp Ile Gly Ala Leu Asp Pro Lys Thr Gly Asp Thr Ala Tyr Ser
65                  70                  75                  80

Gln Lys Phe Lys Gly Lys Ala Thr Leu Thr Ala Asp Lys Ser Ser Ser
                85                  90                  95

Thr Ala Tyr Met Glu Leu Arg Ser Leu Thr Ser Glu Asp Ser Ala Val
            100                 105                 110

Tyr Tyr Cys Thr Arg Phe Tyr Ser Tyr Thr Tyr Trp Gly Gln Gly Thr
        115                 120                 125

Leu Val Thr Val Ser Ala Gly Gly Gly Gly Ser Gly Gly Gly Gly Ser
    130                 135                 140

Gly Gly Gly Gly Ser Asp Val Val Met Thr Gln Thr Pro Leu Ser Leu
145                 150                 155                 160

Pro Val Ser Leu Gly Asp Gln Ala Ser Ile Ser Cys Arg Ser Ser Gln
                165                 170                 175

Ser Leu Val His Ser Asn Gly Asn Thr Tyr Leu His Trp Tyr Leu Gln
            180                 185                 190

Lys Pro Gly Gln Ser Pro Lys Leu Leu Ile Tyr Lys Val Ser Asn Arg
        195                 200                 205

Phe Ser Gly Val Pro Asp Arg Phe Ser Gly Ser Gly Ser Gly Thr Asp
    210                 215                 220

Phe Thr Leu Lys Ile Ser Arg Val Glu Ala Glu Asp Leu Gly Val Tyr
225                 230                 235                 240

Phe Cys Ser Gln Asn Thr His Val Pro Pro Thr Phe Gly Ser Gly Thr
                245                 250                 255

Lys Leu Glu Ile Lys
            260

<210>  2
<211>  783
<212>  PRT
<213>  Homo sapiens

<400>  2
   Ala Thr Gly Gly Ala Thr Thr Gly Gly Ala Thr Thr Thr Gly Gly Cys
   1               5                   10                  15
   Gly Cys Ala Thr Thr Cys Thr Gly Thr Thr Thr Cys Thr Gly Gly Thr
               20                  25                  30
   Gly Gly Gly Ala Gly Cys Cys Gly Cys Ala Ala Cys Cys Gly Gly Ala
           35                  40                  45
   Gly Cys Ala Cys Ala Thr Ala Gly Thr Cys Ala Gly Gly Thr Cys Cys
       50                  55                  60
   Ala Gly Cys Thr Gly Cys Ala Gly Cys Ala Gly Thr Cys Ala Gly Gly
   65                  70                  75                  80
   Ala Gly Cys Cys Gly Ala Ala Cys Thr Gly Gly Thr Gly Cys Gly Gly
                   85                  90                  95
   Cys Cys Cys Gly Gly Cys Gly Cys Ala Ala Gly Thr Gly Thr Cys Ala
   100 105 110
   Ala Ala Cys Thr Gly Thr Cys Ala Thr Gly Cys Ala Ala Gly Gly Cys
   115 120 125
   Cys Ala Gly Cys Gly Gly Gly Thr Ala Thr Ala Cys Cys Thr Thr Cys
   130 135 140
   Ala Cys Ala Gly Ala Cys Thr Ala Cys Gly Ala Gly Ala Thr Gly Cys
   145 150 155 160
   Ala Cys Thr Gly Gly Gly Thr Gly Ala Ala Ala Cys Ala Gly Ala Cys
   165 170 175
   Cys Cys Cys Thr Gly Thr Gly Cys Ala Cys Gly Gly Cys Cys Thr Gly
   180 185 190
   Ala Ala Gly Thr Gly Gly Ala Thr Cys Gly Gly Cys Gly Cys Thr Cys
   195 200 205
   Thr Gly Gly Ala Cys Cys Cys Ala Ala Ala Ala Ala Cys Cys Gly Gly
   210 215 220
   Gly Gly Ala Thr Ala Cys Ala Gly Cys Ala Thr Ala Thr Thr Cys Cys
   225 230 235 240
   Cys Ala Gly Ala Ala Gly Thr Thr Thr Ala Ala Ala Gly Gly Ala Ala
   245 250 255
   Ala Gly Gly Cys Cys Ala Cys Thr Cys Thr Gly Ala Cys Cys Gly Cys
   260 265 270
   Thr Gly Ala Cys Ala Ala Gly Ala Gly Cys Thr Cys Cys Thr Cys Thr
   275 280 285
   Ala Cys Thr Gly Cys Cys Thr Ala Cys Ala Thr Gly Gly Ala Gly Cys
   290 295 300
   Thr Gly Ala Gly Gly Ala Gly Cys Cys Thr Gly Ala Cys Ala Thr Cys
   305 310 315 320
   Cys Gly Ala Ala Gly Ala Thr Ala Gly Cys Gly Cys Cys Gly Thr Gly
   325 330 335
   Thr Ala Cys Thr Ala Thr Thr Gly Cys Ala Cys Cys Cys Gly Cys Thr
   340 345 350
   Thr Cys Thr Ala Cys Thr Cys Cys Thr Ala Thr Ala Cys Ala Thr Ala
   355 360 365
   Cys Thr Gly Gly Gly Gly Cys Cys Ala Gly Gly Gly Gly Ala Cys Thr
   370 375 380
   Cys Thr Gly Gly Thr Gly Ala Cys Cys Gly Thr Cys Thr Cys Thr Gly
   385 390 395 400
   Cys Ala Gly Gly Ala Gly Gly Ala Gly Gly Ala Gly Gly Cys Thr Cys
   405 410 415
   Thr Gly Gly Ala Gly Gly Ala Gly Gly Ala Gly Gly Gly Ala Gly Thr
   420 425 430
   Gly Gly Ala Gly Gly Cys Gly Gly Gly Gly Gly Ala Ala Gly Cys Gly
   435 440 445
   Ala Cys Gly Thr Gly Gly Thr Cys Ala Thr Gly Ala Cys Ala Cys Ala
   450 455 460
   Gly Ala Cys Thr Cys Cys Ala Cys Thr Gly Thr Cys Cys Cys Thr Gly
   465 470 475 480
   Cys Cys Cys Gly Thr Gly Ala Gly Cys Cys Thr Gly Gly Gly Cys Gly
   485 490 495
   Ala Thr Cys Ala Gly Gly Cys Thr Ala Gly Cys Ala Thr Thr Thr Cys
   500 505 510
   Cys Thr Gly Thr Cys Gly Ala Ala Gly Thr Thr Cys Ala Cys Ala Gly
   515 520 525
   Ala Gly Thr Cys Thr Gly Gly Thr Gly Cys Ala Cys Thr Cys Ala Ala
   530 535 540
   Ala Cys Gly Gly Ala Ala Ala Thr Ala Cys Cys Thr Ala Thr Cys Thr
   545 550 555 560
   Gly Cys Ala Thr Thr Gly Gly Thr Ala Cys Cys Thr Gly Cys Ala Gly
   565 570 575
   Ala Ala Gly Cys Cys Ala Gly Gly Cys Cys Ala Gly Thr Cys Thr Cys
   580 585 590
   Cys Cys Ala Ala Ala Cys Thr Gly Cys Thr Gly Ala Thr Cys Thr Ala
   595                 600                 605
   Thr Ala Ala Gly Gly Thr Gly Ala Gly Cys Ala Ala Cys Cys Gly Gly
       610                 615                 620
   Thr Thr Cys Thr Cys Cys Gly Gly Gly Gly Thr Cys Cys Cys Thr Gly
   625                 630                 635                 640
   Ala Cys Ala Gly Ala Thr Thr Thr Thr Cys Thr Gly Gly Ala Ala Gly
                   645                 650                 655
   Thr Gly Gly Cys Thr Cys Ala Gly Gly Gly Ala Cys Ala Gly Ala Thr
               660                 665                 670
   Thr Thr Cys Ala Cys Thr Cys Thr Gly Ala Ala Ala Ala Thr Thr Ala
           675                 680                 685
   Gly Cys Ala Gly Ala Gly Thr Gly Gly Ala Gly Gly Cys Cys Gly Ala
       690                 695                 700
   Ala Gly Ala Thr Cys Thr Gly Gly Gly Cys Gly Thr Cys Thr Ala Cys
   705                 710                 715                 720
   Thr Thr Thr Thr Gly Thr Ala Gly Cys Cys Ala Gly Ala Ala Thr Ala
                   725                 730                 735
   Cys Cys Cys Ala Cys Gly Thr Cys Cys Cys Ala Cys Cys Ala Ala Cys
               740                 745                 750
   Ala Thr Thr Cys Gly Gly Ala Ala Gly Cys Gly Gly Cys Ala Cys Thr
           755                 760                 765
   Ala Ala Ala Cys Thr Gly Gly Ala Ala Ala Thr Cys Ala Ala Gly
       770                 775                 780

<210>  3
<211>  415
<212>  PRT
<213>  Homo sapiens

<400>  3

Met Asp Trp Ile Trp Arg Ile Leu Phe Leu Val Gly Ala Ala Thr Gly
1               5                   10                  15

Ala His Ser Gln Val Gln Leu Gln Gln Ser Gly Ala Glu Leu Val Arg
            20                  25                  30

Pro Gly Ala Ser Val Lys Leu Ser Cys Lys Ala Ser Gly Tyr Thr Phe

35                  40                  45

Thr Asp Tyr Glu Met His Trp Val Lys Gln Thr Pro Val His Gly Leu
    50                  55                  60

Lys Trp Ile Gly Ala Leu Asp Pro Lys Thr Gly Asp Thr Ala Tyr Ser
65                  70                  75                  80

Gln Lys Phe Lys Gly Lys Ala Thr Leu Thr Ala Asp Lys Ser Ser Ser
                85                  90                  95

Thr Ala Tyr Met Glu Leu Arg Ser Leu Thr Ser Glu Asp Ser Ala Val
            100                 105                 110

Tyr Tyr Cys Thr Arg Phe Tyr Ser Tyr Thr Tyr Trp Gly Gln Gly Thr
        115                 120                 125

Leu Val Thr Val Ser Ala Gly Gly Gly Gly Ser Gly Gly Gly Gly Ser
    130                 135                 140

Gly Gly Gly Gly Ser Asp Val Val Met Thr Gln Thr Pro Leu Ser Leu
145                 150                 155                 160

Pro Val Ser Leu Gly Asp Gln Ala Ser Ile Ser Cys Arg Ser Ser Gln
                165                 170                 175

Ser Leu Val His Ser Asn Gly Asn Thr Tyr Leu His Trp Tyr Leu Gln
            180                 185                 190

Lys Pro Gly Gln Ser Pro Lys Leu Leu Ile Tyr Lys Val Ser Asn Arg
        195                 200                 205

Phe Ser Gly Val Pro Asp Arg Phe Ser Gly Ser Gly Ser Gly Thr Asp
    210                 215                 220

Phe Thr Leu Lys Ile Ser Arg Val Glu Ala Glu Asp Leu Gly Val Tyr
225                 230                 235                 240

Phe Cys Ser Gln Asn Thr His Val Pro Pro Thr Phe Gly Ser Gly Thr
                245                 250                 255

Lys Leu Glu Ile Lys Glu Pro Lys Ser Cys Asp Lys Thr His Thr Cys
            260                 265                 270

Pro Pro Cys Pro Asp Pro Lys Phe Trp Val Leu Val Val Val Gly Gly
        275                 280                 285

Val Leu Ala Cys Tyr Ser Leu Leu Val Thr Val Ala Phe Ile Ile Arg
    290                 295                 300

Val Lys Phe Ser Arg Ser Ala Asp Ala Pro Ala Tyr Gln Gln Gly Gln
305                 310                 315                 320

Asn Gln Leu Tyr Asn Glu Leu Asn Leu Gly Arg Arg Glu Glu Tyr Asp
                325                 330                 335

Val Leu Asp Lys Arg Arg Gly Arg Asp Pro Glu Met Gly Gly Lys Pro
            340                 345                 350

Arg Arg Lys Asn Pro Gln Glu Gly Leu Tyr Asn Glu Leu Gln Lys Asp
        355                 360                 365

Lys Met Ala Glu Ala Tyr Ser Glu Ile Gly Met Lys Gly Glu Arg Arg
    370                 375                 380

Arg Gly Lys Gly His Asp Gly Leu Tyr Gln Gly Leu Ser Thr Ala Thr
385                 390                 395                 400

Lys Asp Thr Tyr Asp Ala Leu His Met Gln Ala Leu Pro Pro Arg
                405                 410                 415

<210>  4
<211>  15
<212>  PRT
<213>  Homo sapiens

<400>  4

Glu Pro Lys Ser Cys Asp Lys Thr His Thr Cys Pro Pro Cys Pro
1               5                   10                  15

<210>  5
<211>  24
<212>  PRT
<213>  Homo sapiens

<400>  5

Phe Trp Val Leu Val Val Val Gly Gly Val Leu Ala Cys Tyr Ser Leu
1               5                   10                  15

Leu Val Thr Val Ala Phe Ile Ile
            20

<210>  6
<211>  1254
<212>  DNA
<213>  Homo sapiens

<400>  6
atggattgga tttggcgcat tctgtttctg gtgggagccg caaccggagc acatagtcag      60

gtccagctgc agcagtcagg agccgaactg gtgcggcccg gcgcaagtgt caaactgtca     120

tgcaaggcca gcgggtatac cttcacagac tacgagatgc actgggtgaa acagacccct     180

gtgcacggcc tgaagtggat cggcgctctg gacccaaaaa ccggggatac agcatattcc     240

cagaagttta aaggaaaggc cactctgacc gctgacaaga gctcctctac tgcctacatg      300

gagctgagga gcctgacatc cgaagatagc gccgtgtact attgcacccg cttctactcc     360

tatacatact ggggccaggg gactctggtg accgtctctg caggaggagg aggctctgga     420

ggaggaggga gtggaggcgg gggaagcgac gtggtcatga cacagactcc actgtccctg     480

cccgtgagcc tgggcgatca ggctagcatt tcctgtcgaa gttcacagag tctggtgcac     540

tcaaacggaa atacctatct gcattggtac ctgcagaagc caggccagtc tcccaaactg     600

ctgatctata aggtgagcaa ccggttctcc ggggtccctg acagattttc tggaagtggc     660

tcagggacag atttcactct gaaaattagc agagtggagg ccgaagatct gggcgtctac     720

ttttgtagcc agaataccca cgtcccacca acattcggaa gcggcactaa actggaaatc     780

aaggagccca aatcttgtga caaaactcac acatgcccac cgtgcccgga tccgaaagat     840

cccaaatttt gggtgctggt ggtggttggt ggagtcctgg cttgctatag cttgctagta     900

acagtggcct ttattattag agtgaagttc agcaggagcg cagacgcccc cgcgtaccag     960

cagggccaga accagctcta taacgagctc aatctaggac gaagagagga gtacgatgtt    1020

ttggacaaga gacgtggccg ggaccctgag atggggggaa agccgagaag gaagaaccct    1080

caggaaggcc tgtacaatga actgcagaaa gataagatgg cggaggccta cagtgagatt    1140

gggatgaaag gcgagcgccg gaggggcaag gggcacgatg gcctttacca gggtctcagt    1200

acagccacca aggacaccta cgacgccctt cacatgcagg ccctgccccc tcgc          1254


<210>  7
<211>  54
<212>  DNA
<213>  Homo sapiens

<400>  7
gagcccaaat cttgtgacaa aactcacaca tgcccaccgt gcccggatcc gaaa            54


<210>  8
<211>  9
<212>  DNA
<213>  Homo sapiens

<400>  8
gatcccaaa                                                               9


<210>  9

<211>  72
<212>  DNA
<213>  Homo sapiens

<400>  9
ttttgggtgc tggtggtggt tggtggagtc ctggcttgct atagcttgct agtaacagtg      60

gcctttatta tt                                                          72


<210>  10
<211>  459
<212>  PRT
<213>  Homo sapiens

<400>  10

Met Asp Trp Ile Trp Arg Ile Leu Phe Leu Val Gly Ala Ala Thr Gly
1               5                   10                  15

Ala His Ser Gln Val Gln Leu Gln Gln Ser Gly Ala Glu Leu Val Arg
            20                  25                  30

Pro Gly Ala Ser Val Lys Leu Ser Cys Lys Ala Ser Gly Tyr Thr Phe
        35                  40                  45

Thr Asp Tyr Glu Met His Trp Val Lys Gln Thr Pro Val His Gly Leu
    50                  55                  60

Lys Trp Ile Gly Ala Leu Asp Pro Lys Thr Gly Asp Thr Ala Tyr Ser
65                  70                  75                  80

Gln Lys Phe Lys Gly Lys Ala Thr Leu Thr Ala Asp Lys Ser Ser Ser
                85                  90                  95

Thr Ala Tyr Met Glu Leu Arg Ser Leu Thr Ser Glu Asp Ser Ala Val
            100                 105                 110

Tyr Tyr Cys Thr Arg Phe Tyr Ser Tyr Thr Tyr Trp Gly Gln Gly Thr
        115                 120                 125

Leu Val Thr Val Ser Ala Gly Gly Gly Gly Ser Gly Gly Gly Gly Ser
    130                 135                 140

Gly Gly Gly Gly Ser Asp Val Val Met Thr Gln Thr Pro Leu Ser Leu
145                 150                 155                 160

Pro Val Ser Leu Gly Asp Gln Ala Ser Ile Ser Cys Arg Ser Ser Gln
                165                 170                 175

Ser Leu Val His Ser Asn Gly Asn Thr Tyr Leu His Trp Tyr Leu Gln
            180                 185                 190

Lys Pro Gly Gln Ser Pro Lys Leu Leu Ile Tyr Lys Val Ser Asn Arg
        195                 200                 205

Phe Ser Gly Val Pro Asp Arg Phe Ser Gly Ser Gly Ser Gly Thr Asp
    210                 215                 220

Phe Thr Leu Lys Ile Ser Arg Val Glu Ala Glu Asp Leu Gly Val Tyr
225                 230                 235                 240

Phe Cys Ser Gln Asn Thr His Val Pro Pro Thr Phe Gly Ser Gly Thr
                245                 250                 255

Lys Leu Glu Ile Lys Glu Pro Lys Ser Cys Asp Lys Thr His Thr Cys
            260                 265                 270

Pro Pro Cys Pro Asp Pro Lys Phe Trp Val Leu Val Val Val Gly Gly
        275                 280                 285

Val Leu Ala Cys Tyr Ser Leu Leu Val Thr Val Ala Phe Ile Ile Phe
    290                 295                 300

Trp Val Arg Ser Lys Arg Ser Arg Leu Leu His Ser Asp Tyr Met Asn
305                 310                 315                 320

Met Thr Pro Arg Arg Pro Gly Pro Thr Arg Lys His Tyr Gln Pro Tyr
                325                 330                 335

Ala Pro Pro Arg Asp Phe Ala Ala Tyr Arg Ser Arg Val Lys Phe Ser
            340                 345                 350

Arg Ser Ala Asp Ala Pro Ala Tyr Gln Gln Gly Gln Asn Gln Leu Tyr
        355                 360                 365

Asn Glu Leu Asn Leu Gly Arg Arg Glu Glu Tyr Asp Val Leu Asp Lys
    370                 375                 380

Arg Arg Gly Arg Asp Pro Glu Met Gly Gly Lys Pro Arg Arg Lys Asn
385                 390                 395                 400

Pro Gln Glu Gly Leu Tyr Asn Glu Leu Gln Lys Asp Lys Met Ala Glu
                405                 410                 415

Ala Tyr Ser Glu Ile Gly Met Lys Gly Glu Arg Arg Arg Gly Lys Gly
            420                 425                 430

His Asp Gly Leu Tyr Gln Gly Leu Ser Thr Ala Thr Lys Asp Thr Tyr

435                 440                 445

Asp Ala Leu His Met Gln Ala Leu Pro Pro Arg
    450                 455

<210>  11
<211>  68
<212>  PRT
<213>  Homo sapiens

<400>  11

Phe Trp Val Leu Val Val Val Gly Gly Val Leu Ala Cys Tyr Ser Leu
1               5                   10                  15

Leu Val Thr Val Ala Phe Ile Ile Phe Trp Val Arg Ser Lys Arg Ser
            20                  25                  30

Arg Leu Leu His Ser Asp Tyr Met Asn Met Thr Pro Arg Arg Pro Gly
        35                  40                  45

Pro Thr Arg Lys His Tyr Gln Pro Tyr Ala Pro Pro Arg Asp Phe Ala
    50                  55                  60

Ala Tyr Arg Ser
65

<210>  12
<211>  1386
<212>  DNA
<213>  Homo sapiens

<400>  12
atggattgga tttggcgcat tctgtttctg gtgggagccg caaccggagc acatagtcag      60

gtccagctgc agcagtcagg agccgaactg gtgcggcccg gcgcaagtgt caaactgtca     120

tgcaaggcca gcgggtatac cttcacagac tacgagatgc actgggtgaa acagacccct     180

gtgcacggcc tgaagtggat cggcgctctg gacccaaaaa ccggggatac agcatattcc     240

cagaagttta aaggaaaggc cactctgacc gctgacaaga gctcctctac tgcctacatg     300

gagctgagga gcctgacatc cgaagatagc gccgtgtact attgcacccg cttctactcc     360

tatacatact ggggccaggg gactctggtg accgtctctg caggaggagg aggctctgga     420

ggaggaggga gtggaggcgg gggaagcgac gtggtcatga cacagactcc actgtccctg     480

cccgtgagcc tgggcgatca ggctagcatt tcctgtcgaa gttcacagag tctggtgcac     540

tcaaacggaa atacctatct gcattggtac ctgcagaagc caggccagtc tcccaaactg     600

ctgatctata aggtgagcaa ccggttctcc ggggtccctg acagattttc tggaagtggc     660

tcagggacag atttcactct gaaaattagc agagtggagg ccgaagatct gggcgtctac     720

ttttgtagcc agaataccca cgtcccacca acattcggaa gcggcactaa actggaaatc 780

aaggagccca aatcttgtga caaaactcac acatgcccac cgtgcccgga tccgaaagat 840

cccaaatttt gggtgctggt ggtggttggt ggagtcctgg cttgctatag cttgctagta 900

acagtggcct ttattatttt ctgggtgagg agtaagagga gcaggctcct gcacagtgac 960

tacatgaaca tgactccccg ccgccccggg cccacccgca agcattacca gccctatgcc 1020

ccaccacgcg acttcgcagc ctatcgctcc agagtgaagt tcagcaggag cgcagacgcc 1080

cccgcgtacc agcagggcca gaaccagctc tataacgagc tcaatctagg acgaagagag 1140

gagtacgatg ttttggacaa gagacgtggc cgggaccctg agatgggggg aaagccgaga 1200

aggaagaacc ctcaggaagg cctgtacaat gaactgcaga aagataagat ggcggaggcc 1260

tacagtgaga ttgggatgaa aggcgagcgc cggaggggca aggggcacga tggcctttac 1320

cagggtctca gtacagccac caaggacacc tacgacgccc ttcacatgca ggccctgccc 1380

cctcgc 1386

<210> 13
<211> 204
<212> DNA
<213> Homo sapiens

<400> 13
ttttgggtgc tggtggtggt tggtggagtc ctggcttgct atagcttgct agtaacagtg 60

gcctttatta ttttctgggt gaggagtaag aggagcaggc tcctgcacag tgactacatg 120

aacatgactc cccgccgccc cgggcccacc cgcaagcatt accagcccta tgccccacca 180

cgcgacttcg cagcctatcg ctcc 204

<210> 14
<211> 457
<212> PRT
<213> Homo sapiens

<400> 14

Met Asp Trp Ile Trp Arg Ile Leu Phe Leu Val Gly Ala Ala Thr Gly
1               5                   10                  15

Ala His Ser Gln Val Gln Leu Gln Gln Ser Gly Ala Glu Leu Val Arg
            20                  25                  30

Pro Gly Ala Ser Val Lys Leu Ser Cys Lys Ala Ser Gly Tyr Thr Phe
        35                  40                  45

Thr Asp Tyr Glu Met His Trp Val Lys Gln Thr Pro Val His Gly Leu
    50                  55                  60

Lys Trp Ile Gly Ala Leu Asp Pro Lys Thr Gly Asp Thr Ala Tyr Ser
65                  70                  75                  80

Gln Lys Phe Lys Gly Lys Ala Thr Leu Thr Ala Asp Lys Ser Ser Ser
                85                  90                  95

Thr Ala Tyr Met Glu Leu Arg Ser Leu Thr Ser Glu Asp Ser Ala Val
            100                 105                 110

Tyr Tyr Cys Thr Arg Phe Tyr Ser Tyr Thr Tyr Trp Gly Gln Gly Thr
        115                 120                 125

Leu Val Thr Val Ser Ala Gly Gly Gly Gly Ser Gly Gly Gly Gly Ser
    130                 135                 140

Gly Gly Gly Gly Ser Asp Val Val Met Thr Gln Thr Pro Leu Ser Leu
145                 150                 155                 160

Pro Val Ser Leu Gly Asp Gln Ala Ser Ile Ser Cys Arg Ser Ser Gln
                165                 170                 175

Ser Leu Val His Ser Asn Gly Asn Thr Tyr Leu His Trp Tyr Leu Gln
            180                 185                 190

Lys Pro Gly Gln Ser Pro Lys Leu Leu Ile Tyr Lys Val Ser Asn Arg
        195                 200                 205

Phe Ser Gly Val Pro Asp Arg Phe Ser Gly Ser Gly Ser Gly Thr Asp
    210                 215                 220

Phe Thr Leu Lys Ile Ser Arg Val Glu Ala Glu Asp Leu Gly Val Tyr
225                 230                 235                 240

Phe Cys Ser Gln Asn Thr His Val Pro Pro Thr Phe Gly Ser Gly Thr
                245                 250                 255

Lys Leu Glu Ile Lys Glu Pro Lys Ser Cys Asp Lys Thr His Thr Cys
            260                 265                 270

Pro Pro Cys Pro Asp Pro Lys Phe Trp Val Leu Val Val Val Gly Gly
        275                 280                 285

Val Leu Ala Cys Tyr Ser Leu Leu Val Thr Val Ala Phe Ile Ile Lys
    290                 295                 300

Arg Gly Arg Lys Lys Leu Leu Tyr Ile Phe Lys Gln Pro Phe Met Arg
305                 310                 315                 320

Pro Val Gln Thr Thr Gln Glu Glu Asp Gly Cys Ser Cys Arg Phe Pro
                325                 330                 335

Glu Glu Glu Glu Gly Gly Cys Glu Leu Arg Val Lys Phe Ser Arg Ser
            340                 345                 350

Ala Asp Ala Pro Ala Tyr Gln Gln Gly Gln Asn Gln Leu Tyr Asn Glu
        355                 360                 365

Leu Asn Leu Gly Arg Arg Glu Glu Tyr Asp Val Leu Asp Lys Arg Arg
    370                 375                 380

Gly Arg Asp Pro Glu Met Gly Gly Lys Pro Arg Arg Lys Asn Pro Gln
385                 390                 395                 400

Glu Gly Leu Tyr Asn Glu Leu Gln Lys Asp Lys Met Ala Glu Ala Tyr
                405                 410                 415

Ser Glu Ile Gly Met Lys Gly Glu Arg Arg Arg Gly Lys Gly His Asp
            420                 425                 430

Gly Leu Tyr Gln Gly Leu Ser Thr Ala Thr Lys Asp Thr Tyr Asp Ala
        435                 440                 445

Leu His Met Gln Ala Leu Pro Pro Arg
    450                 455

<210> 15
<211> 42
<212> PRT
<213> Homo sapiens

<400> 15

Lys Arg Gly Arg Lys Lys Leu Leu Tyr Ile Phe Lys Gln Pro Phe Met
1               5                   10                  15

Arg Pro Val Gln Thr Thr Gln Glu Glu Asp Gly Cys Ser Cys Arg Phe
            20                  25                  30

Pro Glu Glu Glu Glu Gly Gly Cys Glu Leu
        35                  40

<210> 16
<211> 1380
<212> DNA
<213> Homo sapiens

<400> 16

atggattgga tttggcgcat tctgtttctg gtgggagccg caaccggagc acatagtcag      60

gtccagctgc agcagtcagg agccgaactg gtgcggcccg gcgcaagtgt caaactgtca     120

tgcaaggcca gcgggtatac cttcacagac tacgagatgc actgggtgaa acagacccct     180

gtgcacggcc tgaagtggat cggcgctctg gacccaaaaa ccggggatac agcatattcc     240

cagaagttta aaggaaaggc cactctgacc gctgacaaga gctcctctac tgcctacatg     300

gagctgagga gcctgacatc cgaagatagc gccgtgtact attgcacccg cttctactcc     360

tatacatact ggggccaggg gactctggtg accgtctctg caggaggagg aggctctgga     420

ggaggaggga gtggaggcgg gggaagcgac gtggtcatga cacagactcc actgtccctg     480

cccgtgagcc tgggcgatca ggctagcatt tcctgtcgaa gttcacagag tctggtgcac     540

tcaaacggaa atacctatct gcattggtac ctgcagaagc caggccagtc tcccaaactg     600

ctgatctata aggtgagcaa ccggttctcc ggggtccctg acagattttc tggaagtggc     660

tcagggacag atttcactct gaaaattagc agagtggagg ccgaagatct gggcgtctac     720

ttttgtagcc agaataccca cgtcccacca acattcggaa gcggcactaa actggaaatc     780

aaggagccca aatcttgtga caaaactcac acatgcccac cgtgcccgga tccgaaagat     840

cccaaatttt gggtgctggt ggtggttggt ggagtcctgg cttgctatag cttgctagta     900

acagtggcct ttattattaa acggggcaga aagaaactcc tgtatatatt caaacaacca     960

tttatgagac cagtacaaac tactcaagag gaagatggct gtagctgccg atttccagaa    1020

gaagaagaag gaggatgtga actgagagtg aagttcagca ggagcgcaga cgcccccgcg    1080

taccagcagg gccagaacca gctctataac gagctcaatc taggacgaag agaggagtac    1140

gatgttttgg acaagagacg tggccgggac cctgagatgg ggggaaagcc gagaaggaag    1200

aaccctcagg aaggcctgta caatgaactg cagaaagata agatggcgga ggcctacagt    1260

gagattggga tgaaaggcga gcgccggagg ggcaaggggc acgatggcct ttaccagggt    1320

ctcagtacag ccaccaagga cacctacgac gcccttcaca tgcaggccct gccccctcgc    1380

<210>  17
<211>  126
<212>  DNA
<213>  Homo sapiens

<400>  17
aaacggggca gaaagaaact cctgtatata ttcaaacaac catttatgag accagtacaa      60

actactcaag aggaagatgg ctgtagctgc cgatttccag aagaagaaga aggaggatgt     120

gaactg                                                                126

<210>  18
<211>  501
<212>  PRT

<213>  Homo sapiens

<400>  18

Met Asp Trp Ile Trp Arg Ile Leu Phe Leu Val Gly Ala Ala Thr Gly
1               5                   10                  15

Ala His Ser Gln Val Gln Leu Gln Gln Ser Gly Ala Glu Leu Val Arg
            20                  25                  30

Pro Gly Ala Ser Val Lys Leu Ser Cys Lys Ala Ser Gly Tyr Thr Phe
        35                  40                  45

Thr Asp Tyr Glu Met His Trp Val Lys Gln Thr Pro Val His Gly Leu
    50                  55                  60

Lys Trp Ile Gly Ala Leu Asp Pro Lys Thr Gly Asp Thr Ala Tyr Ser
65                  70                  75                  80

Gln Lys Phe Lys Gly Lys Ala Thr Leu Thr Ala Asp Lys Ser Ser Ser
                85                  90                  95

Thr Ala Tyr Met Glu Leu Arg Ser Leu Thr Ser Glu Asp Ser Ala Val
            100                 105                 110

Tyr Tyr Cys Thr Arg Phe Tyr Ser Tyr Thr Tyr Trp Gly Gln Gly Thr
        115                 120                 125

Leu Val Thr Val Ser Ala Gly Gly Gly Gly Ser Gly Gly Gly Gly Ser
    130                 135                 140

Gly Gly Gly Gly Ser Asp Val Val Met Thr Gln Thr Pro Leu Ser Leu
145                 150                 155                 160

Pro Val Ser Leu Gly Asp Gln Ala Ser Ile Ser Cys Arg Ser Ser Gln
                165                 170                 175

Ser Leu Val His Ser Asn Gly Asn Thr Tyr Leu His Trp Tyr Leu Gln
            180                 185                 190

Lys Pro Gly Gln Ser Pro Lys Leu Leu Ile Tyr Lys Val Ser Asn Arg
        195                 200                 205

Phe Ser Gly Val Pro Asp Arg Phe Ser Gly Ser Gly Ser Gly Thr Asp
    210                 215                 220

Phe Thr Leu Lys Ile Ser Arg Val Glu Ala Glu Asp Leu Gly Val Tyr
225                 230                 235                 240

Phe Cys Ser Gln Asn Thr His Val Pro Pro Thr Phe Gly Ser Gly Thr
                245                 250                 255

Lys Leu Glu Ile Lys Glu Pro Lys Ser Cys Asp Lys Thr His Thr Cys
            260                 265                 270

Pro Pro Cys Pro Asp Pro Lys Phe Trp Val Leu Val Val Val Gly Gly
        275                 280                 285

Val Leu Ala Cys Tyr Ser Leu Leu Val Thr Val Ala Phe Ile Ile Phe
    290                 295                 300

Trp Val Arg Ser Lys Arg Ser Arg Leu Leu His Ser Asp Tyr Met Asn
305                 310                 315                 320

Met Thr Pro Arg Arg Pro Gly Pro Thr Arg Lys His Tyr Gln Pro Tyr
                325                 330                 335

Ala Pro Pro Arg Asp Phe Ala Ala Tyr Arg Ser Lys Arg Gly Arg Lys
            340                 345                 350

Lys Leu Leu Tyr Ile Phe Lys Gln Pro Phe Met Arg Pro Val Gln Thr
        355                 360                 365

Thr Gln Glu Glu Asp Gly Cys Ser Cys Arg Phe Pro Glu Glu Glu Glu
    370                 375                 380

Gly Gly Cys Glu Leu Arg Val Lys Phe Ser Arg Ser Ala Asp Ala Pro
385                 390                 395                 400

Ala Tyr Gln Gln Gly Gln Asn Gln Leu Tyr Asn Glu Leu Asn Leu Gly
                405                 410                 415

Arg Arg Glu Glu Tyr Asp Val Leu Asp Lys Arg Arg Gly Arg Asp Pro
            420                 425                 430

Glu Met Gly Gly Lys Pro Arg Arg Lys Asn Pro Gln Glu Gly Leu Tyr
        435                 440                 445

Asn Glu Leu Gln Lys Asp Lys Met Ala Glu Ala Tyr Ser Glu Ile Gly
    450                 455                 460

Met Lys Gly Glu Arg Arg Arg Gly Lys Gly His Asp Gly Leu Tyr Gln
465                 470                 475                 480

Gly Leu Ser Thr Ala Thr Lys Asp Thr Tyr Asp Ala Leu His Met Gln
                485                 490                 495

Ala Leu Pro Pro Arg
            500

<210>  19
<211>  1512
<212>  DNA
<213>  Homo sapiens

<400>  19
atggattgga tttggcgcat tctgtttctg gtgggagccg caaccggagc acatagtcag      60

gtccagctgc agcagtcagg agccgaactg gtgcggcccg gcgcaagtgt caaactgtca     120

tgcaaggcca gcgggtatac cttcacagac tacgagatgc actgggtgaa acagacccct     180

gtgcacggcc tgaagtggat cggcgctctg gacccaaaaa ccggggatac agcatattcc     240

cagaagttta aaggaaaggc cactctgacc gctgacaaga gctcctctac tgcctacatg     300

gagctgagga gcctgacatc cgaagatagc gccgtgtact attgcacccg cttctactcc     360

tatacatact ggggccaggg gactctggtg accgtctctg caggaggagg aggctctgga     420

ggaggaggga gtggaggcgg gggaagcgac gtggtcatga cacagactcc actgtccctg     480

cccgtgagcc tgggcgatca ggctagcatt tcctgtcgaa gttcacagag tctggtgcac     540

tcaaacggaa atacctatct gcattggtac ctgcagaagc caggccagtc tcccaaactg     600

ctgatctata aggtgagcaa ccggttctcc ggggtccctg acagattttc tggaagtggc     660

tcagggacag atttcactct gaaaattagc agagtggagg ccgaagatct gggcgtctac     720

ttttgtagcc agaataccca cgtcccacca acattcggaa gcggcactaa actggaaatc     780

aaggagccca aatcttgtga caaaactcac acatgcccac cgtgcccgga tccgaaagat     840

cccaaatttt gggtgctggt ggtggttggt ggagtcctgg cttgctatag cttgctagta     900

acagtggcct ttattatttt ctgggtgagg agtaagagga gcaggctcct gcacagtgac     960

tacatgaaca tgactccccg ccgccccggg cccacccgca agcattacca gccctatgcc    1020

ccaccacgcg acttcgcagc ctatcgctcc aaacggggca gaaagaaact cctgtatata    1080

ttcaaacaac catttatgag accagtacaa actactcaag aggaagatgg ctgtagctgc    1140

cgatttccag aagaagaaga aggaggatgt gaactgagag tgaagttcag caggagcgca    1200

gacgcccccg cgtaccagca gggccagaac cagctctata acgagctcaa tctaggacga    1260

agagaggagt acgatgtttt ggacaagaga cgtggccggg accctgagat ggggggaaag    1320

ccgagaagga agaaccctca ggaaggcctg tacaatgaac tgcagaaaga taagatggcg    1380

gaggcctaca gtgagattgg gatgaaaggc gagcgccgga ggggcaaggg gcacgatggc    1440

ctttaccagg gtctcagtac agccaccaag gacacctacg acgcccttca catgcaggcc    1500

ctgccccctc gc                                                         1512

<210>  20
<211>  344
<212>  DNA
<213>  Homo sapiens

<400>  20
caggtccagc tgcagcagtc aggagccgaa ctggtgcggc ccggcgcaag tgtcaaactg      60

tcatgcaagg ccagcgggta taccttcaca gactacgaga tgcactgggt gaaacagacc     120

cctgtgcacg gcctgaagtg gatcggcgct ctggacccaa aaaccgggga tacagcatat     180

tcccagaagt ttaaaggaaa ggccactctg accgctgaca agagctcctc tactgcctac     240

atggagctga ggagcctgac atccgaagat agcgccgtgt actattgcac ccgcttctac     300

tcctatacat actggggcca ggggactctg gtgaccgtct ctgc                      344


<210>  21
<211>  335
<212>  DNA
<213>  Homo sapiens

<400>  21
gacgtggtca tgacacagac tccactgtcc ctgcccgtga gcctgggcga tcaggctagc      60

atttcctgtc gaagttcaca gagtctggtg cactcaaacg gaaataccta tctgcattgg     120

tacctgcaga agccaggcca gtctcccaaa ctgctgatct ataaggtgag caaccggttc     180

tccggggtcc ctgacagatt ttctggaagt ggctcaggga cagatttcac tctgaaaatt     240

agcagagtgg aggccgaaga tctgggcgtc tacttttgta gccagaatac ccacgtccca     300

ccaacattcg gaagcggcac taaactggaa atcaa                                335


<210>  22
<211>  115
<212>  PRT
<213>  Homo sapiens

<400>  22

Gln Val Gln Leu Gln Gln Ser Gly Ala Glu Leu Val Arg Pro Gly Ala
1               5                   10                  15


Ser Val Lys Leu Ser Cys Lys Ala Ser Gly Tyr Thr Phe Thr Asp Tyr
            20                  25                  30


Glu Met His Trp Val Lys Gln Thr Pro Val His Gly Leu Lys Trp Ile
        35                  40                  45


Gly Ala Leu Asp Pro Lys Thr Gly Asp Thr Ala Tyr Ser Gln Lys Phe
    50                  55                  60

Lys Gly Lys Ala Thr Leu Thr Ala Asp Lys Ser Ser Ser Thr Ala Tyr
65                  70                  75                  80

Met Glu Leu Arg Ser Leu Thr Ser Glu Asp Ser Ala Val Tyr Tyr Cys
                85                  90                  95

Thr Arg Phe Tyr Ser Tyr Thr Tyr Trp Gly Gln Gly Thr Leu Val Thr
            100                 105                 110

Val Ser Ala
        115

<210>  23
<211>  112
<212>  PRT
<213>  Homo sapiens

<400>  23

Asp Val Val Met Thr Gln Thr Pro Leu Ser Leu Pro Val Ser Leu Gly
1               5                   10                  15

Asp Gln Ala Ser Ile Ser Cys Arg Ser Ser Gln Ser Leu Val His Ser
            20                  25                  30

Asn Gly Asn Thr Tyr Leu His Trp Tyr Leu Gln Lys Pro Gly Gln Ser
        35                  40                  45

Pro Lys Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Phe Cys Ser Gln Asn
                85                  90                  95

Thr His Val Pro Pro Thr Phe Gly Ser Gly Thr Lys Leu Glu Ile Lys
            100                 105                 110

<210>  24
<211>  242
<212>  PRT
<213>  Homo sapiens

<400>  24

Gln Val Gln Leu Gln Gln Ser Gly Ala Glu Leu Val Arg Pro Gly Ala
1               5                   10                  15

Ser Val Lys Leu Ser Cys Lys Ala Ser Gly Tyr Thr Phe Thr Asp Tyr

20                  25                  30

Glu Met His Trp Val Lys Gln Thr Pro Val His Gly Leu Lys Trp Ile
        35                  40                  45

Gly Ala Leu Asp Pro Lys Thr Gly Asp Thr Ala Tyr Ser Gln Lys Phe
    50                  55                  60

Lys Gly Lys Ala Thr Leu Thr Ala Asp Lys Ser Ser Ser Thr Ala Tyr
65                  70                  75                  80

Met Glu Leu Arg Ser Leu Thr Ser Glu Asp Ser Ala Val Tyr Tyr Cys
                85                  90                  95

Thr Arg Phe Tyr Ser Tyr Thr Tyr Trp Gly Gln Gly Thr Leu Val Thr
            100                 105                 110

Val Ser Ala Gly Gly Gly Gly Ser Gly Gly Gly Gly Ser Gly Gly Gly
        115                 120                 125

Gly Ser Asp Val Val Met Thr Gln Thr Pro Leu Ser Leu Pro Val Ser
    130                 135                 140

Leu Gly Asp Gln Ala Ser Ile Ser Cys Arg Ser Ser Gln Ser Leu Val
145                 150                 155                 160

His Ser Asn Gly Asn Thr Tyr Leu His Trp Tyr Leu Gln Lys Pro Gly
                165                 170                 175

Gln Ser Pro Lys Leu Leu Ile Tyr Lys Val Ser Asn Arg Phe Ser Gly
            180                 185                 190

Val Pro Asp Arg Phe Ser Gly Ser Gly Ser Gly Thr Asp Phe Thr Leu
        195                 200                 205

Lys Ile Ser Arg Val Glu Ala Glu Asp Leu Gly Val Tyr Phe Cys Ser
    210                 215                 220

Gln Asn Thr His Val Pro Pro Thr Phe Gly Ser Gly Thr Lys Leu Glu
225                 230                 235                 240

Ile Lys

<210>  25
<211>  726
<212>  DNA
<213>  Homo sapiens

<400>  25
caggtccagc tgcagcagtc aggagccgaa ctggtgcggc ccggcgcaag tgtcaaactg      60

tcatgcaagg ccagcgggta taccttcaca gactacgaga tgcactgggt gaaacagacc     120

cctgtgcacg gcctgaagtg gatcggcgct ctggacccaa aaaccgggga tacagcatat     180

tcccagaagt ttaaaggaaa ggccactctg accgctgaca agagctcctc tactgcctac     240

atggagctga ggagcctgac atccgaagat agcgccgtgt actattgcac ccgcttctac     300

tcctatacat actggggcca ggggactctg gtgaccgtct ctgcaggagg aggaggctct     360

ggaggaggag ggagtggagg cgggggaagc gacgtggtca tgacacagac tccactgtcc     420

ctgcccgtga gcctgggcga tcaggctagc atttcctgtc gaagttcaca gagtctggtg     480

cactcaaacg gaaataccta tctgcattgg tacctgcaga agccaggcca gtctcccaaa     540

ctgctgatct ataaggtgag caaccggttc tccggggtcc ctgacagatt ttctggaagt     600

ggctcaggga cagatttcac tctgaaaatt agcagagtgg aggccgaaga tctgggcgtc     660

tacttttgta gccagaatac ccacgtccca ccaacattcg gaagcggcac taaactggaa     720

atcaag                                                                726

<210>  26
<211>  396
<212>  PRT
<213>  Homo sapiens

<400>  26

Gln Val Gln Leu Gln Gln Ser Gly Ala Glu Leu Val Arg Pro Gly Ala
1               5                   10                  15

Ser Val Lys Leu Ser Cys Lys Ala Ser Gly Tyr Thr Phe Thr Asp Tyr
            20                  25                  30

Glu Met His Trp Val Lys Gln Thr Pro Val His Gly Leu Lys Trp Ile
        35                  40                  45

Gly Ala Leu Asp Pro Lys Thr Gly Asp Thr Ala Tyr Ser Gln Lys Phe
    50                  55                  60

Lys Gly Lys Ala Thr Leu Thr Ala Asp Lys Ser Ser Ser Thr Ala Tyr
65                  70                  75                  80

Met Glu Leu Arg Ser Leu Thr Ser Glu Asp Ser Ala Val Tyr Tyr Cys
                85                  90                  95

Thr Arg Phe Tyr Ser Tyr Thr Tyr Trp Gly Gln Gly Thr Leu Val Thr
            100                 105                 110

Val Ser Ala Gly Gly Gly Gly Ser Gly Gly Gly Gly Ser Gly Gly Gly
        115                 120                 125

Gly Ser Asp Val Val Met Thr Gln Thr Pro Leu Ser Leu Pro Val Ser
    130                 135                 140

Leu Gly Asp Gln Ala Ser Ile Ser Cys Arg Ser Ser Gln Ser Leu Val
145                 150                 155                 160

His Ser Asn Gly Asn Thr Tyr Leu His Trp Tyr Leu Gln Lys Pro Gly
                165                 170                 175

Gln Ser Pro Lys Leu Leu Ile Tyr Lys Val Ser Asn Arg Phe Ser Gly
            180                 185                 190

Val Pro Asp Arg Phe Ser Gly Ser Gly Ser Gly Thr Asp Phe Thr Leu
        195                 200                 205

Lys Ile Ser Arg Val Glu Ala Glu Asp Leu Gly Val Tyr Phe Cys Ser
    210                 215                 220

Gln Asn Thr His Val Pro Pro Thr Phe Gly Ser Gly Thr Lys Leu Glu
225                 230                 235                 240

Ile Lys Glu Pro Lys Ser Cys Asp Lys Thr His Thr Cys Pro Pro Cys
                245                 250                 255

Pro Asp Pro Lys Phe Trp Val Leu Val Val Val Gly Gly Val Leu Ala
            260                 265                 270

Cys Tyr Ser Leu Leu Val Thr Val Ala Phe Ile Ile Arg Val Lys Phe
        275                 280                 285

Ser Arg Ser Ala Asp Ala Pro Ala Tyr Gln Gln Gly Gln Asn Gln Leu
    290                 295                 300

Tyr Asn Glu Leu Asn Leu Gly Arg Arg Glu Glu Tyr Asp Val Leu Asp
305                 310                 315                 320

Lys Arg Arg Gly Arg Asp Pro Glu Met Gly Gly Lys Pro Arg Arg Lys
                325                 330                 335

Asn Pro Gln Glu Gly Leu Tyr Asn Glu Leu Gln Lys Asp Lys Met Ala
            340                 345                 350

Glu Ala Tyr Ser Glu Ile Gly Met Lys Gly Glu Arg Arg Arg Gly Lys
        355                 360                 365

Gly His Asp Gly Leu Tyr Gln Gly Leu Ser Thr Ala Thr Lys Asp Thr
    370                 375                 380

Tyr Asp Ala Leu His Met Gln Ala Leu Pro Pro Arg
385                 390                 395

<210>  27
<211>  1197
<212>  DNA
<213>  Homo sapiens

<400>  27
caggtccagc tgcagcagtc aggagccgaa ctggtgcggc ccggcgcaag tgtcaaactg      60

tcatgcaagg ccagcgggta taccttcaca gactacgaga tgcactgggt gaaacagacc     120

cctgtgcacg gcctgaagtg gatcggcgct ctggacccaa aaaccgggga tacagcatat     180

tcccagaagt ttaaaggaaa ggccactctg accgctgaca agagctcctc tactgcctac     240

atggagctga ggagcctgac atccgaagat agcgccgtgt actattgcac ccgcttctac     300

tcctatacat actggggcca ggggactctg gtgaccgtct ctgcaggagg aggaggctct     360

ggaggaggag ggagtggagg cgggggaagc gacgtggtca tgacacagac tccactgtcc     420

ctgcccgtga gcctgggcga tcaggctagc atttcctgtc gaagttcaca gagtctggtg     480

cactcaaacg gaaataccta tctgcattgg tacctgcaga agccaggcca gtctcccaaa     540

ctgctgatct ataaggtgag caaccggttc tccggggtcc ctgacagatt ttctggaagt     600

ggctcaggga cagatttcac tctgaaaatt agcagagtgg aggccgaaga tctgggcgtc     660

tacttttgta gccagaatac ccacgtccca ccaacattcg gaagcggcac taaactggaa     720

atcaaggagc ccaaatcttg tgacaaaact cacacatgcc caccgtgccc ggatccgaaa     780

gatcccaaat tttgggtgct ggtggtggtt ggtggagtcc tggcttgcta tagcttgcta     840

gtaacagtgg cctttattat tagagtgaag ttcagcagga gcgcagacgc ccccgcgtac     900

cagcagggcc agaaccagct ctataacgag ctcaatctag gacgaagaga ggagtacgat     960

gttttggaca agagacgtgg ccgggaccct gagatggggg gaaagccgag aaggaagaac    1020

cctcaggaag gcctgtacaa tgaactgcag aaagataaga tggcggaggc ctacagtgag    1080

attgggatga aaggcgagcg ccggaggggc aaggggcacg atggccttta ccagggtctc    1140

agtacagcca ccaaggacac ctacgacgcc cttcacatgc aggccctgcc ccctcgc       1197

<210>  28
<211>  440
<212>  PRT
<213>  Homo sapiens

<400>  28

Gln Val Gln Leu Gln Gln Ser Gly Ala Glu Leu Val Arg Pro Gly Ala
1               5                   10                  15

Ser Val Lys Leu Ser Cys Lys Ala Ser Gly Tyr Thr Phe Thr Asp Tyr
            20                  25                  30

Glu Met His Trp Val Lys Gln Thr Pro Val His Gly Leu Lys Trp Ile
        35                  40                  45

Gly Ala Leu Asp Pro Lys Thr Gly Asp Thr Ala Tyr Ser Gln Lys Phe
    50                  55                  60

Lys Gly Lys Ala Thr Leu Thr Ala Asp Lys Ser Ser Ser Thr Ala Tyr
65                  70                  75                  80

Met Glu Leu Arg Ser Leu Thr Ser Glu Asp Ser Ala Val Tyr Tyr Cys
                85                  90                  95

Thr Arg Phe Tyr Ser Tyr Thr Tyr Trp Gly Gln Gly Thr Leu Val Thr
            100                 105                 110

Val Ser Ala Gly Gly Gly Gly Ser Gly Gly Gly Gly Ser Gly Gly Gly
        115                 120                 125

Gly Ser Asp Val Val Met Thr Gln Thr Pro Leu Ser Leu Pro Val Ser
    130                 135                 140

Leu Gly Asp Gln Ala Ser Ile Ser Cys Arg Ser Ser Gln Ser Leu Val
145                 150                 155                 160

His Ser Asn Gly Asn Thr Tyr Leu His Trp Tyr Leu Gln Lys Pro Gly
                165                 170                 175

Gln Ser Pro Lys Leu Leu Ile Tyr Lys Val Ser Asn Arg Phe Ser Gly
            180                 185                 190

Val Pro Asp Arg Phe Ser Gly Ser Gly Ser Gly Thr Asp Phe Thr Leu
        195                 200                 205

Lys Ile Ser Arg Val Glu Ala Glu Asp Leu Gly Val Tyr Phe Cys Ser
    210                 215                 220

Gln Asn Thr His Val Pro Pro Thr Phe Gly Ser Gly Thr Lys Leu Glu
225                 230                 235                 240

Ile Lys Glu Pro Lys Ser Cys Asp Lys Thr His Thr Cys Pro Pro Cys
                245                 250                 255

Pro Asp Pro Lys Phe Trp Val Leu Val Val Val Gly Gly Val Leu Ala
            260                 265                 270

Cys Tyr Ser Leu Leu Val Thr Val Ala Phe Ile Ile Phe Trp Val Arg
        275                 280                 285

Ser Lys Arg Ser Arg Leu Leu His Ser Asp Tyr Met Asn Met Thr Pro
    290                 295                 300

Arg Arg Pro Gly Pro Thr Arg Lys His Tyr Gln Pro Tyr Ala Pro Pro
305                 310                 315                 320

Arg Asp Phe Ala Ala Tyr Arg Ser Arg Val Lys Phe Ser Arg Ser Ala
                325                 330                 335

Asp Ala Pro Ala Tyr Gln Gln Gly Gln Asn Gln Leu Tyr Asn Glu Leu
            340                 345                 350

Asn Leu Gly Arg Arg Glu Glu Tyr Asp Val Leu Asp Lys Arg Arg Gly
        355                 360                 365

Arg Asp Pro Glu Met Gly Gly Lys Pro Arg Arg Lys Asn Pro Gln Glu
    370                 375                 380

Gly Leu Tyr Asn Glu Leu Gln Lys Asp Lys Met Ala Glu Ala Tyr Ser
385                 390                 395                 400

Glu Ile Gly Met Lys Gly Glu Arg Arg Arg Gly Lys Gly His Asp Gly
                405                 410                 415

Leu Tyr Gln Gly Leu Ser Thr Ala Thr Lys Asp Thr Tyr Asp Ala Leu
            420                 425                 430

His Met Gln Ala Leu Pro Pro Arg
        435                 440

<210>  29
<211>  1329
<212>  DNA
<213>  Homo sapiens

<400>  29
   caggtccagc tgcagcagtc aggagccgaa ctggtgcggc ccggcgcaag tgtcaaactg      60
   tcatgcaagg ccagcgggta taccttcaca gactacgaga tgcactgggt gaaacagacc     120
   cctgtgcacg gcctgaagtg gatcggcgct ctggacccaa aaaccgggga tacagcatat     180
   tcccagaagt ttaaaggaaa ggccactctg accgctgaca agagctcctc tactgcctac     240
   atggagctga ggagcctgac atccgaagat agcgccgtgt actattgcac ccgcttctac 300
   tcctatacat actggggcca ggggactctg gtgaccgtct ctgcaggagg aggaggctct 360
   ggaggaggag ggagtggagg cgggggaagc gacgtggtca tgacacagac tccactgtcc 420
   ctgcccgtga gcctgggcga tcaggctagc atttcctgtc gaagttcaca gagtctggtg 480
   cactcaaacg gaaataccta tctgcattgg tacctgcaga agccaggcca gtctcccaaa 540
   ctgctgatct ataaggtgag caaccggttc tccggggtcc ctgacagatt ttctggaagt 600
   ggctcaggga cagatttcac tctgaaaatt agcagagtgg aggccgaaga tctgggcgtc 660
   tacttttgta gccagaatac ccacgtccca ccaacattcg gaagcggcac taaactggaa 720
   atcaaggagc ccaaatcttg tgacaaaact cacacatgcc caccgtgccc ggatccgaaa 780
   gatcccaaat tttgggtgct ggtggtggtt ggtggagtcc tggcttgcta tagcttgcta 840
   gtaacagtgg cctttattat tttctgggtg aggagtaaga ggagcaggct cctgcacagt 900
   gactacatga acatgactcc ccgccgcccc gggcccaccc gcaagcatta ccagccctat 960
   gccccaccac gcgacttcgc agcctatcgc tccagagtga agttcagcag gagcgcagac 1020
   gcccccgcgt accagcaggg ccagaaccag ctctataacg agctcaatct aggacgaaga 1080
   gaggagtacg atgttttgga caagagacgt ggccgggacc ctgagatggg gggaaagccg 1140
   agaaggaaga accctcagga aggcctgtac aatgaactgc agaaagataa gatggcggag 1200
   gcctacagtg agattgggat gaaaggcgag cgccggaggg gcaaggggca cgatggcctt 1260
   taccagggtc tcagtacagc caccaaggac acctacgacg cccttcacat gcaggccctg 1320
   ccccctcgc 1329

<210> 30
<211> 438
<212> PRT
<213> Homo sapiens

<400> 30

Gln Val Gln Leu Gln Gln Ser Gly Ala Glu Leu Val Arg Pro Gly Ala
1               5                   10                  15

Ser Val Lys Leu Ser Cys Lys Ala Ser Gly Tyr Thr Phe Thr Asp Tyr
            20                  25                  30

Glu Met His Trp Val Lys Gln Thr Pro Val His Gly Leu Lys Trp Ile
        35                  40                  45

Gly Ala Leu Asp Pro Lys Thr Gly Asp Thr Ala Tyr Ser Gln Lys Phe
    50                  55                  60

Lys Gly Lys Ala Thr Leu Thr Ala Asp Lys Ser Ser Ser Thr Ala Tyr
65                  70                  75                  80

Met Glu Leu Arg Ser Leu Thr Ser Glu Asp Ser Ala Val Tyr Tyr Cys
                85                  90                  95

Thr Arg Phe Tyr Ser Tyr Thr Tyr Trp Gly Gln Gly Thr Leu Val Thr
            100                 105                 110

Val Ser Ala Gly Gly Gly Gly Ser Gly Gly Gly Gly Ser Gly Gly Gly
        115                 120                 125

Gly Ser Asp Val Val Met Thr Gln Thr Pro Leu Ser Leu Pro Val Ser
    130                 135                 140

Leu Gly Asp Gln Ala Ser Ile Ser Cys Arg Ser Ser Gln Ser Leu Val
145                 150                 155                 160

His Ser Asn Gly Asn Thr Tyr Leu His Trp Tyr Leu Gln Lys Pro Gly
                165                 170                 175

Gln Ser Pro Lys Leu Leu Ile Tyr Lys Val Ser Asn Arg Phe Ser Gly
            180                 185                 190

Val Pro Asp Arg Phe Ser Gly Ser Gly Ser Gly Thr Asp Phe Thr Leu
        195                 200                 205

Lys Ile Ser Arg Val Glu Ala Glu Asp Leu Gly Val Tyr Phe Cys Ser
    210                 215                 220

Gln Asn Thr His Val Pro Pro Thr Phe Gly Ser Gly Thr Lys Leu Glu
225                 230                 235                 240

Ile Lys Glu Pro Lys Ser Cys Asp Lys Thr His Thr Cys Pro Pro Cys
                245                 250                 255

Pro Asp Pro Lys Phe Trp Val Leu Val Val Val Gly Gly Val Leu Ala
            260                 265                 270

Cys Tyr Ser Leu Leu Val Thr Val Ala Phe Ile Ile Lys Arg Gly Arg
        275                 280                 285

Lys Lys Leu Leu Tyr Ile Phe Lys Gln Pro Phe Met Arg Pro Val Gln
    290                 295                 300

Thr Thr Gln Glu Glu Asp Gly Cys Ser Cys Arg Phe Pro Glu Glu Glu
305                 310                 315                 320

Glu Gly Gly Cys Glu Leu Arg Val Lys Phe Ser Arg Ser Ala Asp Ala
                325                 330                 335

Pro Ala Tyr Gln Gln Gly Gln Asn Gln Leu Tyr Asn Glu Leu Asn Leu
            340                 345                 350

Gly Arg Arg Glu Glu Tyr Asp Val Leu Asp Lys Arg Arg Gly Arg Asp
        355                 360                 365

Pro Glu Met Gly Gly Lys Pro Arg Arg Lys Asn Pro Gln Glu Gly Leu
    370                 375                 380

Tyr Asn Glu Leu Gln Lys Asp Lys Met Ala Glu Ala Tyr Ser Glu Ile
385                 390                 395                 400

Gly Met Lys Gly Glu Arg Arg Arg Gly Lys Gly His Asp Gly Leu Tyr
                405                 410                 415

Gln Gly Leu Ser Thr Ala Thr Lys Asp Thr Tyr Asp Ala Leu His Met
            420                 425                 430

Gln Ala Leu Pro Pro Arg
        435

<210>  31
<211>  1323
<212>  DNA
<213>  Homo sapiens

<400>  31
caggtccagc tgcagcagtc aggagccgaa ctggtgcggc ccggcgcaag tgtcaaactg      60

tcatgcaagg ccagcgggta taccttcaca gactacgaga tgcactgggt gaaacagacc     120

cctgtgcacg gcctgaagtg gatcggcgct ctggacccaa aaaccgggga tacagcatat     180

tcccagaagt ttaaaggaaa ggccactctg accgctgaca agagctcctc tactgcctac     240

atggagctga ggagcctgac atccgaagat agcgccgtgt actattgcac ccgcttctac     300

tcctatacat actggggcca ggggactctg gtgaccgtct ctgcaggagg aggaggctct     360

ggaggaggag ggagtggagg cgggggaagc gacgtggtca tgacacagac tccactgtcc     420

ctgcccgtga gcctgggcga tcaggctagc atttcctgtc gaagttcaca gagtctggtg     480

cactcaaacg gaaataccta tctgcattgg tacctgcaga agccaggcca gtctcccaaa     540

ctgctgatct ataaggtgag caaccggttc tccggggtcc ctgacagatt ttctggaagt     600

ggctcaggga cagatttcac tctgaaaatt agcagagtgg aggccgaaga tctgggcgtc     660

tacttttgta gccagaatac ccacgtccca ccaacattcg gaagcggcac taaactggaa     720

atcaaggagc ccaaatcttg tgacaaaact cacacatgcc caccgtgccc ggatccgaaa 780

gatcccaaat tttgggtgct ggtggtggtt ggtggagtcc tggcttgcta tagcttgcta 840

gtaacagtgg cctttattat taaacggggc agaaagaaac tcctgtatat attcaaacaa 900

ccatttatga gaccagtaca aactactcaa gaggaagatg gctgtagctg ccgatttcca 960

gaagaagaag aaggaggatg tgaactgaga gtgaagttca gcaggagcgc agacgccccc 1020

gcgtaccagc agggccagaa ccagctctat aacgagctca atctaggacg aagagaggag 1080

tacgatgttt tggacaagag acgtggccgg gaccctgaga tggggggaaa gccgagaagg 1140

aagaaccctc aggaaggcct gtacaatgaa ctgcagaaag ataagatggc ggaggcctac 1200

agtgagattg ggatgaaagg cgagcgccgg aggggcaagg ggcacgatgg cctttaccag 1260

ggtctcagta cagccaccaa ggacacctac gacgcccttc acatgcaggc cctgccccct 1320

cgc 1323

<210> 32
<211> 482
<212> PRT
<213> Homo sapiens

<400> 32

Gln Val Gln Leu Gln Gln Ser Gly Ala Glu Leu Val Arg Pro Gly Ala
1               5                   10                  15

Ser Val Lys Leu Ser Cys Lys Ala Ser Gly Tyr Thr Phe Thr Asp Tyr
            20                  25                  30

Glu Met His Trp Val Lys Gln Thr Pro Val His Gly Leu Lys Trp Ile
        35                  40                  45

Gly Ala Leu Asp Pro Lys Thr Gly Asp Thr Ala Tyr Ser Gln Lys Phe
    50                  55                  60

Lys Gly Lys Ala Thr Leu Thr Ala Asp Lys Ser Ser Ser Thr Ala Tyr
65                  70                  75                  80

Met Glu Leu Arg Ser Leu Thr Ser Glu Asp Ser Ala Val Tyr Tyr Cys
                85                  90                  95

Thr Arg Phe Tyr Ser Tyr Thr Tyr Trp Gly Gln Gly Thr Leu Val Thr
            100                 105                 110

Val Ser Ala Gly Gly Gly Gly Ser Gly Gly Gly Gly Ser Gly Gly Gly
        115                 120                 125

Gly Ser Asp Val Val Met Thr Gln Thr Pro Leu Ser Leu Pro Val Ser

130                 135                 140

Leu Gly Asp Gln Ala Ser Ile Ser Cys Arg Ser Ser Gln Ser Leu Val
145                 150                 155                 160

His Ser Asn Gly Asn Thr Tyr Leu His Trp Tyr Leu Gln Lys Pro Gly
                165                 170                 175

Gln Ser Pro Lys Leu Leu Ile Tyr Lys Val Ser Asn Arg Phe Ser Gly
            180                 185                 190

Val Pro Asp Arg Phe Ser Gly Ser Gly Ser Gly Thr Asp Phe Thr Leu
        195                 200                 205

Lys Ile Ser Arg Val Glu Ala Glu Asp Leu Gly Val Tyr Phe Cys Ser
    210                 215                 220

Gln Asn Thr His Val Pro Pro Thr Phe Gly Ser Gly Thr Lys Leu Glu
225                 230                 235                 240

Ile Lys Glu Pro Lys Ser Cys Asp Lys Thr His Thr Cys Pro Pro Cys
                245                 250                 255

Pro Asp Pro Lys Phe Trp Val Leu Val Val Val Gly Gly Val Leu Ala
            260                 265                 270

Cys Tyr Ser Leu Leu Val Thr Val Ala Phe Ile Ile Phe Trp Val Arg
        275                 280                 285

Ser Lys Arg Ser Arg Leu Leu His Ser Asp Tyr Met Asn Met Thr Pro
    290                 295                 300

Arg Arg Pro Gly Pro Thr Arg Lys His Tyr Gln Pro Tyr Ala Pro Pro
305                 310                 315                 320

Arg Asp Phe Ala Ala Tyr Arg Ser Lys Arg Gly Arg Lys Lys Leu Leu
                325                 330                 335

Tyr Ile Phe Lys Gln Pro Phe Met Arg Pro Val Gln Thr Thr Gln Glu
            340                 345                 350

Glu Asp Gly Cys Ser Cys Arg Phe Pro Glu Glu Glu Glu Gly Gly Cys
        355                 360                 365

Glu Leu Arg Val Lys Phe Ser Arg Ser Ala Asp Ala Pro Ala Tyr Gln
    370                 375                 380

Gln Gly Gln Asn Gln Leu Tyr Asn Glu Leu Asn Leu Gly Arg Arg Glu
385                 390                 395                 400

Glu Tyr Asp Val Leu Asp Lys Arg Arg Gly Arg Asp Pro Glu Met Gly
                405                 410                 415

Gly Lys Pro Arg Arg Lys Asn Pro Gln Glu Gly Leu Tyr Asn Glu Leu
            420                 425                 430

Gln Lys Asp Lys Met Ala Glu Ala Tyr Ser Glu Ile Gly Met Lys Gly
        435                 440                 445

Glu Arg Arg Arg Gly Lys Gly His Asp Gly Leu Tyr Gln Gly Leu Ser
    450                 455                 460

Thr Ala Thr Lys Asp Thr Tyr Asp Ala Leu His Met Gln Ala Leu Pro
465                 470                 475                 480

Pro Arg

<210>  33
<211>  1455
<212>  DNA
<213>  Homo sapiens

<400>  33
caggtccagc tgcagcagtc aggagccgaa ctggtgcggc ccggcgcaag tgtcaaactg      60

tcatgcaagg ccagcgggta taccttcaca gactacgaga tgcactgggt gaaacagacc     120

cctgtgcacg gcctgaagtg gatcggcgct ctggacccaa aaaccgggga tacagcatat     180

tcccagaagt ttaaaggaaa ggccactctg accgctgaca agagctcctc tactgcctac     240

atggagctga ggagcctgac atccgaagat agcgccgtgt actattgcac ccgcttctac     300

tcctatacat actggggcca ggggactctg gtgaccgtct ctgcaggagg aggaggctct     360

ggaggaggag ggagtggagg cgggggaagc gacgtggtca tgacacagac tccactgtcc     420

ctgcccgtga gcctgggcga tcaggctagc atttcctgtc gaagttcaca gagtctggtg     480

cactcaaacg gaaataccta tctgcattgg tacctgcaga agccaggcca gtctcccaaa     540

ctgctgatct ataaggtgag caaccggttc tccggggtcc ctgacagatt ttctggaagt     600

ggctcaggga cagatttcac tctgaaaatt agcagagtgg aggccgaaga tctgggcgtc     660

tacttttgta gccagaatac ccacgtccca ccaacattcg gaagcggcac taaactggaa     720

atcaaggagc ccaaatcttg tgacaaaact cacacatgcc caccgtgccc ggatccgaaa     780

gatcccaaat tttgggtgct ggtggtggtt ggtggagtcc tggcttgcta tagcttgcta     840

gtaacagtgg cctttattat tttctgggtg aggagtaaga ggagcaggct cctgcacagt     900

gactacatga acatgactcc ccgccgcccc gggcccaccc gcaagcatta ccagccctat 960

gccccaccac gcgacttcgc agcctatcgc tccaaacggg gcagaaagaa actcctgtat 1020

atattcaaac aaccatttat gagaccagta caaactactc aagaggaaga tggctgtagc 1080

tgccgatttc cagaagaaga agaaggagga tgtgaactga gagtgaagtt cagcaggagc 1140

gcagacgccc ccgcgtacca gcagggccag aaccagctct ataacgagct caatctagga 1200

cgaagagagg agtacgatgt tttggacaag agacgtggcc gggaccctga gatgggggga 1260

aagccgagaa ggaagaaccc tcaggaaggc ctgtacaatg aactgcagaa agataagatg 1320

gcggaggcct acagtgagat tgggatgaaa ggcgagcgcc ggaggggcaa ggggcacgat 1380

ggcctttacc agggtctcag tacagccacc aaggacacct acgacgccct tcacatgcag 1440

gccctgcccc ctcgc 1455

## Description

### CROSS-REFERENCE TO RELATED APPLICATIONS

This application claims priority to U.S. Provisional Patent Application Serial No. 62/055979, filed September 26, 2014, which is incorporated by reference herein in its entirety.

### STATEMENT REGARDING FEDERALLY SPONSORED RESEARCH OR DEVELOPMENT

This invention was made with government support under K12CA090433, R01CA173750, R01CA116548, and R01CA142636, all awarded by National Institutes of Health. The government has certain rights in the invention.

### TECHNICAL FIELD

The present disclosure concerns at least the fields of immunology, cell biology, molecular biology, and medicine.

### BACKGROUND OF THE INVENTION

Immunotherapy with antigen-specific T cells has shown promise in the treatment of malignancies in preclinical models as well as in Phase I/II clinical studies. One attractive strategy to generate tumor-specific T cells is by genetic modification with chimeric antigen receptors (CARs), which comprise an extracellular antigen recognition domain, a transmembrane domain, and an intracellular signaling domain derived from the T-cell receptor CD3-ζ chain often linked to costimulatory molecule endodomains.

There is a continued search for antigens expressed on cancer cells that are not found on normal mature tissues. Glypican-3 (GPC3) is a member of the glypican family, a group of heparan sulfate proteoglycans linked to the cell surface through a glycosyl-phosphatidylinositol anchor. GPC3 is encoded on Xp26 and its defect causes Simson-Golabi-Behmel syndrome Type 1. GPC3 is expressed in a wide variety of tissues during development, but not in mature tissues because of suppression by DNA methylation within the promoter region. GPC3 is detected on 100% of epithelial hepatoblastoma (HB), up to 100% of hepatocellular carcinoma (HCC), 58% of embryonal sarcoma (ES) 100% of atypical teratoid rhabdoid tumors (ATRT), 38-75% of Wilms tumor (WT), 67% of malignant rhabdoid tumors (RT), up to 100% of choriocarcinoma (CC) and up to 100% of yolk sac tumors (YST). Thus, GPC3 is an ideal immunotherapeutic target.

### BRIEF SUMMARY OF THE INVENTION

The present embodiments are directed to methods and/or compositions for the treatment of cancer. In particular cases, the disclosure concerns methods and/or compositions for the treatment of cancers in which the cancer cells comprise GPC3, for example as a tumor antigen. Although in certain aspects the cancer may be of any kind, in particular cases the cancer is hepatoblastoma, hepatocellular carcinoma, malignant rhabdoid tumors, yok sac tumors, undifferentiated sarcoma of the liver, liposarcoma, Wilm's tumor, or choriocarcinoma. In specific embodiments, the cancer comprises solid tumors. In at least some cases, the cancer is not hepatocellular carcinoma.

In one aspect, provided herein are immune cells that express a GPC3-targeting chimeric antigen receptor (CAR) wherein in at least specific embodiments the CAR does not comprise an antigen targeting domain that is, or is derived from, antibodies 3E11, 2G9, 4G5, 3D8, or 2E10. In certain embodiments, the CAR comprises a single chain variable fragment (scFv) specific for GPC3. In a more specific embodiment, the GPC3 CAR comprises an scFv derived from antibody GC33. In another specific embodiment, the GPC3 CAR comprises an scFv that comprises an amino acid sequence that is at least 90%, 91%, 92%, 93%, 94%, 95%, 96%, 97%, 98%, 99%, or 100% identical to the sequence of SEQ ID NO:1. The GPC3 CAR may be encoded by a polynucleotide sequence encoded by SEQ ID NO:2.

The GPC3-specific CARs described herein (which may be referred to as GPC3 CAR) may include one or more costimulatory endodomains, such as CD28, CD27, 4-1BB, OX40, ICOS, or a combination thereof. The CAR may include one or more transmembrane domains, such as one selected from the group consisting of CD3-zeta, CD28, CD8α, CD4, and a combination thereof. In a specific embodiment, the CAR comprises a transmembrane domain derived from CD28 and not from CD8.

In some embodiments, the GPC3-specific CAR provided herein comprises a transmembrane domain selected from the group consisting of CD3-zeta, CD28, CD8α, CD4, or a combination thereof. In a specific embodiment, the CAR comprises a transmembrane domain derived from CD28 and not from CD8. In particular embodiments, the CAR comprises a co-stimulatory molecule endodomain selected from the group consisting of CD28, CD27, 4-1BB, OX40 ICOS, and a combination thereof. In another specific embodiment, the co-stimulatory domain of the GPC3-specific CARs provided herein comprises a CD28 costimulatory domain. In another specific embodiment, the co-stimulatory domain comprises a 4-1BB (CD137) costimulatory domain. In another specific embodiment, the co-stimulatory domain comprises a CD28 costimulatory domain and a 4-1BB costimulatory domain.

In another aspect, provided herein are immune cells expressing a GPC3-specific CAR described herein. In certain embodiments, the immune cells are T cells, NK cells, dendritic cells, NKT cells, or a mixture thereof. In specific embodiments in which the immune cells are T cells, the T cells may be CD4+ T cells, CD8+ T cells, Treg cells, Th1 T cells, Th2 T cells, Th17 T cells, unspecific T cells, or a population of T cells that comprises a combination of any of the foregoing. In certain other embodiments, the GPC3-specific CARs described herein are expressed in other immune cells, including but not limited to, NK cells, NKT cells, γδT cells, or T cells that recognize specific antigens (e.g., viral or other tumor associated antigens) through to their native T-cell receptor. The immune cells may harbor a polynucleotide that encodes the CAR, and the polynucleotide may further comprise a suicide gene. In some embodiments, the immune cells are one of T cells, Natural Killer (NK) cells, Natural Killer T (NKT) cells, γδ-T cells, Mucosa Associated Invariant T cells (MAIT cells), Innate lymphoid cells, dendritic cells, or a mixture thereof. In addition, GPC3-CARs may be expressed in stem and/or progenitor cells that are subsequently differentiated into the aforementioned immune cells. In certain aspects, T cells redirected against GPC3 control the growth of GPC3-expressing cells, including cancer cells, either *in vitro* or *in vivo, e.g.,* in an individual having a cancer comprising tumor cells that express GPC3. The cells are effective against multiple solid tumors, in particular embodiments.

As described in detail herein, the expression of GPC3 is well described in the scientific literature in various types of cancer in extensive panel of tumor arrays and normal tissues and in gene expression profiling datasets. Herein, GPC3 expression was validated in exemplary tested tumor cell lines. A GPC3-specific CAR was generated that showed that when it was expressed by T cells, hepatoblastoma, hepatocellular carcinoma and malignant rhabdoid tumor (as an example) was effectively targeted *in vitro,* and antitumor activity was observed *in vitro* and *in vivo* against hepatocellular carcinoma and malignant rhabdoid tumor, for example. Redirecting effector cells to GPC3 using CARs thus represents a robust platform to target multiple types of solid tumors.

] Because GPC3 protein is expressed by several types of tumors, this antigen is an optimal target for adoptive T-cell immunotherapy based on GPC3-CAR-redirected T cells. In addition, the lack of significant expression of GPC3 in normal tissues further highlights its relevance for immunotherapy.

In another aspect, therefore, provided herein is a method of inhibiting proliferation and/or activity of GPC3-positive cells, such as cancer cells, comprising the step of contacting the cells with a therapeutically effective amount of immune cells that express a CAR that targets GPC3 , *e.g.,* a GPC3-specific CAR as provided herein, wherein the CAR does not comprise an antigen targeting domain that is, or is derived from, antibodies 3E11, 2G9, 4G5, 3D8, or 2E10. In certain embodiments, the cancer is hepatocellular carcinoma, a hepatoblastoma, an embryonal sarcoma, a rhabdoid tumor, a Wilm's tumor, a squamous cell carcinoma of the lung, a liposarcoma, yolk sac tumor, choriocarcinoma, a breast carcinoma, a head and neck squamous cell carcinoma (HNSCC), or mesothelioma. In certain embodiments, the cancer is a GPC3-expressing cancer that is not melanoma. In certain embodiments, the cancer is a GPC3-expressing cancer that is not hepatocellular carcinoma. In some embodiments, the contacting is performed *in vitro,* in cell culture, or *in vivo.* In particular cases, the contacting is performed *in vivo,* and the immune cells are cells in an individual, such as T cells. In particular cases, the immune cells are autologous or allogeneic to the individual. In a related aspect, provided herein is a method of treating an individual who has a GPC3-expressing cancer, comprising administering to the individual a therapeutically-effective amount of immune cells that express a GPC3-specific CAR, *e.g.,* a GPC3-specific CAR as described herein.

In particular embodiments of the methods, the immune cells are T cells, NK cells, dendritic cells, NKT cells, MAIT cells, γδ-T cells, or a mixture thereof. The T cells may be CD4+ T cells, CD8+ T cells, or Treg cells, Th1 T cells, Th2 T cells, Th17 T cells, unspecific T cells, or a population of T cells that comprises a combination of any of the foregoing. In certain other embodiments, the GLC3-specific CARs provided herein are expressed in other immune cells, including but not limited to, NK cells, NKT cells, γδT cells, or T cells that recognize specific antigens (e.g., viral or other tumor associated antigens) through to their native T-cell receptor. The immune cells may harbor a polynucleotide that encodes the CAR, and the polynucleotide may further comprise a suicide gene.

In certain embodiments, GPC3-specific CARs transmit signals to activate immune cells through CD3zeta, CD28, and/or 4-1BB pathways, although the intracellular CAR domain could be readily modified to include other signaling moieties.

In specific methods of the disclosure, an individual who has received GPC3-CAR-expressing immune cells, is receiving, or will receive an additional cancer treatment, such as chemotherapy, immunotherapy, radiation, surgery, hormone therapy, or a combination thereof.

Additional features and advantages of the invention will be described hereinafter which form the subject of the claims of the invention. It should be appreciated by those skilled in the art that the conception and specific embodiment disclosed may be readily utilized as a basis for modifying or designing other structures for carrying out the same purposes of the present invention. It should also be realized by those skilled in the art that such equivalent constructions do not depart from the spirit and scope of the invention as set forth in the appended claims. The novel features which are believed to be characteristic of the invention, both as to its organization and method of operation, together with further objects and advantages will be better understood from the following description when considered in connection with the accompanying figures. It is to be expressly understood, however, that each of the figures is provided for the purpose of illustration and description only and is not intended as a definition of the limits of the present invention.

### BRIEF DESCRIPTION OF THE DRAWINGS

For a more complete understanding of the present invention, reference is now made to the following descriptions taken in conjunction with the accompanying drawing, in which:

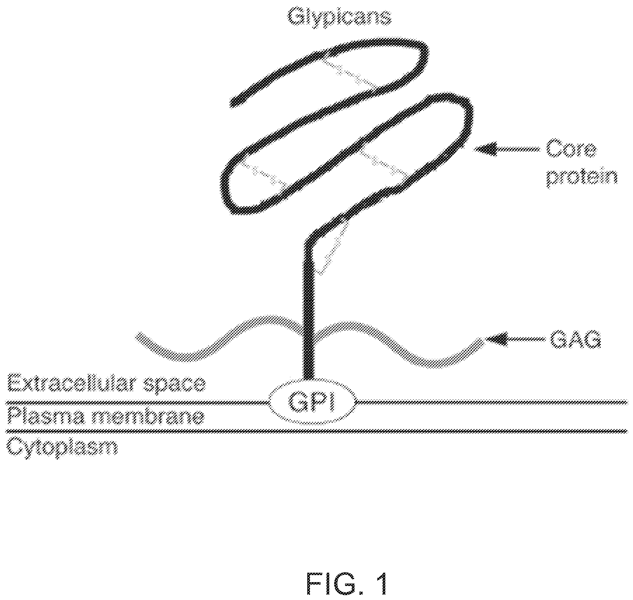

FIG. 1 shows the structure of GPC3;
FIG. 2 illustrates the design of certain GPC3 specific CARs: GPC3-specific scFv was linked with hinge and CD28 transmembrane domain to intracellular signaling endodomains combinations as indicated;

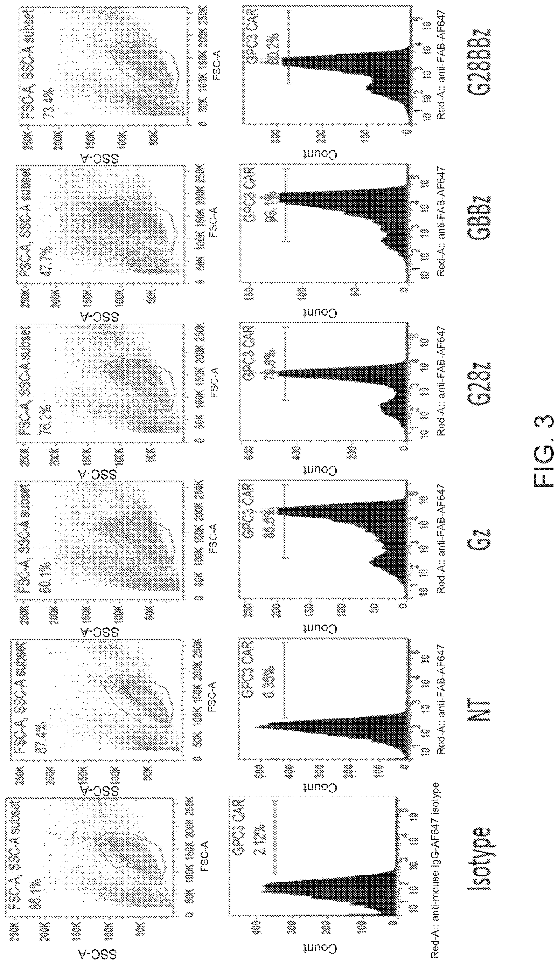

FIG. 3 shows GPC3-CAR expression of T cells: All GPC3-CARs (Gz, G28z, GBBz and G28BBz as shown in FIG. 2) were highly expressed on T cells as detected by FACS analysis. NT: non-transduced T cells;

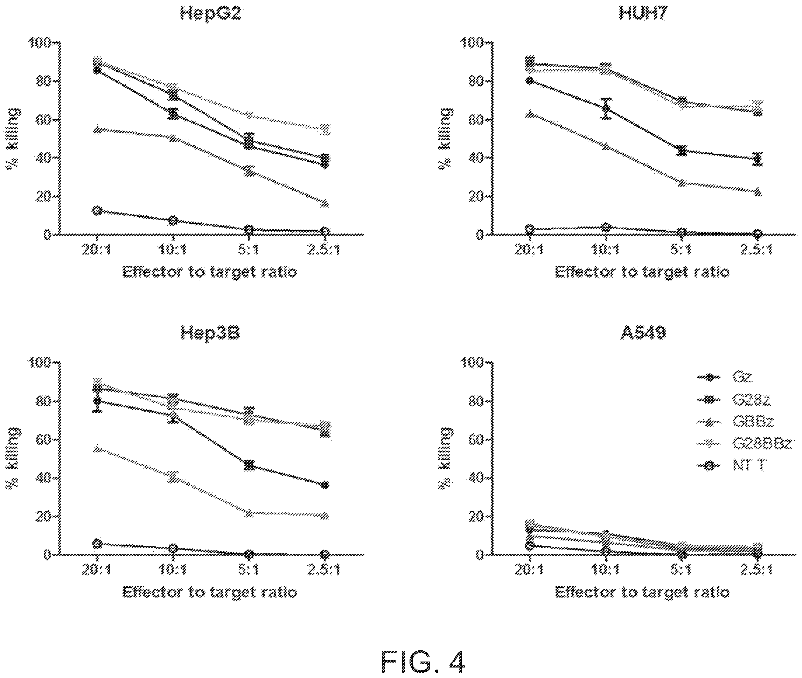

FIG. 4 demonstrates cytotoxicity of GPC3-CAR T cells: % of killing based on Cr⁵¹ release is shown at indicated effector to target ratios for GPC3-CAR T cells and non-transduced T cells. NT: non-transduced T cell. GPC3^{pos} targets: HepG2, HUH7 and Hep3B. GPC3^{neg} target A549;

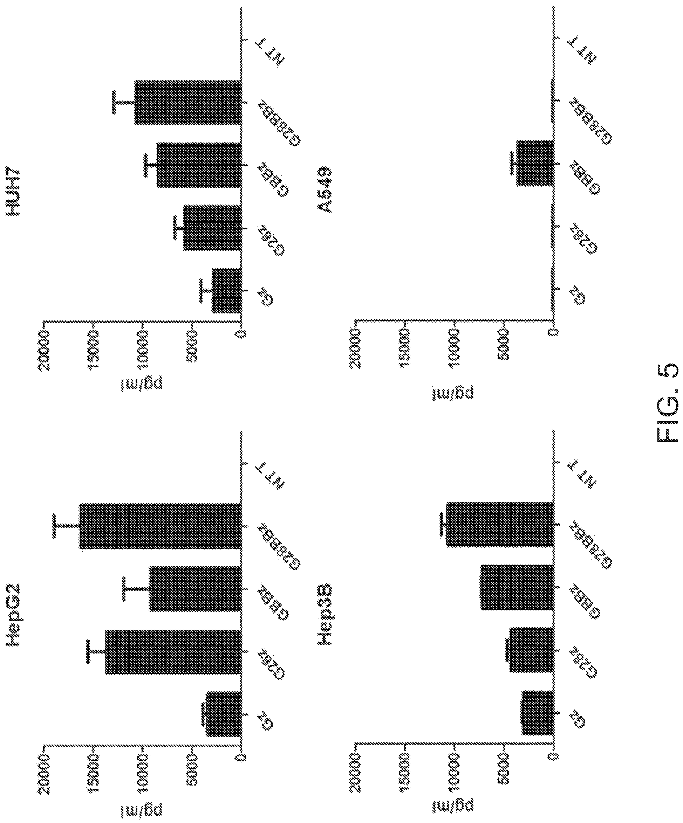

FIG. 5 shows IFNγ release of GPC3-CAR T cells: IFNγ levels in pg/ml are shown of GPC3-CAR T cells after 24 hrs co-culture with GPC3^{pos} and GPC3^{neg} targets. NT: non-transduced T cell. GPC3^{pos} targets: HepG2, HUH7 and Hep3B. GPC3^{neg} target A549;
FIG. 6 shows FACS analysis with a panel of cell lines expressing GPC3-CARs;
FIGS. 7A-7C. FIG. 7A illustrates a variety of representative expression cassettes for GPC3-CARs. GPC3-specific scFv was linked with hinge and CD28 transmembrane domain to intracellular signaling endodomains combinations as indicated. FIG. 7B shows a representative FACS plot for T cells expressing the respective different expression cassettes. FIG. 7C demonstrates CAR expressions of cells from multiple donors;
FIGS. 8A-8E demonstrates cytotoxicity for a variety of GPC3-CAR T cells that killed a variety of GPC3-expressing cells (HepG2 in FIG. 8A; HUH7 in FIG. 8B; Hep3B in FIG. 8C; G401 in FIG. 8D; and G401 in FIG. 8D) but not the control cells that lack expression of GPC3 (8E; A549);
FIGS. 9A-9C show that GPC3-CAR T cells proliferate upon exposure to GPC3^{pos} cells. FIG. 9A show the absolute number of GPC3-CAR T cells. FIG. 9B shows the CFSE dilution of GPC3-CAR T cells 3 days post simulation. FIG. 9C demonstrates 7-AAD staining on day 4 post stimulation;

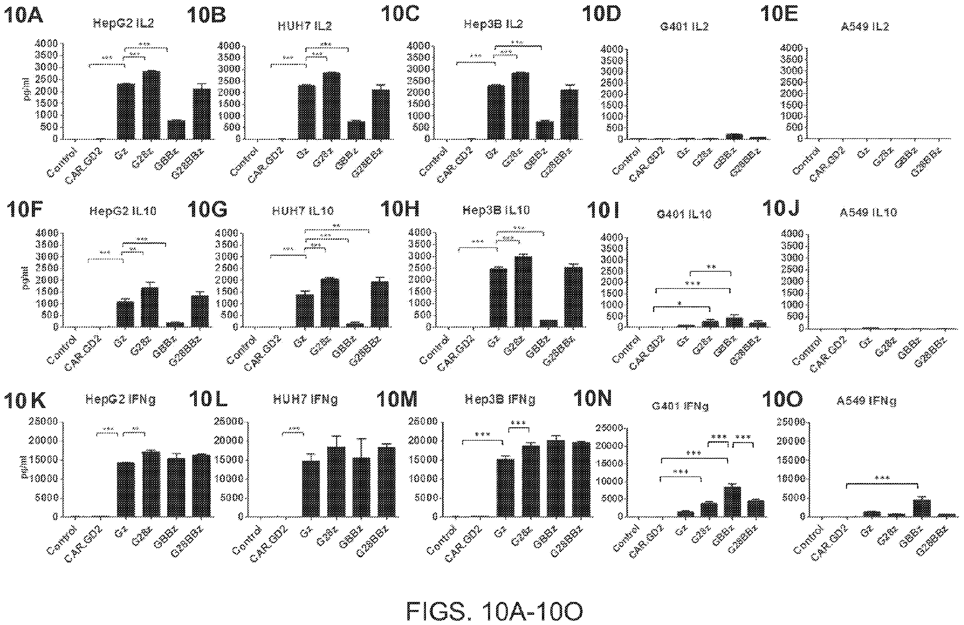

FIGS. 10A-10O demonstrate secretion of cytokines by the GPC3-CAR T cells in the presence of GPC3^{pos} cells. FIGS. 10A, 10B, 10C, 10D and 10E show results for secretion of IL-2 upon co-culture of the T cells with HepG2 cells (10A); HUH7 cells (10B); Hep3B cells (10C); G401 cells (10D); and and control A549 cells (10E). FIGS. 10F, 10G, 10H, 10I, and 10J show results for secretion of IL10 in the presence of HepG2 cells (10F); HUH7 cells (10G); Hep3B cells (10H); G401 (101) and control A549 cells (10J). FIGS. 10K, 10L, 10M, ION, and 100 show results for secretion of IFNγ in the presence of HepG2 cells (10K); HUH7 cells (10L); Hep3B cells (10M); G401 (ION) and control A549 cells (10O)
FIGS. 11A and 11B demonstrate that GPC3-CAR T cells expand after adoptive transfer *in vivo,* as shown by bioluminescence imaging (FIG. 11A) and its quantitation (FIG. 11B).
FIGS. 12A-12C show that GPC3-CAR T cells have antitumor activity in exemplary xenografts *in vivo.* FIG. 12A demonstrates bioluminescence over time and its quantitation is shown in FIG. 12B; FIG. 12C shows percent survival.
FIGS. 13A-13C also show that GPC3-CAR T cells have antitumor activity in exemplary xenografts *in vivo.* Using a smaller dose of the GPC3-CAR T cells in comparison to FIGS. 12A-12C, FIG. 13A demonstrates bioluminescence over time and its quantitation is shown in FIG. 13B; FIG. 13C shows percent survival.
FIGS. 14A-14C show that GPC3-CAR T cells have antitumor activity in second exemplary xenograft model *in vivo.* 14A demonstrates administration schedule and bioluminescence over time with its quantitation is shown in FIG. 14B; FIG. 14C shows percent survival.
FIG. 15. Generation of GPC3 CAR NKT cells. NKTs were isolated from PBMC of 3 healthy donors, stimulated with aGalCer, transduced with GC33.CD28.4-1BB.Z CAR retroviral vector, and expanded in culture with IL-2 for 7 days. The resulting cells were analyzed by FACS using 6B11 (iNKT TCR), anti-CD3, and anti-CAR mAbs

### DETAILED DESCRIPTION OF THE INVENTION

As used herein the specification, "a" or "an" may mean one or more. As used herein in the claim(s), when used in conjunction with the word "comprising", the words "a" or "an" may mean one or more than one. As used herein "another" may mean at least a second or more. In specific embodiments, aspects of the subject matter may "consist essentially of' or "consist of' one or more elements or steps of the subject matter, for example. Some embodiments of the subject matter may consist of or consist essentially of one or more elements, method steps, and/or methods of the subject matter. It is contemplated that any method or composition described herein can be implemented with respect to any other method or composition described herein.

Adoptive transfer of CAR-redirected T lymphocytes represents a useful therapy for patients with malignancies. Here the applicability of this strategy is extended to a broad array of solid tumors by targeting the GPC3 antigen. Particular aspects of the disclosure include methods of treating GPC3-expressing cancers. The cancers may be of any kind, including liver, testicular, lung, ovarian, head and neck cancer, mesothelioma, breast, glioblastoma, kidney, brain, skin, colon, prostate, pancreatic, cervical, thyroid, spleen, or bone cancer, for example. In particular cases, the cancer is hepatoblastoma, hepatocellular carcinoma, malignant rhabdoid tumors, yok sac tumors, undifferentiated sarcoma of the liver, liposarcoma, Wilm's tumor, or choriocarcinoma, although in specific cases the cancer is not hepatocellular carcinoma. In specific embodiments of the disclosure, a particular scFv for GPC3 is employed.

Indicia of successful treatment could be, *e.g.,* detectable reduction in the growth of a tumor (e.g., as seen by MRI or the like), or reduction in one or more symptoms of a cancer or other medical condition that expresses GPC3, including aberrantly expresses GPC3.

GPC3 may also be referred to as OCI-5, SDYS, GTR2-2, SGB, SGBS, SGBS1, MXR7, or DGSX, for example. An example of a GPC3 human nucleotide sequence is L47125 in GenBank® (with corresponding protein sequence in AAA98132 of GenBank®).

### I. Chimeric Antigen Receptors

Genetic engineering of human lymphocytes or other immune cells to express tumor-directed chimeric antigen receptors (CAR) can produce antitumor effector cells that bypass tumor immune escape mechanisms that are due to abnormalities in protein-antigen processing and presentation. Moreover, these transgenic receptors can be directed to tumor-associated antigens that are not protein-derived. In certain embodiments of the disclosure there are cytotoxic T lymphocytes (CTLs) that are modified to comprise a CAR that targets GPC3. In specific embodiments the GPC3 CARs do not include scFvs derived from 3E11, 2G9, 4G5, 3D8, or 2E10. In particular embodiments the scFV is GC33, and in certain embodiments the scFv comprises SEQ ID NO:1.

In particular cases, immune cells include a CAR receptor that is chimeric, non-natural and engineered at least in part by the hand of man. In particular cases, the engineered chimeric antigen receptor (CAR) has one, two, three, four, or more components, and in some embodiments the one or more components facilitate targeting or binding of the T lymphocyte to the GPC3 -comprising cancer cell. In specific embodiments, the CAR comprises an antibody for GPC3, part or all of a cytoplasmic signaling domain, and/or part or all of one or more co-stimulatory molecules, for example endodomains of co-stimulatory molecules. In specific embodiments, the antibody is a scFv.

In certain embodiments, a cytoplasmic signaling domain, such as those derived from the T cell receptor zeta-chain, is employed as at least part of the chimeric receptor in order to produce stimulatory signals for T lymphocyte proliferation and effector function following engagement of the chimeric receptor with the target antigen. Examples would include, but are not limited to, endodomains from co-stimulatory molecules such as CD28, CD27, 4-1BB, ICOS, OX40, a combination thereof, or the signaling components of cytokine receptors such as IL7 and IL15. In particular embodiments, co-stimulatory molecules are employed to enhance the activation, proliferation, and cytotoxicity of T cells produced by the GPC3 CAR after antigen engagement. In specific embodiments, the co-stimulatory molecules are CD28, OX40, and 4-1BB, for example.

The CAR may be first generation, second generation, or third generation (CAR in which signaling is provided by CD3ζ together with co-stimulation provided by CD28 and a tumor necrosis factor receptor (TNFR), such as 4-1BB or OX40), for example. The CAR may be specific for GPC3, and in some embodiments a GPC3-specific CAR-expressing cell may also express a second CAR targeting another antigen, including one or more CARs specific for CD19, CD20, CD22, Kappa or light chain, CD30, CD33, CD123, CD38, ROR1, ErbB2, ErbB3/4, EGFR vIII, carcinoembryonic antigen, EGP2, EGP40, mesothelin, TAG72, PSMA, NKG2D ligands, B7-H6, IL-13 receptor α2, IL-11 receptor R α, MUC1, MUC16, CA9, GD2, GD3, HMW-MAA, CD171, Lewis Y, G250/CAIX, HLA-AI MAGE A1, HLA-A2 NY-ESO-1, PSC1, folate receptor- α, CD44v7/8, 8H9, NCAM, VEGF receptors, 5T4, Fetal AchR, NKG2D ligands, HER2, BCMA, or CD44v6, or other tumor-associated antigens or actionable mutations that are identified through genomic analysis and or differential expression studies of tumors, for example.

In particular cases the CAR is specific for GPC3, and in certain embodiments, the present disclosure provides chimeric T cells specific for GPC3 by joining an extracellular antigen-binding domain derived from a GPC3-specific antibody to cytoplasmic signaling domains derived from the T-cell receptor ζ-chain, optionally with the endodomains of the exemplary costimulatory molecules CD28 and OX40, for examples. This CAR is expressed in human cells, including human T cells, and the targeting of GPC3-positive cancers is encompassed herein.

An example of sequence for a scFv including the GC33 antibody is as follows (where the underlined region is the leader sequence and the remaining sequence is the scFv):

MDWIWRILFLVGAATGAHSQVQLQQSGAELVRPGASVKLSCKASGYTFTDYEM
HWVKQTPVHGLKWIGALDPKTGDTAYSQKFKGKATLTADKSSSTAYMELRSLTSEDSA
VYYCTRFYSYTYWGQGTLVTVSAGGGGSGGGGSGGGGSDVVMTQTPLSLPVSLGDQA
SISCRSSQSLVHSNGNTYLHWYLQKPGQSPKLLIYKVSNRFSGVPDRFSGSGSGTDFTLKI
SRVEAEDLGVYFCSQNTHVPPTFGSGTKLEIK (SEQ ID NO:1)

A scFv sequence without the leader sequence is as follows:

QVQLQQSGAELVRPGASVKLSCKASGYTFTDYEMHWVKQTPVHGLKWIGA
LDPKTGDTAYSQKFKGKATLTADKSSSTAYMELRSLTSEDSAVYYCTRFYSYTYWGQG
TLVTVSAGGGGSGGGGSGGGGSDVVMTQTPLSLPVSLGDQASISCRSSQSLVHSNGNTY
LHWYLQKPGQSPKLLIYKVSNRFSGVPDRFSGSGSGTDFTLKISRVEAEDLGVYFCSQNT
HVPPTFGSGTKLEIK (SEQ ID NO:24)

The nucleotide sequence for the scFv with the leader is as follows (where the underlined region encodes the leader sequence and the remaining sequence encodes the scFv):

ATGGATTGGATTTGGCGCATTCTGTTTCTGGTGGGAGCCGCAACCGGAGC
ACATAGTCAGGTCCAGCTGCAGCAGTCAGGAGCCGAACTGGTGCGGCCCGGCGCAA
GTGTCAAACTGTCATGCAAGGCCAGCGGGTATACCTTCACAGACTACGAGATGCACT
GGGTGAAACAGACCCCTGTGCACGGCCTGAAGTGGATCGGCGCTCTGGACCCAAAA
ACCGGGGATACAGCATATTCCCAGAAGTTTAAAGGAAAGGCCACTCTGACCGCTGA
CAAGAGCTCCTCTACTGCCTACATGGAGCTGAGGAGCCTGACATCCGAAGATAGCG
CCGTGTACTATTGCACCCGCTTCTACTCCTATACATACTGGGGCCAGGGGACTCTGG

TGACCGTCTCTGCAGGAGGAGGAGGCTCTGGAGGAGGAGGGAGTGGAGGCGGGGG
AAGCGACGTGGTCATGACACAGACTCCACTGTCCCTGCCCGTGAGCCTGGGCGATC
AGGCTAGCATTTCCTGTCGAAGTTCACAGAGTCTGGTGCACTCAAACGGAAATACCT
ATCTGCATTGGTACCTGCAGAAGCCAGGCCAGTCTCCCAAACTGCTGATCTATAAGG
TGAGCAACCGGTTCTCCGGGGTCCCTGACAGATTTTCTGGAAGTGGCTCAGGGACAG
ATTTCACTCTGAAAATTAGCAGAGTGGAGGCCGAAGATCTGGGCGTCTACTTTTGTA
GCCAGAATACCCACGTCCCACCAACATTCGGAAGCGGCACTAAACTGGAAATCAAG
(SEQ ID NO:2)

A scFv-encoding nucleotide sequence without the leader sequence is as follows:

CAGGTCCAGCTGCAGCAGTCAGGAGCCGAACTGGTGCGGCCCGGCGCAA
GTGTCAAACTGTCATGCAAGGCCAGCGGGTATACCTTCACAGACTACGAGATGCACT
GGGTGAAACAGACCCCTGTGCACGGCCTGAAGTGGATCGGCGCTCTGGACCCAAAA
ACCGGGGATACAGCATATTCCCAGAAGTTTAAAGGAAAGGCCACTCTGACCGCTGA
CAAGAGCTCCTCTACTGCCTACATGGAGCTGAGGAGCCTGACATCCGAAGATAGCG
CCGTGTACTATTGCACCCGCTTCTACTCCTATACATACTGGGGCCAGGGGACTCTGG
TGACCGTCTCTGCAGGAGGAGGAGGCTCTGGAGGAGGAGGGAGTGGAGGCGGGGG
AAGCGACGTGGTCATGACACAGACTCCACTGTCCCTGCCCGTGAGCCTGGGCGATC
AGGCTAGCATTTCCTGTCGAAGTTCACAGAGTCTGGTGCACTCAAACGGAAATACCT
ATCTGCATTGGTACCTGCAGAAGCCAGGCCAGTCTCCCAAACTGCTGATCTATAAGG
TGAGCAACCGGTTCTCCGGGGTCCCTGACAGATTTTCTGGAAGTGGCTCAGGGACAG
ATTTCACTCTGAAAATTAGCAGAGTGGAGGCCGAAGATCTGGGCGTCTACTTTTGTA
GCCAGAATACCCACGTCCCACCAACATTCGGAAGCGGCACTAAACTGGAAATCAAG
(SEQ ID NO:25)

An example of a GPC3 CAR having the CD28 transmembrane domain (scFvGC33.SH.CD28TM.zeta CAR) is as follows (wherein the single underlined region is the leader and where SH refers to short hinge and TM refers to transmembrane):

MDWIWRILFLVGAATGAHSQVQLQQSGAELVRPGASVKLSCKASGYTFTDY
EMHWVKQTPVHGLKWIGALDPKTGDTAYSQKFKGKATLTADKSSSTAYMELRSLTSE
DSAVYYCTRFYSYTYWGQGTLVTVSAGGGGSGGGGSGGGGSDVVMTQTPLSLPVSLG
DQASISCRSSQSLVHSNGNTYLHWYLQKPGQSPKLLIYKVSNRFSGVPDRFSGSGSGTDF

TLKISRVEAEDLGVYFCSQNTHVPPTFGSGTKLEIKEPKSCDKTHTCPPCPDPKFWVLVV
VGGVLACYSLLVTVAFIIRVKFSRSADAPAYQQGQNQLYNELNLGRREEYDVLDKRRG
RDPEMGGKPRRKNPQEGLYNELQKDKMAEAYSEIGMKGERRRGKGHDGLYQGLSTAT
KDTYDALHMQALPPR (SEQ ID NO:3)

In SEQ ID NO:3, the double underlined region is the zeta signaling domain; EPKSCDKTHTCPPCP (SEQ ID NO:4) is the short hinge; the linker amino acids are DPK; and the CD28 transmembrane domain is FWVLVVVGGVLACYSLLVTVAFII (SEQ ID NO:5).

A scFvGC33.SH.CD28TM.zeta CAR sequence that excludes the leader is as follows:

QVQLQQSGAELVRPGASVKLSCKASGYTFTDYEMHWVKQTPVHGLKWIGA
LDPKTGDTAYSQKFKGKATLTADKSSSTAYMELRSLTSEDSAVYYCTRFYSYTYWGQG
TLVTVSAGGGGSGGGGSGGGGSDVVMTQTPLSLPVSLGDQASISCRSSQSLVHSNGNTY
LHWYLQKPGQSPKLLIYKVSNRFSGVPDRFSGSGSGTDFTLKISRVEAEDLGVYFCSQNT
HVPPTFGSGTKLEIKEPKSCDKTHTCPPCPDPKFWVLVVVGGVLACYSLLVTVAFIIRVK
FSRSADAPAYQQGQNQLYNELNLGRREEYDVLDKRRGRDPEMGGKPRRKNPQEGLYN
ELQKDKMAEAYSEIGMKGERRRGKGHDGLYQGLSTATKDTYDALHMQALPPR (SEQ
ID NO:26)

A corresponding nucleotide sequence for scFvGC33.SH.CD28TM.zeta CAR is as follows (wherein the single underlined region is the leader):

ATGGATTGGATTTGGCGCATTCTGTTTCTGGTGGGAGCCGCAACCGGAGC
ACATAGTCAGGTCCAGCTGCAGCAGTCAGGAGCCGAACTGGTGCGGCCCGGCGCAA
GTGTCAAACTGTCATGCAAGGCCAGCGGGTATACCTTCACAGACTACGAGATGCACT
GGGTGAAACAGACCCCTGTGCACGGCCTGAAGTGGATCGGCGCTCTGGACCCAAAA
ACCGGGGATACAGCATATTCCCAGAAGTTTAAAGGAAAGGCCACTCTGACCGCTGA
CAAGAGCTCCTCTACTGCCTACATGGAGCTGAGGAGCCTGACATCCGAAGATAGCG
CCGTGTACTATTGCACCCGCTTCTACTCCTATACATACTGGGGCCAGGGGACTCTGG
TGACCGTCTCTGCAGGAGGAGGAGGCTCTGGAGGAGGAGGGAGTGGAGGCGGGGG
AAGCGACGTGGTCATGACACAGACTCCACTGTCCCTGCCCGTGAGCCTGGGCGATC
AGGCTAGCATTTCCTGTCGAAGTTCACAGAGTCTGGTGCACTCAAACGGAAATACCT
ATCTGCATTGGTACCTGCAGAAGCCAGGCCAGTCTCCCAAACTGCTGATCTATAAGG

TGAGCAACCGGTTCTCCGGGGTCCCTGACAGATTTCTGGAAGTGGCTCAGGGACAG
ATTTCACTCTGAAAATTAGCAGAGTGGAGGCCGAAGATCTGGGCGTCTACTTTTGTA
GCCAGAATACCCACGTCCCACCAACATTCGGAAGCGGCACTAAACTGGAAATCAAG
GAGCCCAAATCTTGTGACAAAACTCACACATGCCCACCGTGCCCGGATCCGAAAGA
TCCCAAATTTTGGGTGCTGGTGGTGGTTGGTGGAGTCCTGGCTTGCTATAGCTTGCTA
GTAACAGTGGCCTTTATTATTAGAGTGAAGTTCAGCAGGAGCGCAGACGCCCCCGC
GTACCAGCAGGGCCAGAACCAGCTCTATAACGAGCTCAATCTAGGACGAAGAGAGG
AGTACGATGTTTTGGACAAGAGACGTGGCCGGGACCCTGAGATGGGGGGAAAGCCG
AGAAGGAAGAACCCTCAGGAAGGCCTGTACAATGAACTGCAGAAAGATAAGATGG
CGGAGGCCTACAGTGAGATTGGGATGAAAGGCGAGCGCCGGAGGGGCAAGGGGCA
CGATGGCCTTTACCAGGGTCTCAGTACAGCCACCAAGGACACCTACGACGCCCTTCA
CATGCAGGCCCTGCCCCCTCGC (SEQ ID NO:6)

In SEQ ID NO:6, the double underlined region is the zeta signaling domain; the short hinge region is GAGCCCAAATCTTGTGACAAAACTCACACATGCCCACCGTGCCCGGATCCGAAA (SEQ ID NO:7); the linker region is GATCCCAAA (SEQ ID NO:8); and the CD28 transmembrane domain is

TTTTGGGTGCTGGTGGTGGTTGGTGGAGTCCTGGCTTGCTATAGCTTGCTAGTAACA
GTGGCCTTTATTATT (SEQ ID NO:9).

A scFvGC33.SH.CD28TM.zeta CAR polynucleotide sequence without the leader is as follows:

CAGGTCCAGCTGCAGCAGTCAGGAGCCGAACTGGTGCGGCCCGGCGCAA
GTGTCAAACTGTCATGCAAGGCCAGCGGGTATACCTTCACAGACTACGAGATGCACT
GGGTGAAACAGACCCCTGTGCACGGCCTGAAGTGGATCGGCGCTCTGGACCCAAAA
ACCGGGGATACAGCATATTCCCAGAAGTTTAAAGGAAAGGCCACTCTGACCGCTGA
CAAGAGCTCCTCTACTGCCTACATGGAGCTGAGGAGCCTGACATCCGAAGATAGCG
CCGTGTACTATTGCACCCGCTTCTACTCCTATACATACTGGGGCCAGGGGACTCTGG
TGACCGTCTCTGCAGGAGGAGGAGGCTCTGGAGGAGGAGGGAGTGGAGGCGGGGG
AAGCGACGTGGTCATGACACAGACTCCACTGTCCCTGCCCGTGAGCCTGGGCGATC
AGGCTAGCATTTCCTGTCGAAGTTCACAGAGTCTGGTGCACTCAAACGGAAATACCT
ATCTGCATTGGTACCTGCAGAAGCCAGGCCAGTCTCCCAAACTGCTGATCTATAAGG

TGAGCAACCGGTTCTCCGGGGTCCCTGACAGATTTCTGGAAGTGGCTCAGGGACAG
ATTTCACTCTGAAAATTAGCAGAGTGGAGGCCGAAGATCTGGGCGTCTACTTTTGTA
GCCAGAATACCCACGTCCCACCAACATTCGGAAGCGGCACTAAACTGGAAATCAAG
GAGCCCAAATCTTGTGACAAAACTCACACATGCCCACCGTGCCCGGATCCGAAAGA
TCCCAAATTTTGGGTGCTGGTGGTGGTTGGTGGAGTCCTGGCTTGCTATAGCTTGCTA
GTAACAGTGGCCTTTATTATTAGAGTGAAGTTCAGCAGGAGCGCAGACGCCCCCGC
GTACCAGCAGGGCCAGAACCAGCTCTATAACGAGCTCAATCTAGGACGAAGAGAGG
AGTACGATGTTTTGGACAAGAGACGTGGCCGGGACCCTGAGATGGGGGGAAAGCCG
AGAAGGAAGAACCCTCAGGAAGGCCTGTACAATGAACTGCAGAAAGATAAGATGG
CGGAGGCCTACAGTGAGATTGGGATGAAAGGCGAGCGCCGGAGGGGCAAGGGGCA
CGATGGCCTTTACCAGGGTCTCAGTACAGCCACCAAGGACACCTACGACGCCCTTCA
CATGCAGGCCCTGCCCCCTCGC (SEQ ID NO:27)

An example of a GPC3 CAR having the CD28 transmembrane and signaling domain (scFvGC33.SH.CD28.zeta CAR) is as follows (wherein the single underlined region is the leader):

MDWIWRILFLVGAATGAHSQVQLQQSGAELVRPGASVKLSCKASGYTFTDY
EMHWVKQTPVHGLKWIGALDPKTGDTAYSQKFKGKATLTADKSSSTAYMELRSLTSE
DSAVYYCTRFYSYTYWGQGTLVTVSAGGGGSGGGGSGGGGSDVVMTQTPLSLPVSLG
DQASISCRSSQSLVHSNGNTYLHWYLQKPGQSPKLLIYKVSNRFSGVPDRFSGSGSGTDF
TLKISRVEAEDLGVYFCSQNTHVPPTFGSGTKLEIKEPKSCDKTHTCPPCPDPKFWVLVV
VGGVLACYSLLVTVAFIIFWVRSKRSRLLHSDYMNMTPRRPGPTRKHYQPYAPPRDFAA
YRSRVKFSRSADAPAYQQGQNQLYNELNLGRREEYDVLDKRRGRDPEMGGKPRRKNP
QEGLYNELQKDKMAEAYSEIGMKGERRRGKGHDGLYQGLSTATKDTYDALHMQALPP
R (SEQ ID NO:10)

In SEQ ID NO:10, the double underlined region is the zeta signaling domain; SEQ ID NO:4 is the short hinge; the linker amino acids are DPK; and the CD28 transmembrane and signaling domain is

FWVLVVVGGVLACYSLLVTVAFIIFWVRSKRSRLLHSDYMNMTPRRPGPTRKHYQPYA
PPRDFAAYRS (SEQ ID NO:11).

A scFvGC33.SH.CD28.zeta CAR polypeptide sequence without the leader is as follows:

QVQLQQSGAELVRPGASVKLSCKASGYTFTDYEMHWVKQTPVHGLKWIGA
LDPKTGDTAYSQKFKGKATLTADKSSSTAYMELRSLTSEDSAVYYCTRFYSYTYWGQG
TLVTVSAGGGGSGGGGSGGGGSDVVMTQTPLSLPVSLGDQASISCRSSQSLVHSNGNTY
LHWYLQKPGQSPKLLIYKVSNRFSGVPDRFSGSGSGTDFTLKISRVEAEDLGVYFCSQNT
HVPPTFGSGTKLEIKEPKSCDKTHTCPPCPDPKFWVLVVVGGVLACYSLLVTVAFIIFWV
RSKRSRLLHSDYMNMTPRRPGPTRKHYQPYAPPRDFAAYRSRVKFSRSADAPAYQQGQ
NQLYNELNLGRREEYDVLDKRRGRDPEMGGKPRRKNPQEGLYNELQKDKMAEAYSEI
GMKGERRRGKGHDGLYQGLSTATKDTYDALHMQALPPR (SEQ ID NO:28)

A corresponding nucleotide sequence for scFvGC33.SH.CD28.zeta CAR is as follows (where the single underlined region is the leader):

ATGGATTGGATTTGGCGCATTCTGTTTCTGGTGGGAGCCGCAACCGGAGC
ACATAGTCAGGTCCAGCTGCAGCAGTCAGGAGCCGAACTGGTGCGGCCCGGCGCAA
GTGTCAAACTGTCATGCAAGGCCAGCGGGTATACCTTCACAGACTACGAGATGCACT
GGGTGAAACAGACCCCTGTGCACGGCCTGAAGTGGATCGGCGCTCTGGACCCAAAA
ACCGGGGATACAGCATATTCCCAGAAGTTTAAAGGAAAGGCCACTCTGACCGCTGA
CAAGAGCTCCTCTACTGCCTACATGGAGCTGAGGAGCCTGACATCCGAAGATAGCG
CCGTGTACTATTGCACCCGCTTCTACTCCTATACATACTGGGGCCAGGGGACTCTGG
TGACCGTCTCTGCAGGAGGAGGAGGCTCTGGAGGAGGAGGGAGTGGAGGCGGGGG
AAGCGACGTGGTCATGACACAGACTCCACTGTCCCTGCCCGTGAGCCTGGGCGATC
AGGCTAGCATTTCCTGTCGAAGTTCACAGAGTCTGGTGCACTCAAACGGAAATACCT
ATCTGCATTGGTACCTGCAGAAGCCAGGCCAGTCTCCCAAACTGCTGATCTATAAGG
TGAGCAACCGGTTCTCCGGGGTCCCTGACAGATTTTCTGGAAGTGGCTCAGGGACAG
ATTTCACTCTGAAAATTAGCAGAGTGGAGGCCGAAGATCTGGGCGTCTACTTTTGTA
GCCAGAATACCCACGTCCCACCAACATTCGGAAGCGGCACTAAACTGGAAATCAAG
GAGCCCAAATCTTGTGACAAAACTCACACATGCCCACCGTGCCCGGATCCGAAAGA
TCCCAAATTTTGGGTGCTGGTGGTGGTTGGTGGAGTCCTGGCTTGCTATAGCTTGCTA
GTAACAGTGGCCTTTATTATTTTCTGGGTGAGGAGTAAGAGGAGCAGGCTCCTGCAC
AGTGACTACATGAACATGACTCCCCGCCGCCCCGGGCCCACCCGCAAGCATTACCA
GCCCTATGCCCCACCACGCGACTTCGCAGCCTATCGCTCCAGAGTGAAGTTCAGCAG

GAGCGCAGACGCCCCCGCGTACCAGCAGGGCCAGAACCAGCTCTATAACGAGCTCA
ATCTAGGACGAAGAGAGGAGTACGATGTTTTGGACAAGAGACGTGGCCGGGACCCT
GAGATGGGGGGAAAGCCGAGAAGGAAGAACCCTCAGGAAGGCCTGTACAATGAAC
TGCAGAAAGATAAGATGGCGGAGGCCTACAGTGAGATTGGGATGAAAGGCGAGCG
CCGGAGGGGCAAGGGGCACGATGGCCTTTACCAGGGTCTCAGTACAGCCACCAAGG
ACACCTACGACGCCCTTCACATGCAGGCCCTGCCCCCTCGC (SEQ ID NO:12)

In SEQ ID NO:12, the double underlined region is the zeta signaling domain; SEQ ID NO:7 is the short hinge region; the linker region is SEQ ID NO:8; and the CD28 transmembrane and signaling domain is

TTTTGGGTGCTGGTGGTGGTTGGTGGAGTCCTGGCTTGCTATAGCTTGCTAGTAACA
GTGGCCTTTATTATTTCTGGGTGAGGAGTAAGAGGAGCAGGCTCCTGCACAGTGAC
TACATGAACATGACTCCCCGCCGCCCCGGGCCCACCCGCAAGCATTACCAGCCCTAT
GCCCCACCACGCGACTTCGCAGCCTATCGCTCC (SEQ ID NO:13).

A scFvGC33.SH.CD28.zeta CAR polynucleotide sequence without the leader sequence is as follows:

CAGGTCCAGCTGCAGCAGTCAGGAGCCGAACTGGTGCGGCCCGGCGCAA
GTGTCAAACTGTCATGCAAGGCCAGCGGGTATACCTTCACAGACTACGAGATGCACT
GGGTGAAACAGACCCCTGTGCACGGCCTGAAGTGGATCGGCGCTCTGGACCCAAAA
ACCGGGGATACAGCATATTCCCAGAAGTTTAAAGGAAAGGCCACTCTGACCGCTGA
CAAGAGCTCCTCTACTGCCTACATGGAGCTGAGGAGCCTGACATCCGAAGATAGCG
CCGTGTACTATTGCACCCGCTTCTACTCCTATACATACTGGGGCCAGGGGACTCTGG
TGACCGTCTCTGCAGGAGGAGGAGGCTCTGGAGGAGGAGGGAGTGGAGGCGGGGG
AAGCGACGTGGTCATGACACAGACTCCACTGTCCCTGCCCGTGAGCCTGGGCGATC
AGGCTAGCATTTCCTGTCGAAGTTCACAGAGTCTGGTGCACTCAAACGGAAATACCT
ATCTGCATTGGTACCTGCAGAAGCCAGGCCAGTCTCCCAAACTGCTGATCTATAAGG
TGAGCAACCGGTTCTCCGGGGTCCCTGACAGATTTTCTGGAAGTGGCTCAGGGACAG
ATTTCACTCTGAAAATTAGCAGAGTGGAGGCCGAAGATCTGGGCGTCTACTTTTGTA
GCCAGAATACCCACGTCCCACCAACATTCGGAAGCGGCACTAAACTGGAAATCAAG
GAGCCCAAATCTTGTGACAAAACTCACACATGCCCACCGTGCCCGGATCCGAAAGA
TCCCAAATTTTGGGTGCTGGTGGTGGTTGGTGGAGTCCTGGCTTGCTATAGCTTGCTA
GTAACAGTGGCCTTTATTATTTTCTGGGTGAGGAGTAAGAGGAGCAGGCTCCTGCAC

AGTGACTACATGAACATGACTCCCCGCCGCCCCGGGCCCACCCGCAAGCATTACCA
GCCCTATGCCCCACCACGCGACTTCGCAGCCTATCGCTCCAGAGTGAAGTTCAGCAG
GAGCGCAGACGCCCCCGCGTACCAGCAGGGCCAGAACCAGCTCTATAACGAGCTCA
ATCTAGGACGAAGAGAGGAGTACGATGTTTTGGACAAGAGACGTGGCCGGGACCCT
GAGATGGGGGGAAAGCCGAGAAGGAAGAACCCTCAGGAAGGCCTGTACAATGAAC
TGCAGAAAGATAAGATGGCGGAGGCCTACAGTGAGATTGGGATGAAAGGCGAGCG
CCGGAGGGGCAAGGGGCACGATGGCCTTTACCAGGGTCTCAGTACAGCCACCAAGG
ACACCTACGACGCCCTTCACATGCAGGCCCTGCCCCCTCGC (SEQ ID NO:29)

An example of a GPC3 CAR having the CD28 transmembrane domain and the 41BB endodomain (scFvGC33.SH.CD28TM.41BB.zeta CAR) is as follows (where the single underlined region is the leader sequence):

MDWIWRILFLVGAATGAHSQVQLQQSGAELVRPGASVKLSCKASGYTFTDY
EMHWVKQTPVHGLKWIGALDPKTGDTAYSQKFKGKATLTADKSSSTAYMELRSLTSE
DSAVYYCTRFYSYTYWGQGTLVTVSAGGGGSGGGGSGGGGSDVVMTQTPLSLPVSLG
DQASISCRSSQSLVHSNGNTYLHWYLQKPGQSPKLLIYKVSNRFSGVPDRFSGSGSGTDF
TLKISRVEAEDLGVYFCSQNTHVPPTFGSGTKLEIKEPKSCDKTHTCPPCPDPKFWVLVV
VGGVLACYSLLVTVAFIIKRGRKKLLYIFKQPFMRPVQTTQEEDGCSCRFPEEEEGGCEL
RVKFSRSADAPAYQQGQNQLYNELNLGRREEYDVLDKRRGRDPEMGGKPRRKNPQEG
LYNELQKDKMAEAYSEIGMKGERRRGKGHDGLYQGLSTATKDTYDALHMQALPPR
(SEQ ID NO:14)

In SEQ ID NO:14, the double underlined region is the zeta signaling domain; SEQ ID NO:4 is the short hinge; DPK is the linker amino acids; the CD28 transmembrane domain is SEQ ID NO:5; and the 41BB endodomain is KRGRKKLLYIFKQPFMRPVQTTQEEDGCSCRFPEEEEGGCEL (SEQ ID NO:15).

A scFvGC33.SH.CD28TM.41BB.zeta CAR polypeptide sequence without the leader is as follows:

QVQLQQSGAELVRPGASVKLSCKASGYTFTDYEMHWVKQTPVHGLKWIGA
LDPKTGDTAYSQKFKGKATLTADKSSSTAYMELRSLTSEDSAVYYCTRFYSYTYWGQG
TLVTVSAGGGGSGGGGSGGGGSDVVMTQTPLSLPVSLGDQASISCRSSQSLVHSNGNTY
LHWYLQKPGQSPKLLIYKVSNRFSGVPDRFSGSGSGTDFTLKISRVEAEDLGVYFCSQNT

HVPPTFGSGTKLEIKEPKSCDKTHTCPPCPDPKFWVLVVVGGVLACYSLLVTVAFIIKRG
RKKLLYIFKQPFMRPVQTTQEEDGCSCRFPEEEEGGCELRVKFSRSADAPAYQQGQNQL
YNELNLGRREEYDVLDKRRGRDPEMGGKPRRKNPQEGLYNELQKDKMAEAYSEIGMK
GERRRGKGHDGLYQGLSTATKDTYDALHMQALPPR (SEQ ID NO:30)

A corresponding nucleotide sequence for scFvGC33.SH.CD28TM.41BB.zeta CAR is as follows (where the single underlined is the leader sequence):

ATGGATTGGATTTGGCGCATTCTGTTTCTGGTGGGAGCCGCAACCGGAGC
ACATAGTCAGGTCCAGCTGCAGCAGTCAGGAGCCGAACTGGTGCGGCCCGGCGCAA
GTGTCAAACTGTCATGCAAGGCCAGCGGGTATACCTTCACAGACTACGAGATGCACT
GGGTGAAACAGACCCCTGTGCACGGCCTGAAGTGGATCGGCGCTCTGGACCCAAAA
ACCGGGGATACAGCATATTCCCAGAAGTTTAAAGGAAAGGCCACTCTGACCGCTGA
CAAGAGCTCCTCTACTGCCTACATGGAGCTGAGGAGCCTGACATCCGAAGATAGCG
CCGTGTACTATTGCACCCGCTTCTACTCCTATACATACTGGGGCCAGGGACTCTGG
TGACCGTCTCTGCAGGAGGAGGAGGCTCTGGAGGAGGAGGGAGTGGAGGCGGGGG
AAGCGACGTGGTCATGACACAGACTCCACTGTCCCTGCCCGTGAGCCTGGGCGATC
AGGCTAGCATTTCCTGTCGAAGTTCACAGAGTCTGGTGCACTCAAACGGAAATACCT
ATCTGCATTGGTACCTGCAGAAGCCAGGCCAGTCTCCCAAACTGCTGATCTATAAGG
TGAGCAACCGGTTCTCCGGGGTCCCTGACAGATTTTCTGGAAGTGGCTCAGGGACAG
ATTTCACTCTGAAAATTAGCAGAGTGGAGGCCGAAGATCTGGGCGTCTACTTTTGTA
GCCAGAATACCCACGTCCCACCAACATTCGGAAGCGGCACTAAACTGGAAATCAAG
GAGCCCAAATCTTGTGACAAAACTCACACATGCCCACCGTGCCCGGATCCGAAAGA
TCCCAAATTTTGGGTGCTGGTGGTGGTTGGTGGAGTCCTGGCTTGCTATAGCTTGCTA
GTAACAGTGGCCTTTATTATTAAACGGGGCAGAAAGAAACTCCTGTATATATTCAAA
CAACCATTTATGAGACCAGTACAAACTACTCAAGAGGAAGATGGCTGTAGCTGCCG
ATTTCCAGAAGAAGAAGAAGGAGGATGTGAACTGAGAGTGAAGTTCAGCAGGAGC
GCAGACGCCCCCGCGTACCAGCAGGGCCAGAACCAGCTCTATAACGAGCTCAATCT
AGGACGAAGAGAGGAGTACGATGTTTTGGACAAGAGACGTGGCCGGGACCCTGAG
ATGGGGGGAAAGCCGAGAAGGAAGAACCCTCAGGAAGGCCTGTACAATGAACTGC
AGAAAGATAAGATGGCGGAGGCCTACAGTGAGATTGGGATGAAAGGCGAGCGCCG
GAGGGGCAAGGGGCACGATGGCCTTTACCAGGGTCTCAGTACAGCCACCAAGGACA
CCTACGACGCCCTTCACATGCAGGCCCTGCCCCCTCGC (SEQ ID NO:16)

In SEQ ID NO:16, the double underlined is the zeta signaling domain; the short hinge region is SEQ ID NO:7; the linker amino acids are SEQ ID NO:8; the CD28 transmembrane domain is SEQ ID NO:9; and the 41BB endodomain is

AAACGGGGCAGAAAGAAACTCCTGTATATATTCAAACAACCATTTATGAGACCAGT
ACAAACTACTCAAGAGGAAGATGGCTGTAGCTGCCGATTTCCAGAAGAAGAAGAAG
GAGGATGTGAACTG (SEQ ID NO:17).

A scFvGC33.SH.CD28TM.41BB.zeta CAR polynucleotide sequence that excludes the leader sequence is as follows:

CAGGTCCAGCTGCAGCAGTCAGGAGCCGAACTGGTGCGGCCCGGCGCAA
GTGTCAAACTGTCATGCAAGGCCAGCGGGTATACCTTCACAGACTACGAGATGCACT
GGGTGAAACAGACCCCTGTGCACGGCCTGAAGTGGATCGGCGCTCTGGACCCAAAA
ACCGGGGATACAGCATATTCCCAGAAGTTTAAAGGAAAGGCCACTCTGACCGCTGA
CAAGAGCTCCTCTACTGCCTACATGGAGCTGAGGAGCCTGACATCCGAAGATAGCG
CCGTGTACTATTGCACCCGCTTCTACTCCTATACATACTGGGGCCAGGGGACTCTGG
TGACCGTCTCTGCAGGAGGAGGAGGCTCTGGAGGAGGAGGGAGTGGAGGCGGGGG
AAGCGACGTGGTCATGACACAGACTCCACTGTCCCTGCCCGTGAGCCTGGGCGATC
AGGCTAGCATTTCCTGTCGAAGTTCACAGAGTCTGGTGCACTCAAACGGAAATACCT
ATCTGCATTGGTACCTGCAGAAGCCAGGCCAGTCTCCCAAACTGCTGATCTATAAGG
TGAGCAACCGGTTCTCCGGGGTCCCTGACAGATTTTCTGGAAGTGGCTCAGGGACAG
ATTTCACTCTGAAAATTAGCAGAGTGGAGGCCGAAGATCTGGGCGTCTACTTTTGTA
GCCAGAATACCCACGTCCCACCAACATTCGGAAGCGGCACTAAACTGGAAATCAAG
GAGCCCAAATCTTGTGACAAAACTCACACATGCCCACCGTGCCCGGATCCGAAAGA
TCCCAAATTTTGGGTGCTGGTGGTGGTTGGTGGAGTCCTGGCTTGCTATAGCTTGCTA
GTAACAGTGGCCTTTATTATTAAACGGGGCAGAAAGAAACTCCTGTATATATTCAAA
CAACCATTTATGAGACCAGTACAAACTACTCAAGAGGAAGATGGCTGTAGCTGCCG
ATTTCCAGAAGAAGAAGAAGGAGGATGTGAACTGAGAGTGAAGTTCAGCAGGAGC
GCAGACGCCCCCGCGTACCAGCAGGGCCAGAACCAGCTCTATAACGAGCTCAATCT
AGGACGAAGAGAGGAGTACGATGTTTTGGACAAGAGACGTGGCCGGGACCCTGAG
ATGGGGGGAAAGCCGAGAAGGAAGAACCCTCAGGAAGGCCTGTACAATGAACTGC
AGAAAGATAAGATGGCGGAGGCCTACAGTGAGATTGGGATGAAAGGCGAGCGCCG

GAGGGGCAAGGGGCACGATGGCCTTTACCAGGGTCTCAGTACAGCCACCAAGGACA
CCTACGACGCCCTTCACATGCAGGCCCTGCCCCCTCGC (SEQ ID NO:31)

An example of a GPC3 CAR having the CD28 transmembrane and endodomain and the 41BB endodomain (scFvGC33.SH.CD28.41BB.zeta CAR) is as follows (where the single underlined region is the leader sequence):

MDWIWRILFLVGAATGAHSQVQLQQSGAELVRPGASVKLSCKASGYTFTDY
EMHWVKQTPVHGLKWIGALDPKTGDTAYSQKFKGKATLTADKSSSTAYMELRSLTSE
DSAVYYCTRFYSYTYWGQGTLVTVSAGGGGSGGGGSGGGGSDVVMTQTPLSLPVSLG
DQASISCRSSQSLVHSNGNTYLHWYLQKPGQSPKLLIYKVSNRFSGVPDRFSGSGSGTDF
TLKISRVEAEDLGVYFCSQNTHVPPTFGSGTKLEIKEPKSCDKTHTCPPCPDPKFWVLVV
VGGVLACYSLLVTVAFIIFWVRSKRSRLLHSDYMNMTPRRPGPTRKHYQPYAPPRDFAA
YRSKRGRKKLLYIFKQPFMRPVQTTQEEDGCSCRFPEEEEGGCELRVKFSRSADAPAYQ
QGQNQLYNELNLGRREEYDVLDKRRGRDPEMGGKPRRKNPQEGLYNELQKDKMAEA
YSEIGMKGERRRGKGHDGLYQGLSTATKDTYDALHMQALPPR (SEQ ID NO:18)

In SEQ ID NO:18, the zeta signaling domain is double underlined, the short hinge region is SEQ ID NO:4; the CD28 transmembrane and endodomain is SEQ ID NO:11; the linker amino acids are DPK; and the 41BB endodomain is SEQ ID NO:15.

A scFvGC33.SH.CD28.41BB.zeta CAR polypeptide sequence is as follows:

QVQLQQSGAELVRPGASVKLSCKASGYTFTDYEMHWVKQTPVHGLKWIGA
LDPKTGDTAYSQKFKGKATLTADKSSSTAYMELRSLTSEDSAVYYCTRFYSYTYWGQG
TLVTVSAGGGGSGGGGSGGGGSDVVMTQTPLSLPVSLGDQASISCRSSQSLVHSNGNTY
LHWYLQKPGQSPKLLIYKVSNRFSGVPDRFSGSGSGTDFTLKISRVEAEDLGVYFCSQNT
HVPPTFGSGTKLEIKEPKSCDKTHTCPPCPDPKFWVLVVVGGVLACYSLLVTVAFIIFWV
RSKRSRLLHSDYMNMTPRRPGPTRKHYQPYAPPRDFAAYRSKRGRKKLLYIFKQPFMRP
VQTTQEEDGCSCRFPEEEEGGCELRVKFSRSADAPAYQQGQNQLYNELNLGRREEYDV
LDKRRGRDPEMGGKPRRKNPQEGLYNELQKDKMAEAYSEIGMKGERRRGKGHDGLY
QGLSTATKDTYDALHMQALPPR (SEQ ID NO:32)

A corresponding nucleotide sequence for scFvGC33.SH.CD28.41BB.zeta CAR is as follows, wherein the single underlined region is the leader sequence:

ATGGATTGGATTTGGCGCATTCTGTTTCTGGTGGGAGCCGCAACCGGAGC
ACATAGTCAGGTCCAGCTGCAGCAGTCAGGAGCCGAACTGGTGCGGCCCGGCGCAA
GTGTCAAACTGTCATGCAAGGCCAGCGGGTATACCTTCACAGACTACGAGATGCACT
GGGTGAAACAGACCCCTGTGCACGGCCTGAAGTGGATCGGCGCTCTGGACCCAAAA
ACCGGGGATACAGCATATTCCCAGAAGTTTAAAGGAAAGGCCACTCTGACCGCTGA
CAAGAGCTCCTCTACTGCCTACATGGAGCTGAGGAGCCTGACATCCGAAGATAGCG
CCGTGTACTATTGCACCCGCTTCTACTCCTATACATACTGGGGCCAGGGGACTCTGG
TGACCGTCTCTGCAGGAGGAGGAGGCTCTGGAGGAGGAGGGAGTGGAGGCGGGGG
AAGCGACGTGGTCATGACACAGACTCCACTGTCCCTGCCCGTGAGCCTGGGCGATC
AGGCTAGCATTTCCTGTCGAAGTTCACAGAGTCTGGTGCACTCAAACGGAAATACCT
ATCTGCATTGGTACCTGCAGAAGCCAGGCCAGTCTCCCAAACTGCTGATCTATAAGG
TGAGCAACCGGTTCTCCGGGGTCCCTGACAGATTTTCTGGAAGTGGCTCAGGGACAG
ATTTCACTCTGAAAATTAGCAGAGTGGAGGCCGAAGATCTGGGCGTCTACTTTTGTA
GCCAGAATACCCACGTCCCACCAACATTCGGAAGCGGCACTAAACTGGAAATCAAG
GAGCCCAAATCTTGTGACAAAACTCACACATGCCCACCGTGCCCGGATCCGAAAGA
TCCCAAATTTTGGGTGCTGGTGGTGGTTGGTGGAGTCCTGGCTTGCTATAGCTTGCTA
GTAACAGTGGCCTTTATTATTTTCTGGGTGAGGAGTAAGAGGAGCAGGCTCCTGCAC
AGTGACTACATGAACATGACTCCCCGCCGCCCCGGGCCCACCCGCAAGCATTACCA
GCCCTATGCCCCACCACGCGACTTCGCAGCCTATCGCTCCAAACGGGGCAGAAAGA
AACTCCTGTATATATTCAAACAACCATTTATGAGACCAGTACAAACTACTCAAGAGG
AAGATGGCTGTAGCTGCCGATTTCCAGAAGAAGAAGGAGGATGTGAACTGAGA
GTGAAGTTCAGCAGGAGCGCAGACGCCCCCGCGTACCAGCAGGGCCAGAACCAGCT
CTATAACGAGCTCAATCTAGGACGAAGAGAGGAGTACGATGTTTTGGACAAGAGAC
GTGGCCGGGACCCTGAGATGGGGGGAAAGCCGAGAAGGAAGAACCCTCAGGAAGG
CCTGTACAATGAACTGCAGAAAGATAAGATGGCGGAGGCCTACAGTGAGATTGGGA
TGAAAGGCGAGCGCCGGAGGGGCAAGGGGCACGATGGCCTTTACCAGGGTCTCAGT
ACAGCCACCAAGGACACCTACGACGCCCTTCACATGCAGGCCCTGCCCCCTCGC
(SEQ ID NO:19).

In SEQ ID NO:19, the double underlined is the zeta signaling domain; the short hinge region is SEQ ID NO:7; the linker region is SEQ ID NO:8; the CD28 transmembrane domain and endodomain is SEQ ID NO:13; and the 41BB endodomain is SEQ ID NO:17.

A scFvGC33.SH.CD28.41BB.zeta CAR polynucleotide sequence that excludes the leader is as follows:

CAGGTCCAGCTGCAGCAGTCAGGAGCCGAACTGGTGCGGCCCGGCGCAA
GTGTCAAACTGTCATGCAAGGCCAGCGGGTATACCTTCACAGACTACGAGATGCACT
GGGTGAAACAGACCCCTGTGCACGGCCTGAAGTGGATCGGCGCTCTGGACCCAAAA
ACCGGGGATACAGCATATTCCCAGAAGTTTAAAGGAAAGGCCACTCTGACCGCTGA
CAAGAGCTCCTCTACTGCCTACATGGAGCTGAGGAGCCTGACATCCGAAGATAGCG
CCGTGTACTATTGCACCCGCTTCTACTCCTATACATACTGGGGCCAGGGGACTCTGG
TGACCGTCTCTGCAGGAGGAGGAGGCTCTGGAGGAGGAGGGAGTGGAGGCGGGGG
AAGCGACGTGGTCATGACACAGACTCCACTGTCCCTGCCCGTGAGCCTGGGCGATC
AGGCTAGCATTTCCTGTCGAAGTTCACAGAGTCTGGTGCACTCAAACGGAAATACCT
ATCTGCATTGGTACCTGCAGAAGCCAGGCCAGTCTCCCAAACTGCTGATCTATAAGG
TGAGCAACCGGTTCTCCGGGGTCCCTGACAGATTTTCTGGAAGTGGCTCAGGGACAG
ATTTCACTCTGAAAATTAGCAGAGTGGAGGCCGAAGATCTGGGCGTCTACTTTTGTA
GCCAGAATACCCACGTCCCACCAACATTCGGAAGCGGCACTAAACTGGAAATCAAG
GAGCCCAAATCTTGTGACAAAACTCACACATGCCCACCGTGCCCGGATCCGAAAGA
TCCCAAATTTTGGGTGCTGGTGGTGGTTGGTGGAGTCCTGGCTTGCTATAGCTTGCTA
GTAACAGTGGCCTTTATTATTTTCTGGGTGAGGAGTAAGAGGAGCAGGCTCCTGCAC
AGTGACTACATGAACATGACTCCCCGCCGCCCCGGGCCCACCCGCAAGCATTACCA
GCCCTATGCCCCACCACGCGACTTCGCAGCCTATCGCTCCAAACGGGGCAGAAAGA
AACTCCTGTATATATTCAAACAACCATTTATGAGACCAGTACAAACTACTCAAGAGG
AAGATGGCTGTAGCTGCCGATTTCCAGAAGAAGAAGAAGGAGGATGTGAACTGAGA
GTGAAGTTCAGCAGGAGCGCAGACGCCCCCGCGTACCAGCAGGGCCAGAACCAGCT
CTATAACGAGCTCAATCTAGGACGAAGAGAGGAGTACGATGTTTTGGACAAGAGAC
GTGGCCGGGACCCTGAGATGGGGGGAAAGCCGAGAAGGAAGAACCCTCAGGAAGG
CCTGTACAATGAACTGCAGAAAGATAAGATGGCGGAGGCCTACAGTGAGATTGGGA
TGAAAGGCGAGCGCCGGAGGGGCAAGGGGCACGATGGCCTTTACCAGGGTCTCAGT
ACAGCCACCAAGGACACCTACGACGCCCTTCACATGCAGGCCCTGCCCCCTCGC
(SEQ ID NO:33)

In particular embodiments, certain V_{H} and V_{L} sequences are employed in the GC33 compositions of the disclosure, such as follows:
**Nucleotide sequences:**
   V_{H} 
CAGGTCCAGCTGCAGCAGTCAGGAGCCGAACTGGTGCGGCCCGGCGCAA
GTGTCAAACTGTCATGCAAGGCCAGCGGGTATACCTTCACAGACTACGAGATGCACT
GGGTGAAACAGACCCCTGTGCACGGCCTGAAGTGGATCGGCGCTCTGGACCCAAAA
ACCGGGGATACAGCATATTCCCAGAAGTTTAAAGGAAAGGCCACTCTGACCGCTGA
CAAGAGCTCCTCTACTGCCTACATGGAGCTGAGGAGCCTGACATCCGAAGATAGCG
CCGTGTACTATTGCACCCGCTTCTACTCCTATACATACTGGGGCCAGGGGACTCTGG
TGACCGTCTCTGC (SEQ ID NO:20)
 V_{L} 
GACGTGGTCATGACACAGACTCCACTGTCCCTGCCCGTGAGCCTGGGCGA
TCAGGCTAGCATTTCCTGTCGAAGTTCACAGAGTCTGGTGCACTCAAACGGAAATAC
CTATCTGCATTGGTACCTGCAGAAGCCAGGCCAGTCTCCCAAACTGCTGATCTATAA
GGTGAGCAACCGGTTCTCCGGGGTCCCTGACAGATTTTCTGGAAGTGGCTCAGGGAC
AGATTTCACTCTGAAAATTAGCAGAGTGGAGGCCGAAGATCTGGGCGTCTACTTTTG
TAGCCAGAATACCCACGTCCCACCAACATTCGGAAGCGGCACTAAACTGGAAATCA
A (SEQ ID NO:21)
**Amino acid sequences**
   V_{H} 
QVQLQQSGAELVRPGASVKLSCKASGYTFTDYEMHWVKQTPVHGLKWIGA
LDPKTGDTAYSQKFKGKATLTADKSSSTAYMELRSLTSEDSAVYYCTRFYSYTYWGQG
TLVTVSA (SEQ ID NO:22)
 V_{L}

DVVMTQTPLSLPVSLGDQASISCRSSQSLVHSNGNTYLHWYLQKPGQSPKLL
IYKVSNRFSGVPDRFSGSGSGTDFTLKISRVEAEDLGVYFCSQNTHVPPTFGSGTKLEIK
(SEQ ID NO:23)

In specific embodiments, the GPC3-specific CAR comprises sequences that are 70, 71, 72, 73, 74, 75, 76, 77, 78, 79, 80, 81, 82, 83, 84, 85, 86, 87, 88, 89, 90, 91, 92, 93, 94, 95, 96, 97, 98, or 99% identical to at least one of SEQ ID NO:1, 2, 3, 4, 5, 6, 7, 8, 9, 10, 11, 12, 13,14, 15, 16, 17, 18, 19, 20, 21, 22, 23, 24, 25, 26, 27, 28, 29, 30, 31, 32, or 33.

### II. Cells

Cells of the disclosure include immune cells that express a GPC3 CAR. In another aspect, provided herein are immune cells expressing a GPC3-specific CAR described herein. In certain embodiments, the immune cells are T cells, NK cells, dendritic cells, NKT cells, or a mixture thereof. In specific embodiments in which the immune cells are T cells, the T cells may be CD4+ T cells, CD8+ T cells, Treg cells, Th1 T cells, Th2 T cells, Th17 T cells, unspecific T cells, or a population of T cells that comprises a combination of any of the foregoing. In certain other embodiments, the GLC3-specific CARs described herein are expressed in other immune cells, including but not limited to, NK cells, NKT cells, γδT cells, or T cells that recognize specific antigens (e.g., viral or other tumor associated antigens) through to their native T-cell receptor.

As used herein, the terms "cell," "cell line," and "cell culture" may be used interchangeably. All of these terms also include their progeny, which is any and all subsequent generations. It is understood that all progeny may not be identical due to deliberate or inadvertent mutations. In the context of expressing a heterologous nucleic acid sequence, "host cell" refers to a eukaryotic cell that is capable of replicating a vector and/or expressing a heterologous gene encoded by a vector. A host cell can, and has been, used as a recipient for vectors. A host cell may be "transfected" or "transformed," which refers to a process by which exogenous nucleic acid is transferred or introduced into the host cell. A transformed cell includes the primary subject cell and its progeny. As used herein, the terms "engineered" and "recombinant" cells or host cells are intended to refer to a cell into which an exogenous nucleic acid sequence, such as, for example, a vector, has been introduced. Therefore, recombinant cells are distinguishable from naturally occurring cells which do not contain a recombinantly introduced nucleic acid. In embodiments of the disclosure, a host cell is a T cell, including a cytotoxic T cell (also known as TC, Cytotoxic T Lymphocyte, CTL, T-Killer cell, cytolytic T cell, CD8+ T-cells or killer T cell); NK cells and NKT cells are also encompassed in the disclosure.

In certain embodiments, it is contemplated that RNAs or proteinaceous sequences may be co expressed with other selected RNAs or proteinaceous sequences in the same cell, such as the same CTL. Co-expression may be achieved by co-transfecting the CTL with two or more distinct recombinant vectors. Alternatively, a single recombinant vector may be constructed to include multiple distinct coding regions for RNAs, which could then be expressed in CTLs transfected with the single vector.

Some vectors may employ control sequences that allow it to be replicated and/or expressed in both prokaryotic and eukaryotic cells. One of skill in the art would further understand the conditions under which to incubate all of the above described host cells to maintain them and to permit replication of a vector. Also understood and known are techniques and conditions that would allow large-scale production of vectors, as well as production of the nucleic acids encoded by vectors and their cognate polypeptides, proteins, or peptides.

The cells can be autologous cells, syngeneic cells, allogenic cells and even in some cases, xenogeneic cells.

In many situations one may wish to be able to kill the modified CTLs, where one wishes to terminate the treatment, the cells become neoplastic, in research where the absence of the cells after their presence is of interest, or other event, for example. For this purpose one can provide for the expression of certain gene products in which one can kill the modified cells under controlled conditions, such as inducible suicide genes. In certain embodiments, the suicide gene is caspase-9 or HSV thymidine kinase, for example. An inducible suicide gene may be used to reduce the risk of direct toxicity and/or uncontrolled proliferation, for example. In specific aspects, the suicide gene is not immunogenic to the host harboring the polynucleotide or cell. A certain example of a suicide gene that may be used is caspase-9 or caspase-8 or cytosine deaminase. Caspase-9 can be activated using a specific chemical inducer of dimerization (CID), for example. Thymidine kinase-based suicide systems may be utilized.

### III. Illustrative Exemplifications

] By way of illustration, individuals with cancer or at risk for cancer (such as having one or more risk factors) or suspected of having cancer may be treated as follows. Effector lymphocytes such as CTLs modified as described herein may be administered to the individual and retained for extended periods of time. The individual may receive one or more administrations of the cells, and the administrations may or may not occur in conjunction with one or more other cancer therapies. In some embodiments, the genetically modified cells are encapsulated to inhibit immune recognition and placed at the site of the tumor.

In particular cases, an individual is provided with therapeutic CTLs modified to comprise a CAR specific for GPC3 in addition to other types of therapeutic cells. The cells may be delivered at the same time or at different times. The cells may be delivered in the same or separate formulations. The cells may be provided to the individual in separate delivery routes. The cells may be delivered by injection at a tumor site or intravenously or orally, for example. The cells may be delivered systemically or locally. Routine delivery routes for such compositions are known in the art.

### IV. Introduction of Constructs into Immune Cells

Expression vectors that encode the GPC3 CARs can be introduced into cells such as immune cells, including effector immune cells such as CTLs, as a DNA molecule or construct, where there may be at least one marker that will allow for selection of host cells that contain the construct(s). The constructs can be prepared in conventional ways, where the genes and regulatory regions may be isolated, as appropriate, ligated, cloned in an appropriate cloning host, analyzed by restriction or sequencing, or other convenient means. Particularly, using PCR, individual fragments including all or portions of a functional unit may be isolated, where one or more mutations may be introduced using "primer repair", ligation, in vitro mutagenesis, *etc.,* as appropriate. The construct(s) once completed and demonstrated to have the appropriate sequences may then be introduced into the CTL by any convenient means. The constructs may be integrated and packaged into non-replicating, defective viral genomes like Adenovirus, Adeno-associated virus (AAV), or Herpes simplex virus (HSV) or others, including retroviral vectors, for infection or transduction into cells. The constructs may include viral sequences for transfection, if desired. Alternatively, the construct may be introduced by fusion, electroporation, biolistics, transfection, lipofection, or the like. The host cells may be grown and expanded in culture before introduction of the construct(s), followed by the appropriate treatment for introduction of the construct(s) and integration of the construct(s). The cells are then expanded and screened by virtue of a marker present in the construct. Various markers that may be used successfully include hprt, neomycin resistance, thymidine kinase, hygromycin resistance, etc.

In some instances, one may have a target site for homologous recombination, where it is desired that a construct be integrated at a particular locus. For example,) can knockout an endogenous gene and replace it (at the same locus or elsewhere) with the gene encoded for by the construct using materials and methods as are known in the art for homologous recombination. For homologous recombination, one may use either OMEGA or O-vectors. See, for example, Thomas and Capecchi, Cell (1987) 51, 503-512; Mansour, et al., Nature (1988) 336, 348-352; and Joyner, et al., Nature (1989) 338, 153-156.

Vectors containing useful elements such as bacterial or yeast origins of replication, selectable and/or amplifiable markers, promoter/enhancer elements for expression in prokaryotes or eukaryotes, *etc.* that may be used to prepare stocks of construct DNAs and for carrying out transfections are well known in the art, and many are commercially available.

### V. Administration of Cells

The exemplary T cells that have been modified with the construct(s) are then grown in culture under selective conditions and cells that are selected as having the construct may then be expanded and further analyzed, using, for example; the polymerase chain reaction for determining the presence of the construct in the host cells. Once the modified host cells have been identified, they may then be used as planned, e.g. expanded in culture or introduced into a host organism.

Depending upon the nature of the cells, the cells may be introduced into a host organism, e.g. a mammal, in a wide variety of ways. The cells may be introduced at the site of the tumor, in specific embodiments, although in alternative embodiments the cells hone to the cancer or are modified to hone to the cancer. The number of cells that are employed will depend upon a number of circumstances, the purpose for the introduction, the lifetime of the cells, the protocol to be used, for example, the number of administrations, the ability of the cells to multiply, the stability of the recombinant construct, and the like. The cells may be applied as a dispersion, generally being injected at or near the site of interest. The cells may be in a physiologically-acceptable medium.

The DNA introduction need not result in integration in every case. In some situations, transient maintenance of the DNA introduced may be sufficient. In this way, one could have a short term effect, where cells could be introduced into the host and then turned on after a predetermined time, for example, after the cells have been able to home to a particular site.

The cells may be administered as desired. Depending upon the response desired, the manner of administration, the life of the cells, the number of cells present, various protocols may be employed. The number of administrations will depend upon the factors described above at least in part.

It should be appreciated that the system is subject to many variables, such as the cellular response to the ligand, the efficiency of expression and, as appropriate, the level of secretion, the activity of the expression product, the particular need of the patient, which may vary with time and circumstances, the rate of loss of the cellular activity as a result of loss of cells or expression activity of individual cells, and the like. Therefore, it is expected that for each individual patient, even if there were universal cells which could be administered to the population at large, each patient would be monitored for the proper dosage for the individual, and such practices of monitoring a patient are routine in the art

### VI. Nucleic Acid-Based Expression Systems

A polynucleotide encoding the GPC3 CAR and optionally a suicide gene may comprise an expression vector

### A. Vectors

The term "vector" is used to refer to a carrier nucleic acid molecule into which a nucleic acid sequence can be inserted for introduction into a cell where it can be replicated. A nucleic acid sequence can be "exogenous," which means that it is foreign to the cell into which the vector is being introduced or that the sequence is homologous to a sequence in the cell but in a position within the host cell nucleic acid in which the sequence is ordinarily not found. Vectors include plasmids, cosmids, viruses (bacteriophage, animal viruses, and plant viruses), and artificial chromosomes (e.g., YACs). One of skill in the art would be well equipped to construct a vector through standard recombinant techniques (see, for example, Maniatis *et al.,* 1988 and Ausubel *et al.,* 1994, both incorporated herein by reference).

The term "expression vector" refers to any type of genetic construct comprising a nucleic acid coding for a RNA capable of being transcribed. In some cases, RNA molecules are then translated into a protein, polypeptide, or peptide. In other cases, these sequences are not translated, for example, in the production of antisense molecules or ribozymes. Expression vectors can contain a variety of "control sequences," which refer to nucleic acid sequences necessary for the transcription and possibly translation of an operably linked coding sequence in a particular host cell. In addition to control sequences that govern transcription and translation, vectors and expression vectors may contain nucleic acid sequences that serve other functions as well and are described infra.

### B. Promoters and Enhancers

A "promoter" is a control sequence that is a region of a nucleic acid sequence at which initiation and rate of transcription are controlled. It may contain genetic elements at which regulatory proteins and molecules may bind, such as RNA polymerase and other transcription factors, to initiate the specific transcription a nucleic acid sequence. The phrases "operatively positioned," "operatively linked," "under control," and "under transcriptional control" mean that a promoter is in a correct functional location and/or orientation in relation to a nucleic acid sequence to control transcriptional initiation and/or expression of that sequence.

A promoter generally comprises a sequence that functions to position the start site for RNA synthesis. The best known example of this is the TATA box, but in some promoters lacking a TATA box, such as, for example, the promoter for the mammalian terminal deoxynucleotidyl transferase gene and the promoter for the SV40 late genes, a discrete element overlying the start site itself helps to fix the place of initiation. Additional promoter elements regulate the frequency of transcriptional initiation. Typically, these are located in the region 30 110 bp upstream of the start site, although a number of promoters have been shown to contain functional elements downstream of the start site as well. To bring a coding sequence "under the control of" a promoter, one positions the 5' end of the transcription initiation site of the transcriptional reading frame "downstream" of (i.e., 3' of) the chosen promoter. The "upstream" promoter stimulates transcription of the DNA and promotes expression of the encoded RNA.

The spacing between promoter elements frequently is flexible, so that promoter function is preserved when elements are inverted or moved relative to one another. In the tk promoter, the spacing between promoter elements can be increased to 50 bp apart before activity begins to decline. Depending on the promoter, it appears that individual elements can function either cooperatively or independently to activate transcription. A promoter may or may not be used in conjunction with an "enhancer," which refers to a cis-acting regulatory sequence involved in the transcriptional activation of a nucleic acid sequence.

A promoter may be one naturally associated with a nucleic acid sequence, as may be obtained by isolating the 5' non-coding sequences located upstream of the coding segment and/or exon. Such a promoter can be referred to as "endogenous." Similarly, an enhancer may be one naturally associated with a nucleic acid sequence, located either downstream or upstream of that sequence. Alternatively, certain advantages will be gained by positioning the coding nucleic acid segment under the control of a recombinant or heterologous promoter, which refers to a promoter that is not normally associated with a nucleic acid sequence in its natural environment. A recombinant or heterologous enhancer refers also to an enhancer not normally associated with a nucleic acid sequence in its natural environment. Such promoters or enhancers may include promoters or enhancers of other genes, and promoters or enhancers isolated from any other virus, or prokaryotic or eukaryotic cell, and promoters or enhancers not "naturally occurring," i.e., containing different elements of different transcriptional regulatory regions, and/or mutations that alter expression. For example, promoters that are most commonly used in recombinant DNA construction include the beta-lactamase (penicillinase), lactose and tryptophan (trp) promoter systems. In addition to producing nucleic acid sequences of promoters and enhancers synthetically, sequences may be produced using recombinant cloning and/or nucleic acid amplification technology, including PCR™, in connection with the compositions disclosed herein (see U.S. Patent Nos. 4,683,202 and 5,928,906, each incorporated herein by reference). Furthermore, it is contemplated the control sequences that direct transcription and/or expression of sequences within non-nuclear organelles such as mitochondria, chloroplasts, and the like, can be employed as well.

Naturally, it will be important to employ a promoter and/or enhancer that effectively directs the expression of the DNA segment in the organelle, cell type, tissue, organ, or organism chosen for expression. Those of skill in the art of molecular biology generally know the use of promoters, enhancers, and cell type combinations for protein expression, (see, for example Sambrook *et al.* 1989, incorporated herein by reference). The promoters employed may be constitutive, tissue-specific, inducible, and/or useful under the appropriate conditions to direct high level expression of the introduced DNA segment, such as is advantageous in the large-scale production of recombinant proteins and/or peptides. The promoter may be heterologous or endogenous.

Additionally any promoter/enhancer combination could also be used to drive expression. Use of a T3, T7 or SP6 cytoplasmic expression system is another possible embodiment. Eukaryotic cells can support cytoplasmic transcription from certain bacterial promoters if the appropriate bacterial polymerase is provided, either as part of the delivery complex or as an additional genetic expression construct.

The identity of tissue-specific promoters or elements, as well as assays to characterize their activity, is well known to those of skill in the art.

A specific initiation signal also may be required for efficient translation of coding sequences. These signals include the ATG initiation codon or adjacent sequences. Exogenous translational control signals, including the ATG initiation codon, may need to be provided. One of ordinary skill in the art would readily be capable of determining this and providing the necessary signals.

In certain embodiments, the expression of the GPC3-CAR is modulated upon exposure of a corresponding regulatory sequence to one or more factors. In specific embodiments, the expression is modulated upon exposure to tumor-associated factors. Illustrative examples of tumor-associated factors include factors present in hypoxic tissue. In some embodiments, the factors are cytokines and/or chemokines. For example, hypoxia induces the expression of HIF-1α, a transcription factor that could induce engager expression that is under the control of a hypoxia response element (HRE). Hypoxia could also stabilize engager molecules that contain an oxygen-dependent degradation domain (ODDD). Another example of a substance, which is produced by tumor cells and could regulate engager gene expression, is lactic acid. A specific initiation signal also may be required for efficient translation of coding sequences. These signals include the ATG initiation codon or adjacent sequences. Exogenous translational control signals, including the ATG initiation codon, may need to be provided. One of ordinary skill in the art would readily be capable of determining this and providing the necessary signals.

In certain embodiments, the GPC3-CAR may be expressed in two fragments that are inactive without the addition of an exogenous substance. For example, but not limited to, the GPC3-specific ectodomain and the transmembrane domain of the CAR and the cytoplasmic domain of the CAR could be each linked to a heterodimerizer domain (Exto-TM-HD; Cyto-HD). Expression of Exto-TM-HD and Cyto-HD in cells would result in an inactive GPC3-CAR unless a small molecule is added that links Exto-TM-HD and Cyto-HD, allowing for pharmacological control of GPC3-CAR activity.

In certain embodiments of the disclosure, the use of internal ribosome entry sites (IRES) elements are used to create multigene, or polycistronic, messages, and these may be used in the disclosure.

In certain embodiments 2A sequences are used to create multigene messages, and these may be used in the embodiments.

Vectors can include a multiple cloning site (MCS), which is a nucleic acid region that contains multiple restriction enzyme sites, any of which can be used in conjunction with standard recombinant technology to digest the vector. "Restriction enzyme digestion" refers to catalytic cleavage of a nucleic acid molecule with an enzyme that functions only at specific locations in a nucleic acid molecule. Many of these restriction enzymes are commercially available. Use of such enzymes is widely understood by those of skill in the art. Frequently, a vector is linearized or fragmented using a restriction enzyme that cuts within the MCS to enable exogenous sequences to be ligated to the vector. "Ligation" refers to the process of forming phosphodiester bonds between two nucleic acid fragments, which may or may not be contiguous with each other. Techniques involving restriction enzymes and ligation reactions are well known to those of skill in the art of recombinant technology.

Splicing sites, termination signals, origins of replication, and selectable markers may also be employed.

### C. Plasmid Vectors

In certain embodiments, a plasmid vector is contemplated for use to transform a host cell. In general, plasmid vectors containing replicon and control sequences which are derived from species compatible with the host cell are used in connection with these hosts. The vector ordinarily carries a replication site, as well as marking sequences which are capable of providing phenotypic selection in transformed cells. In a non-limiting example, E. coli is often transformed using derivatives of pBR322, a plasmid derived from an E. coli species. pBR322 contains genes for ampicillin and tetracycline resistance and thus provides easy means for identifying transformed cells. The pBR plasmid, or other microbial plasmid or phage must also contain, or be modified to contain, for example, promoters which can be used by the microbial organism for expression of its own proteins.

In addition, phage vectors containing replicon and control sequences that are compatible with the host microorganism can be used as transforming vectors in connection with these hosts. For example, the phage lambda GEMTM 11 may be utilized in making a recombinant phage vector which can be used to transform host cells, such as, for example, E. coli LE392.

Further useful plasmid vectors include pIN vectors (Inouye *et al.,* 1985); and pGEX vectors, for use in generating glutathione S transferase (GST) soluble fusion proteins for later purification and separation or cleavage. Other suitable fusion proteins are those with beta-galactosidase, ubiquitin, and the like.

Bacterial host cells, for example, *E. coli,* comprising the expression vector, are grown in any of a number of suitable media, for example, LB. The expression of the recombinant protein in certain vectors may be induced, as would be understood by those of skill in the art, by contacting a host cell with an agent specific for certain promoters, *e.g*., by adding IPTG to the media or by switching incubation to a higher temperature. After culturing the bacteria for a further period, generally of between 2 and 24 h, the cells are collected by centrifugation and washed to remove residual media.

### D. Viral Vectors

The ability of certain viruses to infect cells or enter cells via receptor mediated endocytosis, and to integrate into host cell genome and express viral genes stably and efficiently have made them attractive candidates for the transfer of foreign nucleic acids into cells (*e.g**.,*** mammalian cells). Components of the present disclosure may be a viral vector that encodes one or more CARs of the disclosure. Non-limiting examples of virus vectors that may be used to deliver a nucleic acid of the present disclosure are described below.

### E. Adenoviral Vectors

A particular method for delivery of the nucleic acid involves the use of an adenovirus expression vector. Although adenovirus vectors are known to have a low capacity for integration into genomic DNA, this feature is counterbalanced by the high efficiency of gene transfer afforded by these vectors. "Adenovirus expression vector" is meant to include those constructs containing adenovirus sequences sufficient to (a) support packaging of the construct and (b) to ultimately express a tissue or cell specific construct that has been cloned therein. Knowledge of the genetic organization or adenovirus, a 36 kb, linear, double stranded DNA virus, allows substitution of large pieces of adenoviral DNA with foreign sequences up to 7 kb (Grunhaus and Horwitz, 1992).

### F. AAV Vectors

The nucleic acid may be introduced into the cell using adenovirus assisted transfection. Increased transfection efficiencies have been reported in cell systems using adenovirus coupled systems (Kelleher and Vos, 1994; Cotten *et al.,* 1992; Curiel, 1994). Adeno associated virus (AAV) is an attractive vector system for use in the cells of the present disclosureas it has a high frequency of integration and it can infect nondividing cells, thus making it useful for delivery of genes into mammalian cells, for example, in tissue culture (Muzyczka, 1992) or in vivo. AAV has a broad host range for infectivity (Tratschin *et al.,* 1984; Laughlin *et al.,* 1986; Lebkowski *et al.,* 1988; McLaughlin *et al.,* 1988). Details concerning the generation and use of rAAV vectors are described in U.S. Patent Nos. 5,139,941 and 4,797,368, each incorporated herein by reference.

### G. Retroviral Vectors

Retroviruses are useful as delivery vectors because of their ability to integrate their genes into the host genome, transferring a large amount of foreign genetic material, infecting a broad spectrum of species and cell types and of being packaged in special cell lines (Miller, 1992).

In order to construct a retroviral vector, a nucleic acid (e.g., one encoding the desired sequence) is inserted into the viral genome in the place of certain viral sequences to produce a virus that is replication defective. In order to produce virions, a packaging cell line containing the gag, pol, and env genes but without the LTR and packaging components is constructed (Mann *et al.,* 1983). When a recombinant plasmid containing a cDNA, together with the retroviral LTR and packaging sequences is introduced into a special cell line (e.g., by calcium phosphate precipitation for example), the packaging sequence allows the RNA transcript of the recombinant plasmid to be packaged into viral particles, which are then secreted into the culture media (Nicolas and Rubenstein, 1988; Temin, 1986; Mann *et al.,* 1983). The media containing the recombinant retroviruses is then collected, optionally concentrated, and used for gene transfer. Retroviral vectors are able to infect a broad variety of cell types. However, integration and stable expression require the division of host cells (Paskind *et al.,* 1975).

Lentiviruses are complex retroviruses, which, in addition to the common retroviral genes gag, pol, and env, contain other genes with regulatory or structural function. Lentiviral vectors are well known in the art (see, for example, Naldini *et al.,* 1996; Zufferey *et al.,* 1997; Blomer *et al.,* 1997; U.S. Pat. Nos. 6,013,516 and 5,994,136). Some examples of lentivirus include the Human Immunodeficiency Viruses: HIV-1, HIV-2 and the Simian Immunodeficiency Virus: SIV. Lentiviral vectors have been generated by multiply attenuating the HIV virulence genes, for example, the genes env, vif, vpr, vpu and nef are deleted making the vector biologically safe.

Recombinant lentiviral vectors are capable of infecting non-dividing cells and can be used for both *in vivo* and *ex vivo* gene transfer and expression of nucleic acid sequences. For example, recombinant lentivirus capable of infecting a non-dividing cell wherein a suitable host cell is transfected with two or more vectors carrying the packaging functions, namely gag, pol and env, as well as rev and tat is described in U.S. Pat. No. 5,994,136, incorporated herein by reference. One may target the recombinant virus by linkage of the envelope protein with an antibody or a particular ligand for targeting to a receptor of a particular cell-type. By inserting a sequence (including a regulatory region) of interest into the viral vector, along with another gene which encodes the ligand for a receptor on a specific target cell, for example, the vector is now target-specific.

### H. Other Viral Vectors

Other viral vectors may be employed as vaccine constructs in the present disclosure. Vectors derived from viruses such as vaccinia virus (Ridgeway, 1988; Baichwal and Sugden, 1986; Coupar *et al.,* 1988), sindbis virus, cytomegalovirus and herpes simplex virus may be employed. They offer several attractive features for various mammalian cells (Friedmann, 1989; Ridgeway, 1988; Baichwal and Sugden, 1986; Coupar *et al.,* 1988; Horwich *et al.,* 1990).

### I. Delivery Using Modified Viruses

A nucleic acid to be delivered may be housed within an infective virus that has been engineered to express a specific binding ligand. The virus particle will thus bind specifically to the cognate receptors of the target cell and deliver the contents to the cell. A novel approach designed to allow specific targeting of retrovirus vectors was developed based on the chemical modification of a retrovirus by the chemical addition of lactose residues to the viral envelope. This modification can permit the specific infection of hepatocytes via sialoglycoprotein receptors.

Another approach to targeting of recombinant retroviruses was designed in which biotinylated antibodies against a retroviral envelope protein and against a specific cell receptor were used. The antibodies were coupled via the biotin components by using streptavidin (Roux *et al.,* 1989). Using antibodies against major histocompatibility complex class I and class II antigens, they demonstrated the infection of a variety of human cells that bore those surface antigens with an ecotropic virus in vitro (Roux *et al.,* 1989).

### J. Vector Delivery and Cell Transformation

Suitable methods for nucleic acid delivery for transfection or transformation of cells are known to one of ordinary skill in the art. Such methods include, but are not limited to, direct delivery of DNA such as by *ex vivo* transfection, by injection, and so forth. Through the application of techniques known in the art, cells may be stably or transiently transformed.

### K. Ex Vivo Transformation

Methods for transfecting eukaryotic cells and tissues removed from an organism in an ex vivo setting are known to those of skill in the art. Thus, it is contemplated that cells or tissues may be removed and transfected *ex vivo* using nucleic acids of the present disclosure. In particular aspects, the transplanted cells or tissues may be placed into an organism. In certain facets, a nucleic acid is expressed in the transplanted cells.

### VII. Kits of the Disclosure

Any of the GPC3-CAR compositions described herein may be comprised in a kit. In a non-limiting example, one or more cells for use in cell therapy and/or the reagents to generate one or more cells for use in cell therapy that harbors recombinant expression vectors may be comprised in a kit. Polynucleotides that encodes the GPC3-CAR or portions thereof may be included in the kit. The kit components are provided in suitable container means.

Some components of the kits may be packaged either in aqueous media or in lyophilized form. The container means of the kits will generally include at least one vial, test tube, flask, bottle, syringe or other container means, into which a component may be placed, and preferably, suitably aliquoted. Where there are more than one component in the kit, the kit also will generally contain a second, third or other additional container into which the additional components may be separately placed. However, various combinations of components may be comprised in a vial. The kits of the present disclosure also will typically include a means for containing the components in close confinement for commercial sale. Such containers may include injection or blow molded plastic containers into which the desired vials are retained.

When the components of the kit are provided in one and/or more liquid solutions, the liquid solution is an aqueous solution, with a sterile aqueous solution being particularly ueful. In some cases, the container means may itself be a syringe, pipette, and/or other such like apparatus, from which the formulation may be applied to an infected area of the body, injected into an animal, and/or even applied to and/or mixed with the other components of the kit.

However, the components of the kit may be provided as dried powder(s). When reagents and/or components are provided as a dry powder, the powder can be reconstituted by the addition of a suitable solvent. It is envisioned that the solvent may also be provided in another container means. The kits may also comprise a second container means for containing a sterile, pharmaceutically acceptable buffer and/or other diluent.

In particular embodiments of the disclosure, cells that are to be used for cell therapy are provided in a kit, and in some cases the cells are essentially the sole component of the kit. The kit may comprise reagents and materials to make the desired cell. In specific embodiments, the reagents and materials include primers for amplifying desired sequences, nucleotides, suitable buffers or buffer reagents, salt, and so forth, and in some cases the reagents include vectors and/or DNA that encodes a CAR as described herein and/or regulatory elements therefor.

In particular embodiments, there are one or more apparatuses in the kit suitable for extracting one or more samples from an individual. The apparatus may be a syringe, scalpel, and so forth.

In some cases of the disclosure, the kit, in addition to cell therapy embodiments, also includes a second cancer therapy, such as chemotherapy, hormone therapy, and/or immunotherapy, for example. The kit(s) may be tailored to a particular cancer for an individual and comprise respective second cancer therapies for the individual.

### VIII. Examples of Methods of Treatment

In various embodiments GPC3-targeting CAR constructs, nucleic acid sequences, vectors, host cells , as contemplated herein and/or pharmaceutical compositions comprising the same are used for the prevention, treatment or amelioration of a cancerous disease, such as a tumorous disease. In particular embodiments, the pharmaceutical composition of the present disclosure may be particularly useful in preventing, ameliorating and/or treating cancer, including cancer that express GPC3 and that may or may not be solid tumors, for example. In specific embodiments, the individual has Simpson-Golabi-Behmel syndrome.

As used herein "treatment" or "treating," includes any beneficial or desirable effect on the symptoms or pathology of a disease or pathological condition, and may include even minimal reductions in one or more measurable markers of the disease or condition being treated, *e.g.,* cancer. Treatment can involve optionally either the reduction or amelioration of symptoms of the disease or condition, or the delaying of the progression of the disease or condition. "Treatment" does not necessarily indicate complete eradication or cure of the disease or condition, or associated symptoms thereof.

As used herein, "prevent," and similar words such as "prevented," "preventing" *etc.,* indicate an approach for preventing, inhibiting, or reducing the likelihood of the occurrence or recurrence of, a disease or condition, *e.g*., cancer. It also refers to delaying the onset or recurrence of a disease or condition or delaying the occurrence or recurrence of the symptoms of a disease or condition. As used herein, "prevention" and similar words also includes reducing the intensity, effect, symptoms and/or burden of a disease or condition prior to onset or recurrence of the disease or condition.

In particular embodiments, the present invention contemplates, in part, GPC3 CAR-expressing cells, GPC3 CAR constructs, GPC3 CAR nucleic acid molecules and GPC3 CAR vectors that can administered either alone or in any combination using standard vectors and/or gene delivery systems, and in at least some aspects, together with a pharmaceutically acceptable carrier or excipient. In certain embodiments, subsequent to administration, the nucleic acid molecules or vectors may be stably integrated into the genome of the subject.

In specific embodiments, viral vectors may be used that are specific for certain cells or tissues and persist in said cells. Suitable pharmaceutical carriers and excipients are well known in the art. The compositions prepared according to the disclosure can be used for the prevention or treatment or delaying the above identified diseases.

Furthermore, the disclosure relates to a method for the prevention, treatment or amelioration of a tumorous disease comprising the step of administering to a subject in the need thereof an effective amount of cells that express a GPC3-targeting CAR, a nucleic acid sequence, a vector, as contemplated herein and/or produced by a process as contemplated herein.

Possible indications for administration of the composition(s) of the exemplary GPC3 CAR cells are cancerous diseases, including tumorous diseases, including hepatocellular carcinoma, a hepatoblastoma, an embryonal sarcoma, a rhabdoid tumor, a Wilm's tumor, yolk sac tumor, choriocarcinoma, a squamous cell carcinoma of the lung, a liposarcoma, a breast carcinoma, a head and neck squamous cell carcinoma (HNSCC), or mesothelioma, for example. Exemplary indications for administration of the composition(s) of GPC3 CAR cells are cancerous diseases, including any malignancies that express GPC3. The administration of the composition(s) of the disclosure is useful for all stages and types of cancer, including for minimal residual disease, early cancer, advanced cancer, and/or metastatic cancer and/or refractory cancer, for example.

The disclosure further encompasses co-administration protocols with other compounds, *e.g.* bispecific antibody constructs, targeted toxins or other compounds, which act *via* immune cells. The clinical regimen for co-administration of the inventive compound(s) may encompass co-administration at the same time, before or after the administration of the other component. Particular combination therapies include chemotherapy, radiation, surgery, hormone therapy, or other types of immunotherapy.

Embodiments relate to a kit comprising a GPC3 CAR construct as defined herein, a nucleic acid sequence as defined herein, a vector as defined herein and/or a host as defined herein. It is also contemplated that the kit of this disclosure comprises a pharmaceutical composition as described herein above, either alone or in combination with further medicaments to be administered to an individual in need of medical treatment or intervention.

### IX. Combination Therapy

In certain embodiments of the disclosure, methods of the present disclosure for clinical aspects are combined with other agents effective in the treatment of hyperproliferative disease, such as anti-cancer agents. An "anti-cancer" agent is capable of negatively affecting cancer in a subject, for example, by killing cancer cells, inducing apoptosis in cancer cells, reducing the growth rate of cancer cells, reducing the incidence or number of metastases, reducing tumor size, inhibiting tumor growth, reducing the blood supply to a tumor or cancer cells, promoting an immune response against cancer cells or a tumor, preventing or inhibiting the progression of cancer, or increasing the lifespan of a subject with cancer. More generally, these other compositions would be provided in a combined amount effective to kill or inhibit proliferation of the cell. This process may involve contacting the cancer cells with the expression construct and the agent(s) or multiple factor(s) at the same time. This may be achieved by contacting the cell with a single composition or pharmacological formulation that includes both agents, or by contacting the cell with two distinct compositions or formulations, at the same time, wherein one composition includes the expression construct and the other includes the second agent(s).

Tumor cell resistance to chemotherapy and radiotherapy agents represents a major problem in clinical oncology. One goal of current cancer research is to find ways to improve the efficacy of chemo- and radiotherapy by combining it with gene therapy. For example, the herpes simplex virus-thymidine kinase (HSV-tK) gene, when delivered to brain tumors by a retroviral vector system, successfully induced susceptibility to the antiviral agent ganciclovir (Culver, *et al.,* 1992). In the context of the present disclosure, it is contemplated that cell therapy could be used similarly in conjunction with chemotherapeutic, radiotherapeutic, or immunotherapeutic intervention, in addition to other pro-apoptotic or cell cycle regulating agents.

Alternatively, the present inventive therapy may precede or follow the other agent treatment by intervals ranging from minutes to weeks. In embodiments where the other agent and present disclosure are applied separately to the individual, one would generally ensure that a significant period of time did not expire between the time of each delivery, such that the agent and inventive therapy would still be able to exert an advantageously combined effect on the cell. In such instances, it is contemplated that one may contact the cell with both modalities within about 12-24 h of each other and, more preferably, within about 6-12 h of each other. In some situations, it may be desirable to extend the time period for treatment significantly, however, where several d (2, 3, 4, 5, 6 or 7) to several wk (1, 2, 3, 4, 5, 6, 7 or 8) lapse between the respective administrations.

Various combinations may be employed, present disclosure is "A" and the secondary agent, such as radio- or chemotherapy, is "B":
A/B/A B/A/B B/B/A A/A/B A/B/B B/A/A A/B/B/B B/A/B/B
B/B/B/A B/B/A/B A/A/B/B A/B/A/B A/B/B/A B/B/A/A
B/A/B/A B/A/A/B A/A/A/B B/A/A/A A/B/A/A A/A/B/A

It is expected that the treatment cycles would be repeated as necessary. It also is contemplated that various standard therapies, as well as surgical intervention, may be applied in combination with the inventive cell therapy.

### A. Chemotherapy

Cancer therapies also include a variety of combination therapies with both chemical and radiation based treatments. Combination chemotherapies include, for example, abraxane, altretamine, docetaxel, herceptin, methotrexate, novantrone, zoladex, cisplatin (CDDP), carboplatin, procarbazine, mechlorethamine, cyclophosphamide, camptothecin, ifosfamide, melphalan, chlorambucil, busulfan, nitrosurea, dactinomycin, daunorubicin, doxorubicin, bleomycin, plicomycin, mitomycin, etoposide (VP16), tamoxifen, raloxifene, estrogen receptor binding agents, taxol, gemcitabien, navelbine, farnesyl-protein tansferase inhibitors, transplatinum, 5-fluorouracil, vincristin, vinblastin and methotrexate, or any analog or derivative variant of the foregoing and also combinations thereof.

In specific embodiments, chemotherapy for the individual is employed in conjunction with the disclosure, for example before, during and/or after administration of the disclosure.

### B. Radiotherapy

Other factors that cause DNA damage and have been used extensively include what are commonly known as γ-rays, X-rays, and/or the directed delivery of radioisotopes to tumor cells. Other forms of DNA damaging factors are also contemplated such as microwaves and UV-irradiation. It is most likely that all of these factors effect a broad range of damage on DNA, on the precursors of DNA, on the replication and repair of DNA, and on the assembly and maintenance of chromosomes. Dosage ranges for X-rays range from daily doses of 50 to 200 roentgens for prolonged periods of time (3 to 4 wk), to single doses of 2000 to 6000 roentgens. Dosage ranges for radioisotopes vary widely, and depend on the half-life of the isotope, the strength and type of radiation emitted, and the uptake by the neoplastic cells.

The terms "contacted" and "exposed," when applied to a cell, are used herein to describe the process by which a therapeutic construct and a chemotherapeutic or radiotherapeutic agent are delivered to a target cell or are placed in direct juxtaposition with the target cell. To achieve cell killing or stasis, both agents are delivered to a cell in a combined amount effective to kill the cell or prevent it from dividing.

### C. Immunotherapy

Immunotherapeutics generally rely on the use of immune effector cells and molecules to target and destroy cancer cells. The immune effector may be, for example, an antibody specific for some marker on the surface of a tumor cell. The antibody alone may serve as an effector of therapy or it may recruit other cells to actually effect cell killing. The antibody also may be conjugated to a drug or toxin (chemotherapeutic, radionuclide, ricin A chain, cholera toxin, pertussis toxin, etc.) and serve merely as a targeting agent. Alternatively, the effector may be a lymphocyte carrying a surface molecule that interacts, either directly or indirectly, with a tumor cell target. Various effector cells include cytotoxic T cells and NK cells.

Immunotherapy other than the inventive therapy described herein could thus be used as part of a combined therapy, in conjunction with the present cell therapy. The general approach for combined therapy is discussed below. Generally, the tumor cell must bear some marker that is amenable to targeting, *i.e.,* is not present on the majority of other cells. Many tumor markers exist and any of these may be suitable for targeting in the context of the present disclosure. Common tumor markers include carcinoembryonic antigen, prostate specific antigen, urinary tumor associated antigen, fetal antigen, tyrosinase (p97), gp68, TAG-72, HMFG, Sialyl Lewis Antigen, MucA, MucB, PLAP, estrogen receptor, laminin receptor, erb B and p155.

### D. Genes

In yet another embodiment, the secondary treatment is a gene therapy in which a therapeutic polynucleotide is administered before, after, or at the same time as the present disclosure clinical embodiments. A variety of expression products are encompassed within the disclosure, including inducers of cellular proliferation, inhibitors of cellular proliferation, or regulators of programmed cell death.

### E. Surgery

Approximately 60% of persons with cancer will undergo surgery of some type, which includes preventative, diagnostic or staging, curative and palliative surgery. Curative surgery is a cancer treatment that may be used in conjunction with other therapies, such as the treatment of the present disclosure, chemotherapy, radiotherapy, hormonal therapy, gene therapy, immunotherapy and/or alternative therapies.

Curative surgery includes resection in which all or part of cancerous tissue is physically removed, excised, and/or destroyed. Tumor resection refers to physical removal of at least part of a tumor. In addition to tumor resection, treatment by surgery includes laser surgery, cryosurgery, electrosurgery, and miscopically controlled surgery (Mohs' surgery). It is further contemplated that the present disclosuremay be used in conjunction with removal of superficial cancers, precancers, or incidental amounts of normal tissue.

Upon excision of part of all of cancerous cells, tissue, or tumor, a cavity may be formed in the body. Treatment may be accomplished by perfusion, direct injection or local application of the area with an additional anti-cancer therapy. Such treatment may be repeated, for example, every 1, 2, 3, 4, 5, 6, or 7 days, or every 1, 2, 3, 4, and 5 weeks or every 1, 2, 3, 4, 5, 6, 7, 8, 9, 10, 11, or 12 months. These treatments may be of varying dosages as well.

### F. Other agents

It is contemplated that other agents may be used in combination with the present disclosure to improve the therapeutic efficacy of treatment. These additional agents include immunomodulatory agents, agents that affect the upregulation of cell surface receptors and GAP junctions, cytostatic and differentiation agents, inhibitors of cell adhesion, or agents that increase the sensitivity of the hyperproliferative cells to apoptotic inducers. Immunomodulatory agents include tumor necrosis factor; interferon alpha, beta, and gamma; IL-2 and other cytokines; F42K and other cytokine analogs; or MIP-1, MIP-1beta, MCP-1, RANTES, and other chemokines. It is further contemplated that the upregulation of cell surface receptors or their ligands such as Fas / Fas ligand, DR4 or DR5 / TRAIL would potentiate the apoptotic inducing abilities of the present disclosure by establishment of an autocrine or paracrine effect on hyperproliferative cells. Increases intercellular signaling by elevating the number of GAP junctions would increase the anti-hyperproliferative effects on the neighboring hyperproliferative cell population. In other embodiments, cytostatic or differentiation agents can be used in combination with the present disclosure to improve the anti-hyperproliferative efficacy of the treatments. Inhibitors of cell adhesion are contemplated to improve the efficacy of the present disclosure. Examples of cell adhesion inhibitors are focal adhesion kinase (FAKs) inhibitors and Lovastatin. It is further contemplated that other agents that increase the sensitivity of a hyperproliferative cell to apoptosis, such as the antibody c225, could be used in combination with the present disclosure to improve the treatment efficacy.

### EXAMPLE 1

### GPC3-SPECIFIC CARS

As described herein, there are glypican-3 specific CARs (GPC3-CAR) with distinct combination of costimulatory endodomains based on published sequences of monoclonal antibodies. High cell surface expression of all CARs on T cells was detected. Glypican-3 specific T cells were generated by retroviral transduction, and they recognize glypican-3 expressing tumor cells.

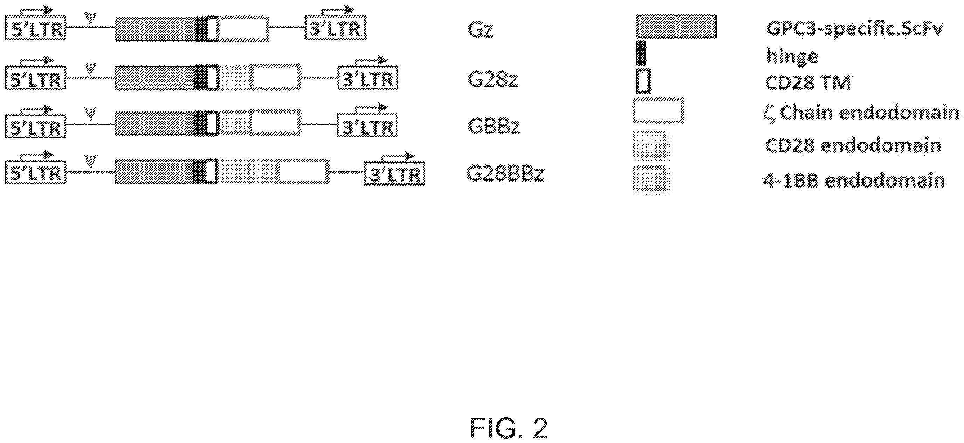

**Design of glypican-3 specific CARs:** CARs were generated with GPC3-specific scFvs fused to CAR backbones (FIG. 2).

**Cell surface expression of GPC3-CARs:** T cells were stimulated with OKT3 and anti-CD28 followed by retroviral transduction. CAR expression was measured with anti-Fab mab-AF647 by FACS. GPC3-CAR expression was detected at or above 80% of cells.

**GPC3-CAR T cells recognize and kill GPC3^{pos} targets:** GPC3^{pos} (HepG2, HUH7 and Hep3B) as well as GPC3^{neg} (A549) cell lines were loaded with Cr⁵¹ and incubated with GPC3-CAR T cells in a standard 4hr Cr⁵¹-release assay. Effective killing was detected at all 3 GPC3^{pos} targets dependent on effector-to-target ration while GPC3^{neg} target was not killed.

**GPC3-CAR T cells release IFN-γ upon stimulation with GPC3^{pos} targets:** GPC3-CAR T cells were co-cultured for 24hrs with GPC3^{pos} (HepG2, HUH7 and Hep3B) as well as GPC3^{neg} (A549) cell lines at 1:1 ratio. IFN-γ level of tissue culture supernatant was measured by Luminex (Millipore) according to the manufacturer's manual. High level of IFN-γ production was detected for all GPC3-CAR constructs when stimulated with GPC3^{pos} targets while non-transduced T cells did not produce IFN-γ. No IFN-γ production was detected for GPC3-CAR T cells when stimulated with GPC^{neg} target except for GBBz which induced low level spontaneous IFN-γ release.

Thus, provided herein are new GPC3-specific CARs, and demonstrated herein are T cells genetically modified with this CAR that recognize and kill GPC3-positive tumor cells in an antigen-specific manner. This technology has broad applications for the immunotherapy of GPC3-positive diseases either as monotherapy or in combination with other treatment modalities.

### EXAMPLE 2

### GLYPICAN-3 CARS

Glypican-3 (GPC3) is one of the six mammalian members of the Glypican family of proteoglycans. Glypican-3 contains heparan sulfate sidechains, is anchored to the cell surface by a glycosyl-phosphatidyl-inositol (Filmus and Selleck, 2001). GPC3 does not directly signal through the cell membrane, the main function of GPC3 is to stabilize wnt thereby increasing its effect on cell proliferation (Capurro *et al.,* 2014). GPC3 is a unique immunotherapeutic target as it is expressed on several solid tumors including the majority of hepatocellular carcinomas (not expressed on normal hepatocytes after fetal development or on cirrhotic liver), hepatoblastomas, embryonal sarcomas, rhabdoid tumors, yolk sac tumors, Wilm's tumors, squamous cell carcinoma of the lung, and liposarcomas (Wang *et al.,* 2006; Yamauchi *et al.,* 2005; Enan *et al.,* 2013; Coston *et al.,* 2008; Baumhoer *et al.,* 2008; Chan *et al.,* 2013; Levy *et al.,* 2012; Tretiakova *et al.,* 2015; Zynger *et al.,* 2008; Zynger *et al.,* 2006; Zynger *et al.,* 2008)). Given its specific expression on cancer cells and its role in cancer progression, GPC3 is an attractive immunotherapeutic target recently demonstrated in early phase clinical trials, where a GPC3 targeting monoclonal antibody (MAb) GC33 was well tolerated and induced antitumor responses in patients with advanced HCC (Zhu *et al.,* 2013; Ikeda *et al.,* 2014).

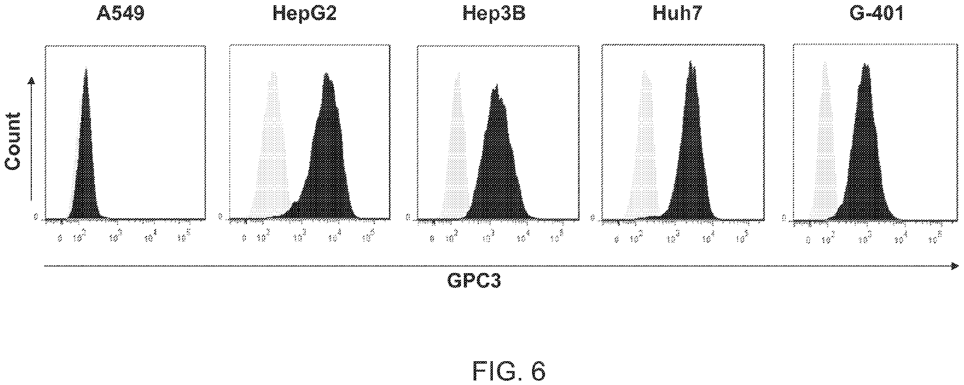

**Glypican-3 expression on tumor** cell lines. HepG2 (HB), HUH7 (HCC) and Hep3B (Hepatitis B virus positive HCC), G401 (malignant rhabdoid tumor) and A549 (lung cancer) were evaluated for the expression of GPC3 by FACS analysis using the glypican-3 specific YP7 antibody. HepG2 (HB), HUH7 (HCC) and Hep3B (Hepatitis B virus positive HCC), G401 (malignant rhabdoid tumor) were GPC3 positive whereas A549 (lung cancer) negative (FIG. 6). This panel of cell lines was used to characterize the function of T cells expressing novel GPC3-specific chimeric antigen receptors (GPC3-CARs).

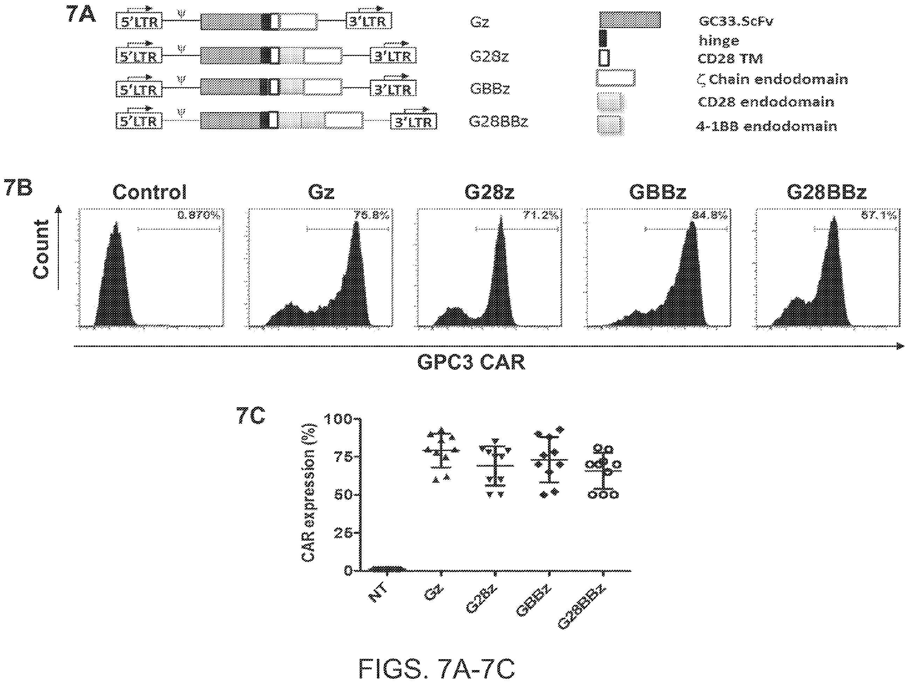

**Structure and cell surface expression of GPC3-CARs.** The single chain variable fragment of the anti-glypican-3 specific MAb GC33 was cloned in frame into SFG retroviral vectors containing CAR expression cassettes. The following four GPC3-CARs were generated: GC33.ζ (Gz), GC33.CD28.ζ (G28z), GC33.4-1BB.ζ (GBBz) and GC33.CD28.4-1BB.ζ (G28BBz) (FIG. 7A). T cells were transduced with retroviral vectors encoding the indicated constructs. Cell surface expression of CARs was measured by flow cytometry using goat anti-mouse F(ab)2 fragment IgG conjugated with AF647. All 4 constructs were stably expressed on the cell surface of transduced T cells. (FIG. 7B shows one representative FACS plot, FIG. 7C shows CAR expressions of cells from 10 independent donors, error bars represent standard deviations).

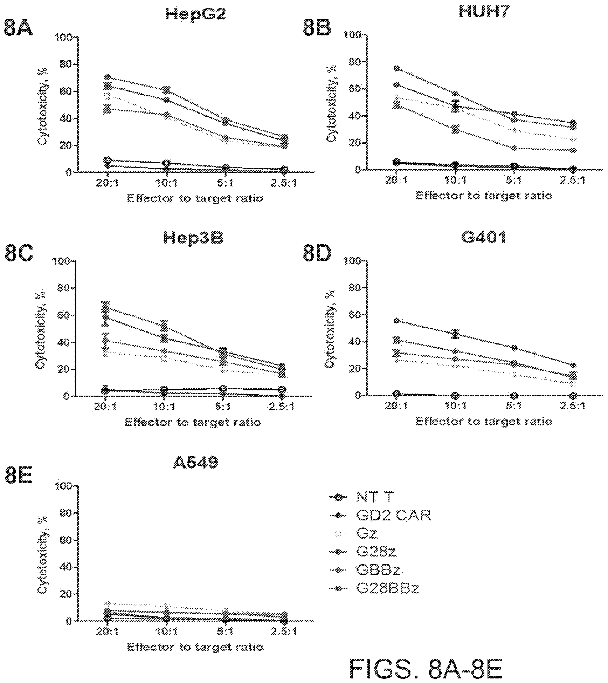

**GPC3-CAR T cells kill GPC3^{pos} cell lines.** To test whether transgenic expression of GPC3-CARs renders the T cells cytotoxic to GPC3^{pos} targets, a standard 4hr ⁵¹Cr assay was performed. In brief, target cells were loaded with ⁵¹Cr, washed and co-cultured with the indicated GPC3 CAR expressing cells in at 37C standard cell culture incubator. Supernatants were collected after 4 hrs percent cell lysis was determined. GPC3-CAR T cells killed GPC3^{pos} HepG2, HUH7, Hep3B, and G401 cells at all effector to target ratios tested. The GPC3^{neg} A549 cell line was not killed by any of the GPC3-CAR T cells, confirming specificity (FIG. 8).

**GPC3-CAR T cells secrete cytokines in the presence of GPC3^{pos} cells.** Having shown that GPC3-CAR T cells kill GPC3-positive tumor cells, it was next determined if they also produce cytokines. GPC3-CAR T cells were co-cultured with indicated cell lines and after 24 hr of coculture, cytokine levels were measured by Luminex analysis. T cells expressing all four GPC3-CARs produced high levels of cytokines when engaging GPC3^{pos} cell lines (FIG. 10). GBBz produced less IL-2 (FIG. 10A-10E) and IL-10 (FIG. 10F-10J). While no IFNγ was detected when T cells expressing Gz, G28z or G28BBz were co-cultured with GPC3^{neg} A549 cell line, spontaneous cytokine production was detected by T cells expressing GBBz CAR (FIG. 10K-10O).

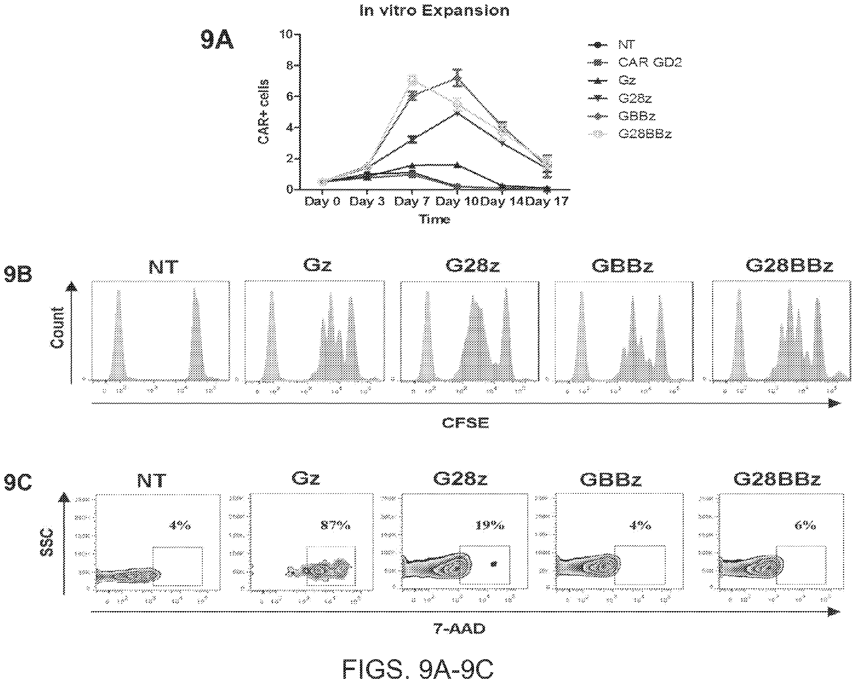

**GPC3-CAR T cells proliferate upon encounter of GPC3^{pos} cells.** Having shown that GPC3-CAR T cells recognize (as judged by cytokine production) and kill GPC3-positive tumor cells, it was next evaluated if they had ability to proliferate post encounter of GPC3-positive tumor cells. GPC3-CAR T cells were co-cultured with HUH7 cells and expanded without cytokines. GPC3-CAR T cells expanded in a GPC3 dependent manner and G28z, GBBz and G28BBz CAR T cells outperformed Gz CAR T cells. Absolute number of GPC3-CAR T cells is shown at indicated time points (FIG. 9A). CFSE dilution (as measure of cell division) of GPC3-CAR T cells is shown 3 days post stimulation (FIG. 9B). 7-AAD staining (as a measure of cell death) is shown on day 5 post stimulation (FIG. 9C).

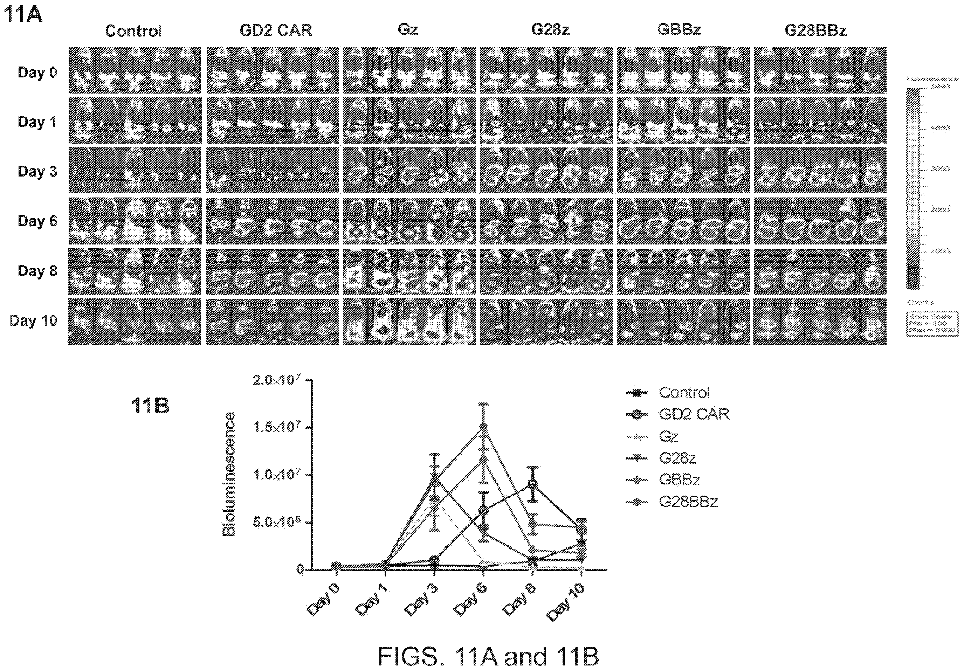

**GPC3-CAR T cells expand after adoptive transfer *in vivo.*** To the test the proliferative potential of GPC3-CAR T cells *in vivo,* NOD/SCID/IL2γ^{null} mice were injected with GPC3^{pos} HUH7 HCC cell line intraperitoneally followed 14 days later by the intravenous (iv) injection of 1x10⁷ GPC3-CAR T cells that also were transduced with a retroviral vector encoding a GFP firefly luciferase fusion gene (GFP.ffLuc) to allow for serial bioluminescence imaging. GD2-CAR T cells and T cells that were only transduced with GFP.ffLuc served as controls. GPC3-CAR T cells expanded for up to 6 days post injection. T cells expressing GPC3-CARs with 41BB containing costimulatory endodomains had showed superior expansion compared to Gz or G28z CAR T cells (FIG. 11A,B).

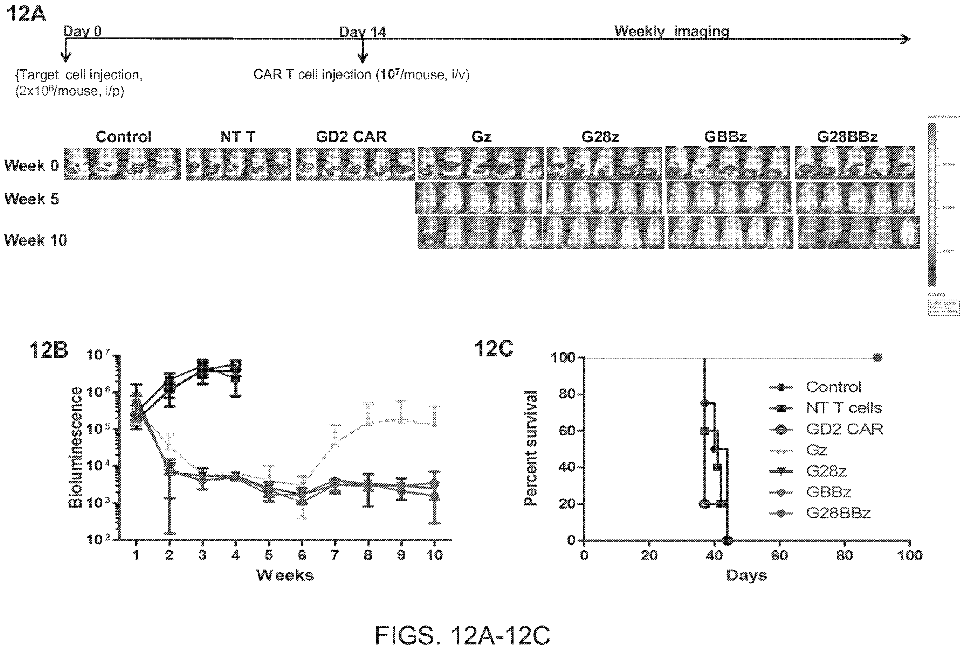

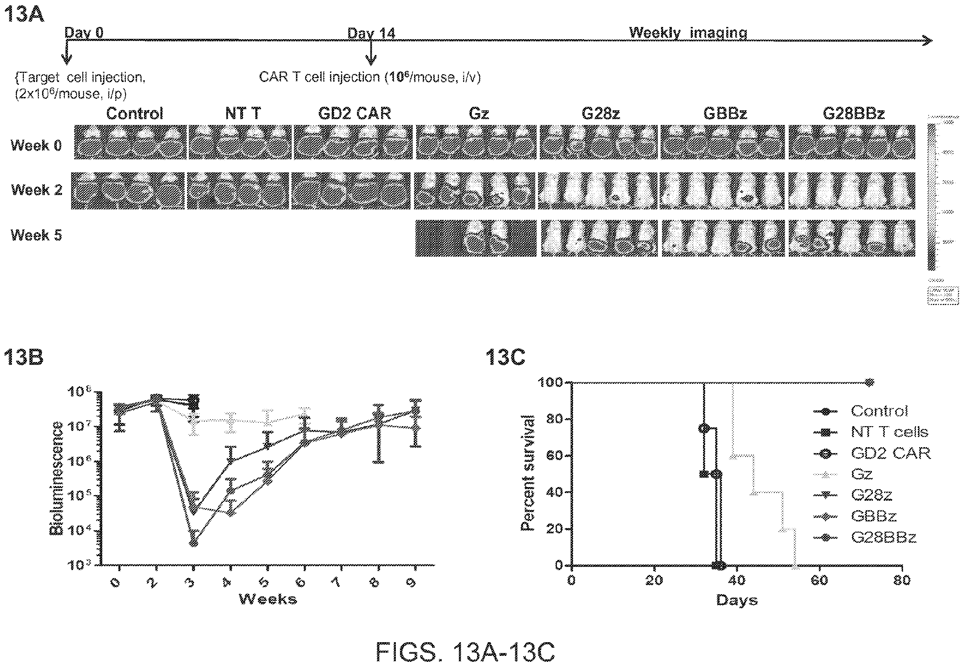

**GPC3-CAR T cells have potent antitumor activity in the HUH-7 HCC xenograft models *in vivo.*** To test the antitumor activity of GPC3-CAR T cells *in vivo* and find the construct with the greatest antitumor potential, NOD/SCID/IL2γ^{null} mice were injected with the GPC3^{pos} HUH7 HCC cell line, which was genetically modified with GFP.ffLuc to allow for serial bioluminescence imaging, intraperitoneally followed 14 days later by the iv infusion of 1x10⁷ GPC3-CAR T cells (FIG. 12). Regardless of endodomain, GPC3 CAR T cells produced robust antitumor effect resulting in complete cure mice with large tumor burden. Survival of mice and tumor bioluminescence is shown in FIG. 12A-C respectively. In contrast, mice injected with placebo, non-transduced T cells (NT T cells) or GD2-CAR T cells died of tumor progression by Day 45 post tumor cell injection. Since there was not observe significant differences between different GPC3-CARs, only 1/10 of the cell dose (1x10⁶ CAR T cells) were injected in the follow up study. T cells expressing GPC3-CARs with costimulatory domains had greater antitumor activity in comparison to the GPC3-CAR with only a zeta endodomain (FIG. 13A-13C).

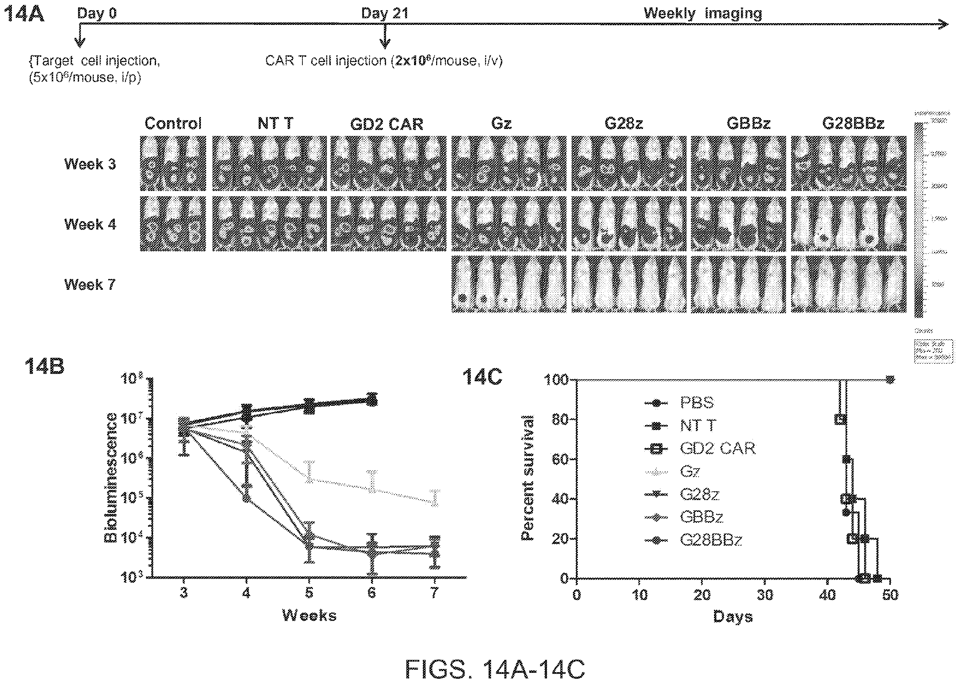

**GPC3-CAR T cells have potent antitumor activity in the G401 malignant rhabdoid tumor xenograft model *in vivo.*** To test the antitumor activity of GPC3-CAR T cells in a second exemplary animal model *in vivo* and find the construct with the greatest antitumor potential, NOD/SCID/IL2γ^{null} mice were injected with the GPC3^{pos} G401 malignant rhabdoid tumor cell line, which was genetically modified with GFP.ffLuc to allow for serial bioluminescence imaging, intraperitoneally followed 21 days later by the iv infusion of 2x10⁷ GPC3-CAR T cells (FIG. 12). GPC3 CAR T cells produced robust antitumor effect resulting in complete cure mice with large tumor burden. Survival of mice and tumor bioluminescence is shown in FIG. 14A-C respectively. In contrast, mice injected with placebo, non-transduced T cells (NT T cells) or GD2-CAR T cells died of tumor progression by Day 46 post tumor cell injection. T cells expressing GPC3-CARs with costimulatory domains had greater antitumor activity in comparison to the GPC3-CAR with only a zeta endodomain (FIG. 14A,14B).

**Summary of results:** Four novel GPC3-CARs (Gz, G28z, GBBz and G28BBz) were produced and stably expressed them on the cell surface of T cells by retroviral transduction (FIG. 7). It was demonstrated in cell culture experiments that GPC3-CAR T cells recognize GPC3-positive cancer cells in an antigen-dependent fashion as judged by their i) cytolytic activity (FIG. 8), and ii) ability to produce cytokines (FIG. 9) and proliferate (FIG. 10) in the presence of GPC3-positive cancer cells. In addition, GPC3-CAR T cells with costimulatory endodomains (G28z, GBBz, G28BBz) expanded in xenograft models (FIG. 11), and had robust antitumor activity (FIG. 12, 13, 14). Thus therein there is GPC3-targeted cell therapy with broad application to malignancies that express GPC3 including but not limited to HCC, hepatoblastoma, embryonal sarcoma, rhabdoid tumor, yolk sac tumor, Wilm's tumor, squamous cell carcinoma of the lung, and liposarcoma. While here, as an example, these CARs are expressed in T cells, novel GPC3-CARs may be expressed in a broad range of i) immune cells including but not limited to NK cells, NKT cells, cytokine-induced killer cells (CIKs), or γδT cells, or ii) stem cells that can be differentiated into immune cells, for example.

### EXAMPLE 3

### THE USE OF Vα24-INVARIANT NATURAL KILLER T CELLS (NKTS) AS A CARRIER FOR GPC3-SPECIFIC CARS

NKTs are an evolutionary conserved subset of innate lymphocytes that are characterized by the expression of an invariant TCR α-chain Vα24-Jα18 and reactivity to self- and microbial-derived glycolipids presented by monomorphic HLA class-I-like molecule CD1d. (Porcelli *et al.,* 1993; Lantz *et al.,* 1994; Bendelac *et al.,* 1995). NKTs can directly target tumor-supportive macrophages and have an indirect antitumor activity even in CD 1d-negative malignancies (Song et al,. 2009; Liu *et al.,* 2012). In addition, NKTs can activate NK-cell cytotoxicity which is a well-studied axis of overall NKT-cell antitumor activity (Metelitsa *et al.,* 2001; Smyth *et al.,* 2002).

NKTs hold special promise for immunotherapy of hepatocellular carcinoma (HCC) as they can eliminate HCC supporting activated hepatic stellate cells (aHSCs) (Anson *et al.,* 2012) and the presence of NKTs in HCC is associated with improved survival (Guo *et al.,* 2012). Transgenic expression of GPC3 CARs in NKTs would therefore enable them with dual reactivity against HCC tumor cells and tumor-supportive aHSCs.

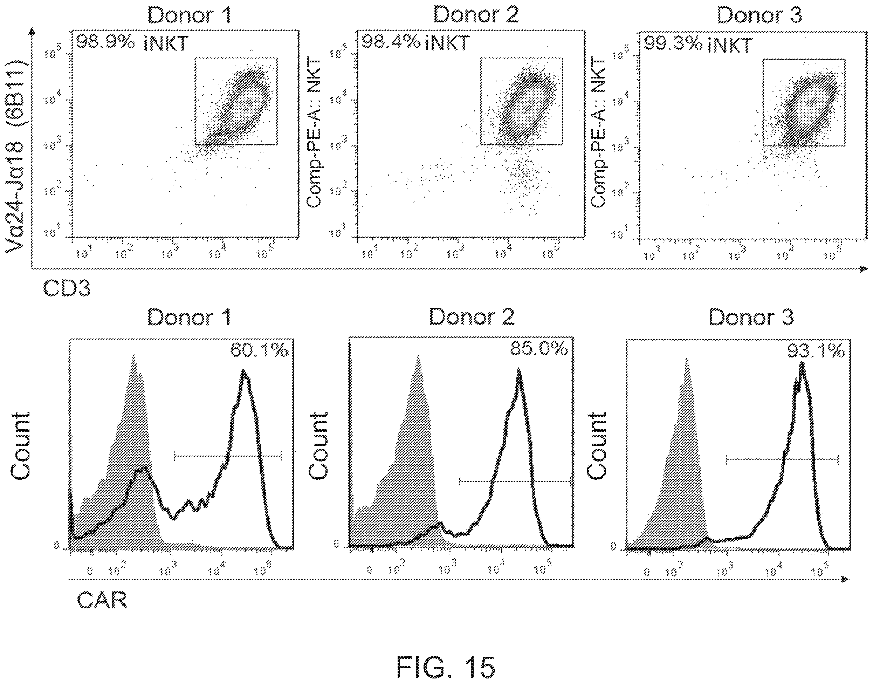

To demonstrate the feasibility of using NKTs as a carrier of GPC3 CAR, primary NKTs were positively sorted from PBMC of three healthy donors with Vα24-Jα18-specific Milteneyi microbeads, activated with alpha-galactosylceramide (aGalCer)-pulsed negative fraction of autologous irradiated PBMC, transduced on day 3 with GPC3 CAR (GC33.CD28.4-1BB.Z) retroviral vector, and expanded in culture with IL-2. On day 7 after transduction, at least 98% of cells in the culture were NKTs as determined by co-expression of CD3 and the invariant TCRα chain (Vα24Jα18). Importantly, 60% - 93% of NKTs expressed GPC3 CAR (FIG. 15). Therefore, primary human NKTs can be stably transduced with a GPC3 CAR and used for immunotherapy of GPC3-positive malignancies.

### REFERENCES

Anson,M. et al. Oncogenic beta-catenin triggers an inflammatory response that determines the aggressiveness of hepatocellular carcinoma in mice. J. Clin. Invest 122, 586-599 (2012).
Baumhoer D, Tornillo L, Stadlmann S, Roncalli M, Diamantis EK, Terracciano LM. Glypican 3 expression in human nonneoplastic, preneoplastic, and neoplastic tissues: a tissue microarray analysis of 4,387 tissue samples. Am.J Clin.Pathol 2008 Jun;129(6):899-906
Bendelac,A. et al. CD1 recognition by mouse NK1+ T lymphocytes. Science 268, 863-865 (1995).
Capurro M, Martin T, Shi W, Filmus J. Glypican-3 binds to Frizzled and plays a direct role in the stimulation of canonical Wnt signaling. J Cell Sci. 2014 Apr 1;127(Pt 7):1565-75
Chan ES, Pawel BR, Corao DA, Venneti S, Russo P, Santi M, Sullivan LM. Immunohistochemical expression of glypican-3 in pediatric tumors: an analysis of 414 cases. Pediatr.Dev.Pathol. 2013 Jul;16(4):272-7
Coston WM, Loera S, Lau SK, Ishizawa S, Jiang Z, Wu CL, Yen Y, Weiss LM, Chu PG. Distinction of hepatocellular carcinoma from benign hepatic mimickers using Glypican-3 and CD34 immunohistochemistry. Am.J Surg.Pathol 2008 Mar;32(3):433-44
Enan ET, El-Hawary AK, El-Tantawy DAE-A, Abu-Hashim MM, Helal NM. Diagnostic role of glypican 3 and CD34 for differentiating hepatocellular carcinoma from nonmalignant hepatocellular lesions. Annals of Diagnostic Pathology 2013 Dec;17(6):490-3
Filmus J, Selleck SB. Glypicans: proteoglycans with a surprise. J.Clin. Invest 2001 Aug;108(4):497-501. PMCID:PMC209407
Filmus J, Capurro M. Glypican-3: a marker and a therapeutic target in hepatocellular carcinoma. FEBS J. 2013 May;280(10):2471-6
Guo,C.L. et al. Associations between infiltrating lymphocyte subsets and hepatocellular carcinoma. Asian Pac. J. Cancer Prev. 13, 5909-5913 (2012).
Ikeda M, Ohkawa S, Okusaka T, Mitsunaga S, Kobayashi S, Morizane C, Suzuki I, Yamamoto S, Furuse J. Japanese phase I study of GC33, a humanized antibody against glypican-3 for advanced hepatocellular carcinoma. Cancer Sci. 2014 Apr;105(4):455-62
Lantz,O. & Bendelac,A. An invariant T cell receptor alpha chain is used by a unique subset of major histocompatibility complex class I-specific CD4+ and CD4-8- T cells in mice and humans. J. Exp. Med. 180, 1097-1106 (1994).
Levy M, Trivedi A, Zhang J, Miles L, Mattis AN, Kim GE, Lassman C, Anders RA, Misdraji J, Yerian LM, et al. Expression of glypican-3 in undifferentiated embryonal sarcoma and mesenchymal hamartoma of the liver. Hum.Pathol. 2012 May;43(5):695-701. PMCID:PMC3568522
Liu,D. et al. IL-15 protects NKT cells from inhibition by tumor-associated macrophages and enhances antimetastatic activity. J. Clin. Invest 122, 2221-2233 (2012).
Metelitsa,L.S. et al. Human NKT cells mediate antitumor cytotoxicity directly by recognizing target cell CD1d with bound ligand or indirectly by producing IL-2 to activate NK cells. J. Immunol. 167, 3114-3122 (2001).
Porcelli,S., Yockey,C.E., Brenner,M.B., & Balk,S.P. Analysis of T cell antigen receptor (TCR) expression by human peripheral blood CD4-8- alpha/beta T cells demonstrates preferential use of several V beta genes and an invariant TCR alpha chain. J. Exp. Med. 178, 1-16 (1993).
Smyth,M.J. et al. Sequential production of interferon-gamma by NK1.1(+) T cells and natural killer cells is essential for the antimetastatic effect of alpha-galactosylceramide. Blood 99, 1259-1266 (2002).
Song,L. et al. Valpha24-invariant NKT cells mediate antitumor activity via killing of tumor-associated macrophages. J. Clin. Invest 119, 1524-1536 (2009).
Tretiakova M, Zynger DL, Luan C, Andeen NK, Finn LS, Kocherginsky M, Teh BT, Yang XJ. Glypican 3 overexpression in primary and metastatic Wilms tumors. Virchows Arch. 2015 Jan;466(1):67-76
Wang XY, Degos F, Dubois S, Tessiore S, Allegretta M, Guttmann RD, Jothy S, Belghiti J, Bedossa P, Paradis Vr. Glypican-3 expression in hepatocellular tumors: diagnostic value for preneoplastic lesions and hepatocellular carcinomas. Human Pathology 2006 Nov;37(11):1435-41
Yamauchi N, Watanabe A, Hishinuma M, Ohashi Ki, Midorikawa Y, Morishita Y, Niki T, Shibahara J, Mori M, Makuuchi M, et al. The glypican 3 oncofetal protein is a promising diagnostic marker for hepatocellular carcinoma. Mod Pathol 2005 May 13;18(12):1591-8
Zynger DL, Gupta A, Luan C, Chou PM, Yang GY, Yang XJ. Expression of glypican 3 in hepatoblastoma: an immunohistochemical study of 65 cases. Hum.Pathol. 2008 Feb;39(2):224-30
Zynger DL, Dimov ND, Luan C, Teh BT, Yang XJ. Glypican 3: a novel marker in testicular germ cell tumors. Am.J.Surg.Pathol. 2006 Dec;30(12):1570-5
Zynger DL, Everton MJ, Dimov ND, Chou PM, Yang XJ. Expression of glypican 3 in ovarian and extragonadal germ cell tumors. Am.J Clin.Pathol 2008 Aug;130(2):224-30
Zhu AX, Gold PJ, El-Khoueiry AB, Abrams TA, Morikawa H, Ohishi N, Ohtomo T, Philip PA. First-in-man phase I study of GC33, a novel recombinant humanized antibody against glypican-3, in patients with advanced hepatocellular carcinoma. Clin.Cancer Res. 2013 Feb 15;19(4):920-8

### FURTHER EMBODIMENTS

1. A method of inhibiting proliferation and/or activity of Glypican-3 (GPC3)-positive cells in an individual, comprising the step of contacting the cells with a therapeutically effective amount of immune cells that express a chimeric antigen receptor (CAR) that targets Glypican-3 (GPC3), wherein the CAR comprises an scFv antibody other than 3E11, 2G9, 4G5, 3D8, or 2E10.
2. The method of item 1, wherein the cells are cancer cells.
3. The method of item 2, wherein the cancer is liver cancer, embryonal sarcoma, rhabdoid tumor, Wilms tumor, choriocarcinoma, or yolk sac tumor.
4. The method of item 2, wherein said cancer cells are not hepatocellular carcinoma cells.
5. The method of item 1, wherein the individual has Simpson-Golabi-Behmel syndrome.
6. The method of any one of items 1-5, wherein said contacting is performed *in vitro.*
7. The method of any one of items 1-5, wherein said contacting is performed in cell culture.
8. The method of any one of items 1-5, wherein said contacting is performed *in vivo,* and said immune cells are cells in an individual.
9. The method of any one of items 1-5 and 8, wherein said contacting is performed *in vivo,* and said immune cells are immune cells in an individual.
10. The method of item 8 or 9, wherein said immune cells are autologous to the individual.
11. The method of item 8 or 9, wherein said immune cells are allogeneic to the individual.
12. The method of any one of items 1-11, wherein said immune cells are T cells, NK cells, Natural Killer T cells, Mucosa Associated Invariant Cells (MAIT cells), γδ T cells, Innate Lymphoid cells, dendritic cells, or a mixture thereof.
13. The method of any one of items 1-12, wherein said immune cells are T cells.
14. The method of item 12, wherein said T cells are CD4+ T cells.
15. The method of item 12, wherein said T cells are CD8+ T cells.
16. The method of item 12, wherein said T cells are Treg cells.
17. The method of any one of items 1-16, wherein the CAR comprises a transmembrane domain selected from the group consisting of CD3-zeta, CD28, CD8α, CD4, or a combination thereof.
18. The method of any one of items 1-16, wherein the CAR comprises a costimulatory molecule endodomain selected from the group consisting of CD28, CD27, 4-1BB, OX40 ICOS, and a combination thereof.
19. The method of any one of items 2 and 7-18, wherein the individual has received, is receiving, or will receive an additional cancer treatment.
20. The method of item 19, wherein the additional cancer treatment comprises chemotherapy, immunotherapy, radiation, surgery, hormone therapy, or a combination thereof.
21. The method of any one of items 1-20, wherein the immune cells harbor a polynucleotide that encodes the CAR.
22. The method of item 21, wherein the polynucleotide comprises SEQ ID NO:2, SEQ ID NO:6, SEQ ID NO:7, SEQ ID NO:8, SEQ ID NO:9, SEQ ID NO: 12, SEQ ID NO: 13, SEQ ID NO: 16, SEQ ID NO: 17, SEQ ID NO: 19, SEQ ID NO:20, SEQ ID NO:21, SEQ ID NO:25, SEQ ID NO:27, SEQ ID NO:29, SEQ ID NO:31, SEQ ID NO:33, or a combination thereof.
23. The method of item 21 or 22, wherein the polynucleotide further comprises a suicide gene.
24. The method of any one of items 1-23, wherein the CAR comprises the GC33 antibody.
25. The method of any one of items 1-24, wherein a transmembrane domain for the CAR comprises the CD28 transmembrane domain.
26. The method of any one of items 1-25, wherein the CAR comprises the amino acid sequence of SEQ ID NO: 1, SEQ ID NO:3, SEQ ID NO: 10, SEQ ID NO:14, SEQ ID NO:18, SEQ ID NO:24, SEQ ID NO:26, SEQ ID NO:28, SEQ ID NO:30, SEQ ID NO:32, or a combination thereof.
27. The method of any one of items 1-26, wherein the CAR comprises the GC33 scFv, linker amino acids, a short hinge, a CD28 transmembrane domain, and a zeta signaling domain.
28. The method of any one of items 1-26, wherein the CAR comprises the GC33 scFv, linker amino acids, a short hinge, a CD28 transmembrane domain, a CD28 signaling domain, and a zeta signaling domain.
29. The method of any one of items 1-26, wherein the CAR comprises the GC33 scFv, linker amino acids, a short hinge, a CD28 transmembrane domain, a 4-1BB endodomain, and a zeta signaling domain.
30. The method of any one of items 1-26, wherein the CAR comprises the GC33 scFv, linker amino acids, a short hinge, a CD28 transmembrane domain, a CD28 endodomain, a 4-1BB endodomain, and a zeta signaling domain.

## Claims

1. T cells that express a chimeric antigen receptor (CAR) that targets Glypican-3 (GPC3) for use in a method of treating a disease associated with proliferation and/or activity of GPC3-positive cells in an individual, the method comprising the step of contacting the cells with a therapeutically effective amount of the T cells, and the CAR comprising (i) an scFv antibody other than 3E11, 2G9, 4G5, 3D8, or 2E10, and (ii) a CD28 costimulatory domain, wherein:
(a) the T cells harbour a polynucleotide that encodes the CAR and comprises SEQ ID NO: 19, SEQ ID NO: 33, SEQ ID NO:12 or SEQ ID NO: 29, or a combination thereof; and/or
(b) the CAR comprises the amino acid sequence of SEQ ID NO: 18, SEQ ID, SEQ ID NO: 32, SEQ ID NO: 10 or SEQ ID NO: 28, or a combination thereof;
wherein the disease is cancer or Simpson-Golabi-Behmel syndrome.

2. The T cells for use of claim 1, wherein the GPC3-positive cells are cancer cells and:
(a) the cancer is liver cancer, embryonal sarcoma, rhabdoid tumor, Wilms tumor, choriocarcinoma, or yolk sac tumor; or
(b) said cancer cells are not hepatocellular carcinoma cells.

3. The T cells for use of claim 1 or 2, wherein:
(a) said contacting is performed *in vitro;* or
(b) said contacting is performed in cell culture.

4. The T cells for use of claim 1 or 2, wherein said contacting is performed *in vivo.*

5. The T cells for use of claim 4, wherein:
(a) said T cells are autologous to the individual; or
(b) said T cells are allogeneic to the individual.

6. The T cells for use of any one of claims 1, 3(b), 4 and 5, wherein the GPC3-positive cells are cancer cells, further wherein the individual has received, is receiving, or will receive an additional cancer treatment.

7. The T cells for use of claim 6, wherein the additional cancer treatment comprises chemotherapy, immunotherapy, radiation, surgery, hormone therapy, or a combination thereof.

8. The T cells for use of any one of the preceding claims, wherein the polynucleotide further comprises a suicide gene.
